# EUROPEAN PATENT SPECIFICATION

(11) **EP 3 450 569 B1**
(45) Date of publication and mention of the grant of the patent: **13.05.2026**
(21) Application number: 17788769.2
(22) Date of filing: 26.04.2017
(51) Int. Cl.: C12Q 1/6848, C12Q 1/6853

(54) **DNA AMPLIFICATION METHOD**
VERFAHREN ZUR DNA-AMPLIFIZIERUNG
PROCÉDÉ D'AMPLIFICATION D'ADN

(30) Priority: 26.04.2016 CN 201610264059
(43) Date of publication of application: 06.03.2019
(73) Proprietor: Xukang (SUZHOU) Medical Science & Technology Co., Ltd, Suzhou, Jiangsu 215123 (CN)
(72) Inventor: GAO, Fangfang, Changping Beijing 102205 (CN); LU, Sijia, Changping Beijing 102205 (CN); REN, Jun, Shanghai 201499 (CN)
(74) Representative: Carpmaels & Ransford LLP
(86) International application number: PCT/CN2017/081977
(87) International publication number: WO 2017/186117

(56) References cited:
- WO-A1-2015/171656
- WO-A2-2012/166425
- CN-A- 103 890 191
- CN-A- 104 630 202
- CN-A- 105 368 936
- CN-A- 105 925 675
- US-A1- 2003 100 006
- US-A1- 2009 099 040
- US-A1- 2015 275 282
- US-B2- 7 993 839
- DIANA L BERNSTEIN ET AL: "The BisPCR2 method for targeted bisulfite sequencing", EPIGENETICS & CHROMATIN, vol. 8, no. 1, August 2015 (2015-08-01), XP055634206, DOI: 10.1186/s13072-015-0020-x
- VOS P ET AL: "AFLP: A NEW TECHNIQUE FOR DNA FINGERPRINTING", NUCLEIC ACIDS RESEARCH,, vol. 23, no. 21, 11 November 1995 (1995-11-11), pages 4407 - 4414, XP002922931, ISSN: 0305-1048
- GROTHUES D ET AL: "PCR amplification of megabase DNA with tagged random primers (T-PCR)", NUCLEIC ACIDS RESEARCH,, vol. 21, no. 5, 1993, XP002126258, ISSN: 0305-1048
- KUN ZHANG ET AL: "Sequencing genomes from single cells by polymerase cloning", NATURE BIOTECHNOLOGY, vol. 24, no. 6, 28 May 2006 (2006-05-28), pages 680 - 686, XP055149835, ISSN: 1087-0156, DOI: 10.1038/nbt1214
- CHAPMAN, A.R. ET AL.: "Single Cell Transcriptome Amplification with MALBAC", PLOS ONE, vol. 10, no. 3, 30 March 2015 (2015-03-30), pages e0120889, XP055433842

## Description

### FIELD OF THE INVENTION

The present invention relates to a method of amplifying DNA, in particular, a method for amplifying and sequencing single-cell whole genomic DNA.

### BACKGROUND

Single-cell whole genome sequencing is a new technique for amplifying and sequencing whole-genome at single-cell level. Its principle is to amplify minute amount whole-genome DNA isolated from a single cell, and perform high-throughput sequencing after obtaining a high coverage of the complete genome.

Currently, there are four major types of whole-genome amplification techniques: Primer Extension Preamplification-Polymerase Chain Reaction (referred to as PEP-PCR, for detailed method see Zhang L, Cui X, Schmitt K, Hubert R, Navidi W, Arnheim N. 1992. Whole genome amplification from a single cell: implications for genetic analysis. Proc Natl Acad Sci U S A.89 (13):5847-51.), Degenerate Oligonucleotide-Primed Polymerase Chain Reaction (referred to as DOP-PCR, for detailed method see Telenius H, Carter NP, Bebb CE, Nordenskjo M, Ponder BA, Tunnacliffe A. 1992. Degenerate oligonucleotide-primed PCR: general amplification of target DNA by a single degenerate primer.Genomics13:718-25), Multiple Displacement Amplification (referred to as MDA, for detailed method see Dean FB, Nelson JR, Giesler TL, LaskenRS. 2001. Rapid amplification of plasmid and phageDNA using phi29 DNA polymerase and multiply-primed rolling circle amplification. Genome Res.11:1095-99), and Multiple Annealing and Looping Based Amplification Cycles (referred to as MALBAC, for detailed method see PCT patent application No. WO2012166425).

Gene sequencing technology experienced three stages of development: the first-generation DNA sequencing technology includes chemical degradation, dideoxy chain termination method, and various sequencing technologies developed on the basis thereof, wherein the most representative is the chain termination method proposed by Sanger and Coulson in 1975. The first-generation technology has high accuracy and long read, and is so far the only method that can perform "head-to-tail" sequencing, but it has drawbacks such as being costly and slow, and is thus not the ideal method for sequencing. The succeeding second- and third-generation sequencing technologies have a common characteristic of high throughput, and are also known as "next-generation sequencing technology (NGS)", wherein the second-generation sequencing technology is represented by pyrosequencing technology, sequencing-by-synthesis (SBS) technology, and sequencing-by-ligation technology. Upon several years of development, pyrosequencing technique and sequencing-by-ligation technology are being rarely used, while the mainstream second-generation sequencing technology nowadays is sequencing-by-synthesis technology, semiconductor sequencing technology and CG sequencing technology. The third-generation sequencing technology is generally divided into two categories, one is single-molecule fluorescence sequencing, the representative technologies of which are TSMS technology and SMRT technology, and the other is nanopore single molecule technology. Compared with the previous two generations of technology, the major feature of the third-generation sequencing technology is single-molecule sequencing. Although the third-generation sequencing technology has made certain progress, the current mainstream sequencing technology remains to be the second-generation sequencing technology.

The whole-genome sequence amplified using current whole-genome amplification technology cannot be directly applied in second-generation sequencing technology. Therefore, no matter the whole-genome sequence described above is applied in sequencing-by-synthesis technology, semiconductor sequencing technology or CG sequencing technology of the second-generation sequencing technologies, a library preparation process is required before loading the whole-genome sequence for sequencing. Each sequencing technology has a corresponding library preparation method, among which library preparations for sequencing-by-synthesis platform are mainly divided into two categories, one is the technology of Y-shaped linker addition or stem-loop linker addition to fragmented DNA after end repair, and the other is transpson technology. Library preparations for semiconductor sequencing platform are also divided into two categories, one is the technology of linker addition to fragmented DNA after end repair, and the other is transpson technology. The library preparation process for CG platform is relatively complex: fragmented DNA need to be subject to enzymatic digestion and two cyclization processes after end repair, which is complicated to operate and time-consuming.

When products amplified in the current mainstream amplification methods are used for the sequencing technologies described above, either library needs to be built separately, or the sequencing yields poor results. Therefore, at present there is an urgent need for an improved amplification method that overcomes one, more, or all defects of the mainstream amplification methods.

### BRIEF SUMMARY OF THE INVENTION

The present invention is defined by the scope of the appended claims and relates to a method of amplifying genomic DNA of a cell and a kit for amplifying genomic DNA.

In one aspect of the present application, a method of amplifying genomic DNA is provided according to claim 1, said method comprises:
(a) providing a first reaction mixture, wherein the first reaction mixture comprises a sample containing the genomic DNA, primers, a mixture of nucleotide monomers, and a nucleic acid polymerase, wherein the primers consist of first primers and third primers, wherein the first primers consist of, in a 5' to 3' orientation, a common sequence and a first variable sequence, or the first primers consist of, in a 5' to 3' orientation, a common sequence, a first spacer sequence and a first variable sequence, wherein the first primers are a mixture of primers comprising the same common sequence and different first variable sequences, wherein each first variable sequence consists of a first random sequence and a fixed sequence at its 3' end, wherein the first random sequence is, in a 5' to 3' orientation, sequentially XalXa2 ...... Xan, and Xai (i=1-n) of the first random sequence all belong to a same set, said set is selected from B, or D, or H, or V, wherein B={T, G, C}, D={ A, T, G}, H={T, A, C}, V={A, C, G}, wherein Xai represents the ith nucleotide from 5' end of the first random sequence, n is a positive integer selected from 4-16, and wherein the first spacer sequence is Yal...... Yam, wherein Yaj (j=1-m) E {A, T, G, C}, wherein Yaj represents the jth nucleotide from 5' end of the first spacer sequence, m is a positive integer selected from 1-3; wherein the third primers consist of, in a 5' to 3' orientation, the common sequence and a third variable sequence, or wherein the third primers consist of, in a 5' to 3' orientation, the common sequence, a third spacer sequence and a third variable sequence, wherein the third primers are a mixture of primers comprising the same common sequence and different third variable sequences, wherein each third variable sequence consists of a third random sequence and a fixed sequence at its 3' end, wherein the third random sequence is, in a 5' to 3' orientation, sequentially XblXbz......Xbn, and Xbi (i=1-n) of the third random sequence all belong to a same set, said set is selected from B- , or D, or H, or V, wherein B={T, G, C}, D={A, T, G}, H={T, A, C}, V={A, C, G}, and Xbi (i=1-n) and Xai (i=1-n) belong to different sets, wherein Xbi represents the ith nucleotide from 5' end of the third random sequence, n is a positive integer selected from 4-16, and wherein the third spacer sequence is Ybi ...... Ybm, wherein Ybj (j=1-m) E {A, T, G, C}, wherein Ybj represents the jth nucleotide from 5' end of the third spacer sequence, m is a positive integer selected from 1-3;
(b) placing the first reaction mixture in a first thermal cycle program for pre-amplification, to obtain a pre-amplification product; (c) providing a second reaction mixture, said second reaction mixture comprises the pre-amplification product obtained from step (b), a second primer, a mixture of nucleotide monomers, and a nucleic acid polymerase, wherein the second primer comprises or consists of, in a 5' to 3' orientation, a specific sequence and the common sequence; (d) placing the second reaction mixture in a second thermal cycle program for amplification, to obtain an amplification product.

In some embodiments, Xₐᵢ (i=1-n) of the first random sequence all belong to set B, X_{bi} (i=1-n) of the third random sequence all belong to set D.

In some embodiments, the fixed sequence is selected from the group consisting of CCC, AAA, TGGG, GTTT, GGG, TTT, TNTNG or GTGG.

In some embodiments, the first variable sequence is selected from Xₐ₁Xₐ₂......XₐₙTGGG or Xₐ₁Xₐ₂......XₐₙGTTT, the third variable sequence is selected from X_{b1}X_{b2}......X_{bn}TGGG or X_{b1}X_{b2}......X_{bn}GTTT.

In some embodiments, the common sequence is selected such that it substantially does not bind to genomic DNA to generate amplification, and the common sequence is 6-60 bp in length. In some embodiments, the common sequence is selected such that an amplification product can be sequenced directly. In some embodiments, the common sequence is selected from SEQ ID NO: 1 [TTGGTAGTGAGTG], SEQ ID NO: 2 [GAGGTGTGATGGA], SEQ ID NO: 3 [GTGATGGTTGAGGTA], SEQ ID NO: 4 [AGATGTGTATAAGAGACAG], SEQ ID NO: 5 [GTGAGTGATGGTTGAGGTAGTGTGGAG] or SEQ ID NO: 6 [GCTCTTCCGATCT].

In some embodiments, the first primer comprises
GCTCTTCCGATCTYₐ₁Xₐ₁Xₐ₂Xₐ₃Xₐ₄Xₐ₅TGGG,
GCTCTTCCGATCTYₐ₁Xₐ₁Xₐ₂Xₐ₃Xₐ₄Xₐ₅GTTT, or a combination thereof, the third primer comprises GCTCTTCCGATCTY_{b1}X_{b1}X_{b2}X_{b3}X_{b4}X_{b5}TGGG,
GCTCTTCCGATCTY_{b1}X_{b1}X_{b2}X_{b3}X_{b4}X_{b5}GTTT, or a combination thereof, wherein Yₐ₁ ∈ {A, T, G, C}, Y_{b1} ^{∈} {A, T, G, C}, said Xₐᵢ (i=1-5) ∈ {T, G, C}, said X_{bi} (i=1-5) ∈ {A, T, G}.

In some embodiments, the method further comprises a step of sequencing an amplification product obtained in step (d), wherein the second primer comprises a sequence complementary or identical to part of or whole of a primer used for sequencing.

In some embodiments, the common sequence comprises a sequence complementary or identical to part of or whole of a primer used for sequencing.

In some embodiments, the specific sequence of the second primer comprises a sequence complementary or identical to part of or whole of a primer used for sequencing.

In some embodiments, the specific sequence of the second primer further comprises a sequence complementary or identical to part of or whole of a capture sequence of a sequencing platform.

In some embodiments, the sequence which is comprised in the specific sequence of the second primer and complementary or identical to part of or whole of a primer used for sequencing comprises or consists of SEQ ID NO: 31 [ACACTCTTTCCCTACACGAC], or SEQ ID NO: 32 [GTGACTGGAGTTCAGACGTGT].

In some embodiments, the sequence which is comprised in the specific sequence of the second primer and complementary or identical to part of or whole of a capture sequence of a sequencing platform comprises or consists of SEQ ID NO: 33
[AATGATACGGCGACCACCGAGATCT], or SEQ ID NO: 34
[CAAGCAGAAGACGGCATACGAGAT].

In some embodiments, the specific sequence of the second primer further comprises a barcode sequence, said barcode sequence is located between the sequence complementary or identical to part of or whole of a capture sequence of a sequencing platform and the sequence complementary or identical to part of or whole of a primer used for sequencing.

In some embodiments, the second primer comprises a primer mixture having identical common sequence and different specific sequences, said different specific sequences are complementary or identical to part of or whole of different primers in a sequencing primer pair used in a same sequencing, respectively.

In some embodiments, the second primer comprises a mixture of sequences set forth in SEQ ID NO: 35 [AATGATACGGCGACCACCGAGATCTACACTCTTTCCCTACACGAC GCTCTTCCGATCT] and SEQ ID NO: 36 [CAAGCAGAAGACGGCATACGAGATCGTGATGTGACTGGAGTTCAGACGTGTGCT CTTCCGATCT].

In some embodiments, the nucleic acid polymerase has thermostablity and/or strand displacement activity. In some embodiments, the nucleic acid polymerase is selected from the group consisting of Phi29 DNA polymerase, Bst DNA polymerase, Pyrophage 3137, Vent polymerase, TOPOTaq DNA polymerase, 9° Nm polymerase, Klenow Fragment DNA polymerase I, MMLV reverse transcriptase, AMV reverse transcriptase, HIV reverse transcriptase, T7 phase DNA polymerase variant, Phusion^{®} High-Fidelity DNA polymerase, Taq polymerase, Bst DNApolymerase, E.coli DNA polymerase, LongAmp Taq DNA polymerase, OneTaq DNA polymerase, Deep Vent DNA polymerase, Vent (exo-)DNA polymerase, Deep Vent (exo-)DNA polymerase, and any combination thereof.

In some embodiments, step (b) enables the variable sequence of the first primer to pair with the genomic DNA and the genomic DNA is amplified to obtain a genomic pre-amplification product, wherein the genomic pre-amplification product comprises the common sequence at its 5' end and a complementary sequence of the common sequence at its 3' end.

In some embodiments, the first thermal cycle program comprises: (b1) a thermal program capable of opening the DNA double strands to obtain a single-strand DNA template; (b2) a thermal program that enables binding of the first primer and the third primer to the single-strand DNA template; (b3) a thermal program that enables extension of the length of the first primer that binds to the single-strand DNA template under the action of the nucleic acid polymerase, to produce a pre-amplification product; (b4) repeating steps (b1) to (b3) to a designated first cycle number, wherein the designated first cycle number is more than 1.

In some embodiments, when undergoing the first cycle, the DNA double strands in step (b1) are genomic DNA double strands, the thermal program comprises a denaturing reaction at a temperature between 90-95°C for 1-20 minutes. In some embodiments, after the first cycle, the thermal program in step (b1) comprises a melting reaction at a temperature between 90-95°C for 3-50 seconds.

After undergoing a second cycle, the pre-amplification product comprises a genomic pre-amplification product comprising the common sequence at its 5' end and a complementary sequence of the common sequence at its 3' end.

In some embodiments, after step (b1) and prior to step (b2), said method does not comprise an additional step of placing the first reaction mixture in a suitable thermal program such that the 3' end and 5' end of the genomic pre-amplification product hybridize to form a hairpin structure (b2'). In some embodiments, the step (b2) comprises placing the reaction mixture in more than one thermal programs to facilitate sufficient binding of the first primer to the DNA template. In some embodiments, the more than one thermal program comprises: a first temperature between 10-20°C, a second temperature between 20-30°C, and a third temperature between 30-50°C. In some embodiments, the step (b2) comprises an annealing reaction at a first temperature for 3-60 s, an annealing reaction at a second temperature for 3-50 s, and an annealing reaction at a third temperature for 3-50 s. In some embodiments, the thermal program of the step (b3) comprises an extension reaction at a temperature between 60-80°C for 10 s-15 min. In some embodiments, the first cycle number of the step (b4) is 2-40.

The step (d) enables the common sequence of the second primer to pair with 3' end of the genomic pre-amplification product and the genomic pre-amplification product is amplified to obtain an extended genomic amplification product.

In some embodiments, the step (d) comprises: (d1) a thermal program capable of opening DNA double strands; (d2) a thermal program further capable of opening DNA double strands; (d3) a thermal program that enables binding of the second primer to single strand of the genomic pre-amplification product obtained in step (b); (d4) a temperature program that enables extension of the length of the second primer that binds to the single strand of the genomic pre-amplification product, under the action of the nucleic acid polymerase; (d5) repeating steps (d2) to (d4) to a designated second cycle number, wherein the designated second cycle number is more than 1.

In some embodiments, the DNA double strands in step (d1) are the genomic pre-amplification product, and the DNA double strands comprise double strands within a DNA hairpin structure comprises, the thermal program comprises a denaturing reaction at a temperature between 90-95°C for 5 s-20 min.

In some embodiments, the thermal program in step (d2) comprises a melting reaction at a temperature between 90-95°C for 3-50 s. In some embodiments, the thermal program in the step (d3) comprises an annealing reaction at a temperature between 45-65°C for 3-50 s. In some embodiments, the thermal program in the step (d4) comprises an extension reaction at a temperature between 60-80°C for 10 s-15 min.

In some embodiments, the method further comprises analyzing the amplification product to identify disease- or phenotype-associated sequence features. In some embodiments, the disease- or phenotype-associated sequence features include chromosomal abnormalities, chromosomal translocation, aneuploidy, partial or complete chromosomal deletion or duplication, fetal HLA haplotypes and paternal mutations, or the disease or phenotype is selected from the group consisting of: beta-thalassemia, Down's syndrome, cystic fibrosis, sickle cell disease, Tay-Sachs disease, Fragile X syndrome, spinal muscular atrophy, hemoglobinopathy, Alpha-thalassemia, X-linked diseases (diseases dominated by genes on the X chromosome), spina bifida, anencephaly, congenital heart disease, obesity, diabetes, cancer, fetal sex, and fetal RHD. In some embodiments, the genomic DNA is derived from a blastomere, blastula trophoblast layer, cultured cells, extracted gDNA or blastula culture medium. Steps (b) and (d) may be as follows: (b) placing the first reaction mixture in a first thermal cycle program, such that the first variable sequence of the first primer and the third variable sequence of the third primer are capable of pairing with the genomic DNA and the genomic DNA is amplified to obtain a genomic pre-amplification product, wherein the genomic pre-amplification product comprises the common sequence at its 5' end and a complementary sequence of the common sequence at its 3' end; wherein the first thermal cycle program comprises: (b1) for the first cycle, reacting at a first denaturing temperature at a temperature between 90-95°C for 1-20 min; for the cycle following the first cycle, reacting at a first denaturing temperature at a temperature between 90-95°C for 3-50 s; (b2) reacting at a first annealing temperature between 10-20°C for 3-60 s, reacting at a second annealing temperature between 20-30°C for 3-50 s, and reacting at a third annealing temperature between 30-50°C for 3-50 s; (b3) reacting at a first extension temperature between 60-80°C for 10 s-15 min; (b4) repeating steps (b1) to (b3) for 2-40 cycles; and (d) placing the second reaction mixture in a second thermal cycle program, such that the common sequence of the second primer is capable of pairing with 3' end of the genomic pre-amplification product and the genomic pre-amplification product is amplified to obtain an extended genomic amplification product, wherein the second thermal cycle program comprises: (d1) reacting at a second denaturing temperature between 90-95°C for 5 s-20 min; (d2) reacting at a second melting temperature between 90-95°C for 3-50 s; (d3) reacting at a fourth annealing temperature between 45-65°C for 3-50 s; (d4) reacting at a second extension temperature between 60-80°C for 10 s-15 min; (d5) repeating steps (d2) to (d4) for 2-40 cycles.

In some embodiments, the common sequence comprises or consists of SEQ ID NO: 6; Xₐᵢ (i=1-n) of the first random sequence all belong to D, X_{bi} (i=1-n) of the third random sequence all belong to B.

In some embodiments, the amplified product obtained in step (d) has completed library construction.

In another aspect of the present application, a kit for amplifying genomic DNA is provided according to claim 14, said kit comprises primers, wherein the primers consist of first primers and third primers, wherein the first primers consist of, in a 5' to 3' orientation, a common sequence and a first variable sequence, or wherein the first primers consist of, in a 5' to 3' orientation, a common sequence, a first spacer sequence and a first variable sequence, wherein the first primers are a mixture of primers comprising the same common sequence and different first variable sequences, wherein each first variable sequence consists of a first random sequence and a fixed sequence at its 3' end, wherein the first random sequence is, in a 5' to 3' orientation, sequentially Xa1Xa2......Xan, and Xai (i=1-n) of the first random sequence all belong to a same set, said set is selected from B , or D, or H, or V, wherein B={T, G, C}, D={A, T, G}, H={T, A, C}, V={A, C, G}, wherein Xai represents the ith nucleotide from 5' end of a first random sequence, n is a positive integer selected from 4-16, and wherein the first spacer sequence is Yal...... Yam, wherein Yaj (j=1-m) E {A, T, G, C}, wherein Yaj represents the jth nucleotide from 5' end of the first spacer sequence, m is a positive integer selected from 1-3, wherein the third primers consist of, in a 5' to 3' orientation, the common sequence and a third variable sequence, or wherein the third primers consist of, in a 5' to 3' orientation, the common sequence, a third spacer sequence and a third variable sequence, wherein the third primers are a mixture of primers comprising the same common sequence and different third variable sequences, wherein each third variable sequence consists of a third random sequence and a fixed sequence at its 3' end, wherein the third random sequence is, in a 5' to 3' orientation, sequentially Xb1Xb2......Xbn, and Xbi (i=1-n) of the third random sequence all belong to a same set, said set is selected from B , or D, or H, or V, wherein B={T, G, C}, D={A, T, G}, H={T, A, C}, V={A, C, G}, and Xbi (i=1-n) and Xai (i=1-n) belong to different sets, wherein Xbi represents the ith nucleotide from 5' end of the third random sequence, n is a positive integer selected from 4-16, and wherein the third spacer sequence is Ybi ...... Yb., wherein Ybj (j=1-m) E {A, T, G, C}, wherein Ybj represents the jth nucleotide from 5' end of the third spacer sequence, m is a positive integer selected from 1-3.

In some embodiments, the common sequence comprises or consists of SEQ ID NO: 6; Xₐᵢ (i=1-n) of the first random sequence all belong to D, X_{bi} (i=1-n) of the third random sequence all belong to B. In some embodiments, the common sequence comprises or consists of SEQ ID NO: 1; Xₐᵢ (i=1-n) of the first random sequence all belong to D, X_{bi} (i=1-n) of the third random sequence all belong to B. In some embodiments, the common sequence comprises or consists of SEQ ID NO: 2; Xₐᵢ (i=1-n) of the first random sequence all belong to D, X_{bi} (i=1-n) of the third random sequence all belong to B.

In some embodiments, the kit is used to construct a whole-genome DNA library.

In some embodiments, the kit further comprises a nucleic acid polymerase, wherein the nucleic acid polymerase is selected from the group consisting of Phi29 DNA polymerase, Bst DNA polymerase, Pyrophage 3137, Vent polymerase, TOPOTaq DNA polymerase, 9° Nm polymerase, Klenow Fragment DNA polymerase I, MMLV reverse transcriptase, AMV reverse transcriptase, HIV reverse transcriptase, T7 phase DNA polymerase variant, Phusion^{®} High-Fidelity DNA polymerase, Taq polymerase, Bst DNApolymerase, E.coli DNA polymerase, LongAmp Taq DNA polymerase, OneTaq DNA polymerase, Deep Vent DNA polymerase, Vent (exo-)DNA polymerase, Deep Vent (exo-)DNA polymerase, and any combination thereof.

In some embodiments, the kit further comprises one or more reagents comprising one or more component selected from the group consisting of a mixture of nucleotide monomers, Mg²⁺, dTT, bovine serum albumin, a pH adjusting agent, a DNase inhibitor, RNase, SO₄²⁻, Cl⁻, K⁺, Ca²⁺, Na⁺, (NH₄)⁺.

In some embodiments, the mixture further comprises a cell lysis agent, said cell lysis agent is selected from one or more of protease K, pepsin, papain, NP-40, Tween, SDS, Triton X-100, EDTA and guanidinium isothiocyanate.

### BRIEF DESCFRIPTION OF FIGURES

The above and other features of the present disclosure will be more comprehensively described through the following specification and claims appended, in combination with the drawings. It is understood that these drawings only depict several embodiments of the present disclosure and therefore should not be considered as limiting the scope of the disclosure. By applying the drawings, the present disclosure will be described more clearly and in more details.
Figure 1 shows basic principle of the amplification method of the present application.

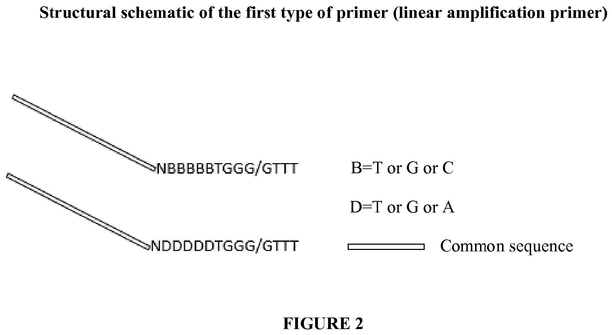

Figure 2 is a structural schematic of the first type of primer (linear amplification primer) used in the method of the present application.
Figure 3 shows gel electrophoresis results of amplification products obtained from amplification of 50 pg human genomic DNA using different mixtures of first type of primers, in which from left to right, lane 1 is a molecular weight marker (M), lanes 2-13 are amplified samples obtained from amplification of gDNA using primer mixtures of experimental groups 1-12 (see Table 1 for details), and lane 14 is molecular weight marker.
Figure 4 shows distribution of A, T, C, and G at each read position in SBS sequencing for amplification products obtained in experimental groups 1-12.
Figure 5 shows amplification results of amplification using primer mixtures of experimental groups 1-12 shown in Table 1, and normal human epidermal fibroblasts (AFP cells) as initial sample. From left to right, lane 1 is molecular weight marker, lanes 2-11 are amplified samples of single cells, and lane 12 is molecular weight marker.

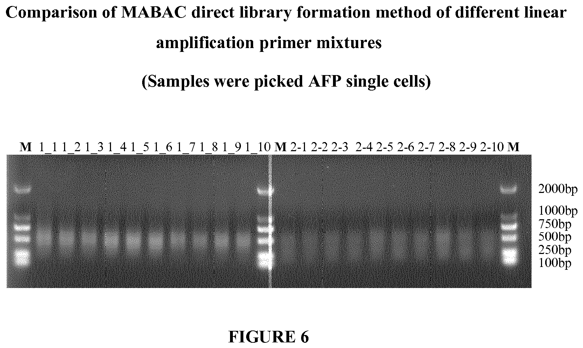

Figure 6 shows gel electrophoresis results of amplification products obtained from amplification using primer mixtures of experimental groups 9/10 and 11/12 shown in Table 1, respectively, and normal human epidermal fibroblasts (AFP cells) as initial sample. From left to right, lane 1 is molecular weight marker, lanes 2-11 are amplified samples obtained from amplification of single cell using primer mixtures of experimental groups 11/12, lane 12 is molecular weight marker, lanes 13-22 are amplified samples obtained from amplification of single cells using primer mixtures of experimental groups 9/10, and lane 23 is molecular weight marker.
Figure 7 shows data amount of each sample, 1_1, 1_2 ... 1_10 and 2_1, 2_2 ... 2_10 in Figure 6 in SBS sequencing (sequencing using equal volume of amplification products).
Figure 8 shows copy number variation coefficient of each sample, 1_1, 1_2 ... 1_10 and 2_1, 2_2 ... 2_10 in Figure 6 in SBS sequencing.
Figure 9 shows copy number of each chromosome of each sample, 1_1, 1_2 ... 1_10 and 2_1, 2_2 ... 2_10 in Figure 6 in SBS sequencing.
Figure 10 shows gel electrophoresis results of amplification products from further PCR amplification of amplified samples 1_1, 1_2, and 2_1, 2_2 in Figure 6, respectively, targeting genes at 35 pathogenic sites listed in Table 8. Each lane, from left to right successively, represents molecular weight marker, amplification results targeting pathogenic sites 1-23 shown in Table 8, molecular weight marker, amplification results targeting pathogenic sites 24-35 shown in Table 8, and molecular weight marker.
Figure 11 shows gel electrophoresis results of amplification products obtained from amplification using primer mixtures of experimental groups 9/10 shown in Table 1, and normal human epidermal fibroblasts (AFP cells) as an initial sample. The lanes, from left to right successively, represent molecular weight marker, amplified samples obtained from amplification of single cells using primer mixtures of experimental groups 9/10 (from 4 parallel experiment wells), and molecular weight marker, respectively.
Figure 12 shows gel electrophoresis results of amplification products from a further PCR amplification of amplified samples 1 and 2 in Figure 11, respectively, targeting genes at 35 pathogenic sites listed in Table 8. The lanes, from left to right successively, represent molecular weight marker, amplification results targeting pathogenic sites 1-23 shown in Table 8, molecular weight marker, amplification results targeting pathogenic sites 24-35 shown in Table 8, and molecular weight marker, respectively.
Figure 13 shows copy number of each chromosome of the amplified samples in Figure 11 in semiconductor sequencing.
Figure 14 shows copy number of chromosomes obtained from SBS sequencing of amplified samples from amplification using primer mixtures of experimental groups 9/10 shown in Table 1, and DNA in blastocyst culture medium as initial sample.

### DETAILED DESCRIPTION

The present invention provides a method according to claim 1 of amplifying genomic DNA, in particular a method of amplifying whole genomic DNA of a single cell.

Before the present invention, library construction is usually performed after completion of gene amplification, and gene sequencing is performed after completion of library construction. Such method has a complicated process and is time-consuming. However, by designing a primer with a special structure and optimizing the amplification process, the inventors of the present application enable direct library formation after single-cell amplification, and thereby dramatically reduces time required to construct a single-cell whole genomic DNA library. Although some designs for primers have been reported in some literatures, these designs all have defects of one kind or another. For example, during the step of single cell whole genome pre-amplification in WO2012/166425, random sequence of primer is selected from four types of bases (i.e., A, T, C and G), but during direct amplification and library construction using this method, auto- or mutual formation of loops or dimers are inevitable, thereby significantly reducing the efficiency of amplification. For another example, it is reported in US8,206,913 that random sequence of primer is selected from two types of bases (i.e., G and T, G and A, A and C, C and T) to avoid auto- or mutual loop formation, however, due to poor randomness of bases before target sequence in sequences amplified using such primers, a positive control sample must be added when the whole plate is loaded for SBS sequencing, in order to rectify base randomness, otherwise the test cannot be processed. Therefore, such method will inevitably cause a waste of some data amount. In contrast to the prior art above, while the primers involved in the present invention comprise high base-randomness, auto- or mutual formation of loops or dimers by primers are substantially absent or extremely rare compared to four-base random primers, and there is high base randomness before target sequence in the library constructed in the present invention. Therefore, the amplification products obtained from amplification using the method of the present invention comprise fewer dimmers, can directly form libraries, and are applicable for whole-plate loading and produce good sequencing results.

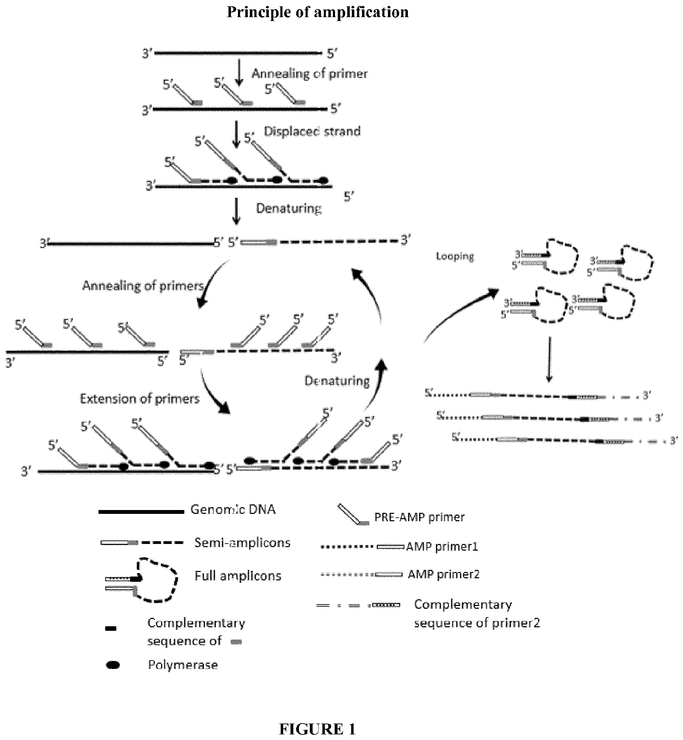

In one aspect, the present application provides a method of amplifying genomic DNA, said method comprises: (a) providing a first reaction mixture, wherein the first reaction mixture comprises a sample containing the genomic DNA, primers, a mixture of nucleotide monomers, and a nucleic acid polymerase, wherein the primers consist of first primers and third primers, wherein the first primers consist of, in a 5' to 3' orientation, a common sequence and a first variable sequence, or the first primers consist of, in a 5' to 3' orientation, a common sequence, a first spacer sequence and a first variable sequence, wherein the first primers are a mixture of primers comprising the same common sequence and different first variable sequences, wherein each first variable sequence consists of a first random sequence and a fixed sequence at its 3' end, wherein the first random sequence is, in a 5' to 3' orientation, sequentially XalXa2 ...... Xan, and Xai (i=1-n) of the first random sequence all belong to a same set, said set is selected from B, or D, or H, or V, wherein B={T, G, C}, D={ A, T, G}, H={T, A, C}, V={ A, C, G}, wherein Xai represents the ith nucleotide from 5' end of the first random sequence, n is a positive integer selected from 4-16, and wherein the first spacer sequence is Yal...... Yam, wherein Yaj (j=1-m) E {A, T, G, C}, wherein Yaj represents the jth nucleotide from 5' end of the first spacer sequence, m is a positive integer selected from 1-3; wherein the third primers consist of, in a 5' to 3' orientation, the common sequence and a third variable sequence, or wherein the third primers consist of, in a 5' to 3' orientation, the common sequence, a third spacer sequence and a third variable sequence, wherein the third primers are a mixture of primers comprising the same common sequence and different third variable sequences, wherein each third variable sequence consists of a third random sequence and a fixed sequence at its 3' end, wherein the third random sequence is, in a 5' to 3' orientation, sequentially XblXbz......Xbn, and Xbi (i=1-n) of the third random sequence all belong to a same set, said set is selected from B- , or D, or H, or V, wherein B={T, G, C}, D={A, T, G}, H={T, A, C}, V={A, C, G}, and Xbi (i=1-n) and Xai (i=1-n) belong to different sets, wherein Xbi represents the ith nucleotide from 5' end of the third random sequence, n is a positive integer selected from 4-16, and wherein the third spacer sequence is Ybi ...... Ybm, wherein Ybj (j=1-m) E {A, T, G, C}, wherein Ybj represents the jth nucleotide from 5' end of the third spacer sequence, m is a positive integer selected from 1-3; (b) placing the first reaction mixture in a first thermal cycle program for pre-amplification, to obtain a pre-amplification product; (c) providing a second reaction mixture, said second reaction mixture comprises the pre-amplification product obtained from step (b), a second primer, a mixture of nucleotide monomers, and a nucleic acid polymerase, wherein the second primer comprises or consists of, in a 5' to 3' orientation, a specific sequence and the common sequence; (d) placing the second reaction mixture in a second thermal cycle program for amplification, to obtain an amplification product. See Figure 1 for illustration of one embodiment of the method provided in the present application.

### Step (a): providing a first reaction mixture

The method of the present application is broadly applicable for amplification of genomic DNA, particularly for amplification of trace-amount genomic DNA.

### i. Genomic DNA

The method of the present application is preferably useful for genomic DNA. In certain embodiments, the initial amount of genomic DNA contained in a reaction mixture is no more than 10 ng, no more than 5 ng, no more than 1 ng, no more than 500 pg, no more than 200 pg, no more than 100 pg, no more than 50 pg, no more than 20 pg, or no more than 10 pg.

A genomic DNA may be from a biological sample, e.g., biological tissue, or body fluid that contains cells or free DNA. Samples containing genomic DNA can be obtained through known methods, e.g. obtained through oral mucosal samples, nasal samples, hair, mouthwash, cord blood, plasma, amniotic fluid, embryonic tissue, endothelial cells, nail samples, hoof samples, etc. A biological sample can be provided in any suitable form, for example, in paraffin embedded form, in freshly isolated form, etc. Genomic DNA may be from any species or biological species, including, but not limited to, humans, mammals, cattle, pigs, sheep, horses, rodents, birds, fish, zebrafish, shrimp, plants, yeasts, viruses or bacteria.

In certain embodiments, genomic DNA is that from a single cell, or that from two or more cells of the same type. Single cells or cells of the same type may be from, e.g., pre-implantation embryos, embryonic cells in peripheral blood of pregnant women, single sperms, egg cells, non-human fertilized eggs, cancer cells, bacterial cells, tumor circulating cells, tumor tissue cells, or single cells or multiple cells of the same type obtained from any tissue. The method of the present application can be used to amplify DNA in some valuable samples or samples with low initial amount, e.g., human egg cells, germ cells, tumor circulating cells, tumor tissue cells, etc.

In some embodiments, genomic DNA is derived from blastomeres, blastula trophoblast, cultured cells, extracted gDNA or blastula culture medium.

Methods for obtaining single cells are also known in the art, e.g., by the method of flow cytometry sorting (Herzenberg et al., Proc Natl Acad Sci USA 76:1453-55, 1979; lverson et al., Prenatal Diagnosis 1:61-73, 1981; Bianchi et al., Prenatal Diagnosis 11:523-28, 1991), fluorescence-activated cell sorting, the method of separation using magnetic beads (MACS, Ganshirt-Ahlert et al., Am J Obstet Gynecol 166:1350, 1992), by using a semi-automatic cell picker (e.g., the QuixellTM cell transfer system by Stoelting Co.) or a combination thereof. In some embodiments, gradient centrifugation and flow cytometry techniques can be used to increase the efficiency of separation and sorting. In some embodiments, cells of particular types, such as cells expressing particular biomarkers, can be selected according to different properties of single cells.

Methods for obtaining genomic DNA are also well known in the art. In certain embodiments, genomic DNA can be released and obtained by lysing cells from biological samples or single cells. Lysing may be performed using any suitable method known in the art, for example, lysing can be performed by means of thermal lysing, base lysing, enzymatic lysing, mechanical lysing, or any combination thereof (see, specifically, e.g., U.S. 7,521,246, Thermo Scientific Pierce Cell Lysis Technical Handbook v2 and Current Protocols in Molecular Biology (1995). John Wiley and Sons, Inc.(supplement 29) pp. 9.7.1-9.7.2.).

Mechanical lysing includes methods that break cells using mechanical forces such as using ultrasonication, high speed stirring, homogenization, pressurization (e.g., French press), decompression and grinding. The most commonly used mechanical lysing method is the liquid homogenization method, which compels cell suspension to pass through a very narrow space, and thus shear force is applied on cell membrane (e.g., as described in WO2013153176 A1).

In certain embodiments, mild lysing methods may be used. For example, cells can be lysed by being heated in a Tween-20-containing solution at 72°C for 2 min, heated in water at 65°C for 10 min (Esumi et al., Neurosci Res 60(4):439-51 (2008)), heated in PCR buffer II (Applied Biosystems) containing 0.5% NP-40 at 70°C for 90 s (Kurimoto et al., Nucleic Acids Res 34(5):e42 (2006)), or using Protease (e.g. Protease K) or a chaotropic salt solution (e.g. guanidine isothiocyanate) (e.g., as described in U.S. Patent Application No. US 20070281313).

Thermal lysing includes heating and repeated freeze-thaw methods. In some embodiments, the thermal lysing comprises lysing for 10-100 minutes at a temperature between 20-100 centigrade. In some embodiments, temperature for thermal lysing can be any temperature between 20-90, 30-90, 40-90, 50-90, 60-90, 70-90, 80-90, 30-80, 40-80, 50-80, 60-80 or 70-80°C. In some embodiments, temperature for thermal lysing is no less than 20, 30, 40 or 50°C. In some embodiments, temperature for thermal lysing is no more than 100, 90 or 80°C. In some embodiments, time for thermal lysing can be any period between 20-100, 20-90, 20-80, 20-70, 20-60, 20-50, 20-40, 20-30, 30-100, 30-90, 30-80, 30-70, 30-60, 30-50, or 30-40 minutes. In some embodiments, time for thermal lysing is no less than 20, 30, 40, 50, 60, 70, 80, or 90 minutes. In some embodiments, time for thermal lysing is no more than 90, 80, 70, 60, 50, 40, 30, or 20 minutes. In some embodiments, temperature for thermal lysing varies over time. In some embodiments, the thermal lysing is maintained under a temperature at 30-60°C for 10-30 minutes, followed by a temperature at 70-90°C for 5-20 minutes.

In some embodiments, the thermal lysing is carried out in the presence of a lysing reagent. In the presence of a lysing reagent, time or temperature required for lysing can be reduced. A lysing reagent can break protein-protein, lipid-lipid and/or protein-lipid interactions, thereby promoting release of genomic DNA from a cell.

In some embodiments, the lysing reagent comprises a surfactant and/or a lyase. Surfactants can be categorized into ionic, amphoteric and non-ionic surfactants. Generally, lysing efficacies of amphoteric and nonionic surfactants are weaker than that of ionic surfactants. Exemplary surfactants include, but are not limited to, one or more of NP-40, Tween, SDS, GHAPS, TritonX-100, TritonX-114, EDTA, sodium deoxycholate, sodium cholate, and guanidine isothiocyanate. Those skilled in the art can select type and concentration of a surfactant based on practical need. In some embodiments, working concentration of a surfactant is 0.01%-5%, 0.1%-3%, 0.3%-2% or 0.5-1%.

Exemplary lyases can be proteinase K, pepsin, papain, etc., or any combination thereof. In some embodiments, working concentration of a lyase is 0.01% -1%, 0.02% -0.5%, 0.03% -0.2%, or 0.4-0.1%.

In the method provided herein, a lysate containing genomic DNA can be used directly in a first reaction mixture. For example, a biological sample may be pre-treated by lysing to obtain a lysate, which is then mixed with other components of the first reaction mixture. If needed, the lysate can be further processed so that the genomic DNA therein is isolated, and then the isolated genomic DNA is further mixed with other components of the first reaction mixture to provide a reaction mixture.

In some embodiments, a nucleic acid sample obtained through lysing can be amplified without being purified. In some embodiments, a nucleic acid sample obtained through lysing is amplified after being purified. In some embodiments, DNA has been subject to various degrees of breakage during the lysing process and can be used for amplification without a particular breaking step. In some embodiments, a nucleic acid sample obtained through lysing is subject to breaking treatment before being amplified.

The present application further provides a simpler method, i.e., directly mixing a genomic DNA-containing cell with other components required for amplification to obtain a first reaction mixture, in other words, genomic DNA in the first reaction mixture is present within a cell. In such circumstances, the first reaction mixture may further contain surfactants (such as, but not limited to, one or more of NP-40, Tween, SDS, TritonX-100, EDTA, and guanidine isothiocyanate) and/or lyase (e.g., one or more of Protease K, pepsin, and papain) capable of lysing the cell. In this way, cell lysing and genomic DNA amplification both occur in the same reaction mixture, which improves reaction efficiency and shortens reaction time.

In certain embodiments, the method provided herein may further comprise placing the reaction mixture in a lysing thermal cycle program after completion of step (a) and prior to step (b), such that the cell is lysed and the genomic DNA is released. Those skilled in the art can select a suitable lysing thermal cycle program according to the lysate components contained in the reaction mixture, type of the cell, etc. Exemplary lysing thermal cycle program includes placing the reaction mixture at 50°C for 3 minutes to 8 hours (e.g., any time period between 3 minutes to 7 hours, 3 minutes to 6 hours, 3 minutes to 5 hours, 3 minutes to 4 hours, 3 minutes to 3 hours, 3 minutes to 2 hours, 3 minutes to 1 hour, 3 minutes to 40 minutes, 3 minutes to 20 minutes; such as 10 minutes, 20 minutes, 30 minutes, etc.), then at 80°C for 2 minutes to 8 hours (e.g., any time period between 2 minutes to 7 hours, 2 minutes to 6 hours, 2 minutes to 5 hours, 2 minutes to 4 hours, 2 minutes to 3 hours, 2 minutes to 2 hours, 2 minutes to 1 hour, 2 minutes to 40 minutes, 2 minutes and 20 minutes; such as 10 minutes, 20 minutes, 30 minutes, etc.). The lysing thermal program can be run for 1 cycle, or 2 or more cycles as needed, depending on specific lysing conditions.

### ii. First type of primers

The method of the present application relates to two different types of primers, of which the first type of primer consists of, in a 5' to 3' orientation, a common sequence, an optional spacer sequence and a variable sequence as set out in claim 1, and the second type of primer comprises a specific sequence and a common sequence, but does not comprise any variable sequence. The "first primer" and the "third primer" described herein both belong to the first type of primer described above. The first primer included in the first reaction mixture consists of, in a 5' to 3' orientation, a common sequence, an optional spacer sequence and a first variable sequence; while the third primer included in a first reaction mixture consists of, in a 5' to 3' orientation, a common sequence, an optional spacer sequence and a third variable sequence. In some embodiments, the first type of primer consists of a common sequence and a variable sequence. In other embodiments, the first type of primer consists of a common sequence, a variable sequence, and a spacer sequence.

### Common sequence

The common sequence in the present application refers to a nucleotide sequence which a first type of primer and a second type of primer both have at their 5' ends. Length of a common sequence can be, e.g., 6-60, 8-50, 9-40, 10-30, 10-15 or 25-30 bases. In the present application, a suitable common sequence is selected, such that it substantially does not bind to genomic DNA, which results in amplification, and avoids cases of polymerization between first type of primers (e.g., between a first primer and a first primer, between a third primer and a third primer, or between a first primer and a third primer) and auto- loop formulation by a first type of primer (e.g., auto- formation of hairpin structure by a first primer due to the complementarity between part of 5' end sequence and part of 3' end sequence of the first primer, or auto- formation of hairpin structure by a third primer due to the complementarity between part of 5' end sequence and part of 3' end sequence of the third primer), as well as polymerization or loop formation between a first type of primer and a second type of primer.

In certain embodiments, a common sequence comprises all four types of bases, A, T, C, G. In certain embodiments, a common sequence only comprises three or two types of bases with poor ability of self-complementary pairing, and does not comprise the other one or two types of bases. In certain embodiments, the common sequence consists of three types of bases, G, A and T, i.e., the common sequence does not contain the C base. In certain embodiments, the common sequence consists of three types of bases, C, A and T, i.e., the common sequence does not contain the G base. In certain embodiments, the common sequence consists of two types of bases, A and T, A and C, A and G, T and C, or, T and G, i.e., the common sequence does not contain G and C at the same time. Without wishing to be bound by theory, it is believed that if a common sequence contains C or G base, primer-primer polymerization may happen, which generates polymers and thereby impairs the ability to amplify genomic DNA. Preferably, a common sequence does not have any self-pairing sequence, or any sequence that would cause primer-primer pairing, or multiple bases of the same type in succession.

In certain embodiments, a suitable base sequence of common sequence and proportion of each base thereof can be selected, to ensure that the common sequence itself does not undergo base pairing with genomic DNA template sequence or resulted in amplification.

In certain embodiments, common sequence can be selected so that the amplification product can be sequenced directly. Without wishing to be bound by theory, a common sequence may be designed to comprise sequences that are complementary or identical to part or all of the primers used for sequencing (e.g., a sequence that is partially identical to, totally identical to, partially complementary to, or totally complementary to a primer used for sequencing). In certain embodiments, the common sequence is specifically selected based on different sequencing platforms. In certain embodiments, the common sequence is specifically selected based on a second-generation or a third-generation sequencing platform. In certain embodiments, the common sequence is specifically selected based on Illumina's NGS sequencing platform. In certain embodiments, a common sequence is specifically selected based on Ion torrent sequencing platform.

In certain embodiments, the common sequence is selected from the group consisting of: SEQ ID NO: 1 [TTGGTAGTGAGTG], SEQ ID NO: 2 [GAGGTGTGATGGA], SEQ ID NO: 3 [GTGATGGTTGAGGTA], SEQ ID NO: 4 [AGATGTGTATAAGAGACAG], SEQ ID NO: 5 [GTGAGTGATGGTTGAGGTAGTGTGGAG] and SEQ ID NO: 6 [GCTCTTCCGATCT].

### Variable sequence

A first type of primer consists of, in a 5' to 3' orientation, a common sequence, an optional spacer sequence and a variable sequence (e.g., a first primer/ third primer comprises a first/third variable sequence, respectively), wherein common sequences in first type of primers are all identical, while variable sequences may vary from each other. A first/third primer is a mixture of primers that comprise a same common sequence and different variable sequences, respectively. The variable sequence in the present application refers to a base sequence whose sequence is not fixed, which comprises a random sequence (e.g., a first/third variable sequence comprises a first/third random sequence, respectively). A variable sequence consists of a random sequence and a fixed sequence.

### a) Random sequence

A random sequence means that bases at each base position of the sequence are all independently and randomly selected from a specific set. Therefore, the random sequence described above represents a set of base sequences composed of different combinations of bases.

Specifically, for example, a first variable sequence may comprise a first random sequence, wherein the base number of the first random sequence is n, n is a positive integer selected from 3-20, and the first random sequence can be represented as, in a 5' to 3' orientation, Xₐ₁Xₐ₂......Xₐₙ, wherein a base at any base position i (i.e., the i^{th} nucleotide from 5' end of the first random sequence, i=1-n) can be represented by Xₐᵢ, wherein each Xₐᵢ is randomly selected from a particular set, e.g., a set consisting of two or three specific types of nucleotides A, T, G, and C. Generally, a selectable set at any base position described above can be represented by means of degenerate codes. For example, a set containing only two types of nucleotides A and G can be represented as R (i.e., R={A, G}). Other sets that can be represented by means of degenerate codes include: Y= {C, T}, M= {A, C}, K= {G, T}, S= {C, G}, W= {A, T}, H= {A, C, T}, B= {C, G, T}, V= {A, C, G}, D= {A, G, T}, N={A, C, G, T}.

A random sequence can be selected in a completely random manner (i.e., any base position in a random sequence), and certain limitations can be further added on the basis of random selection, in order to eliminate some undesirable conditions or to increase the matching degree to a target genomic DNA. In certain embodiments, to avoid generation of complementary pairing between a variable sequence and a common sequence, when the common sequence contains a large amount of G, any base position in the random sequence is selected from set D (i.e., not being C); or when the common sequence contains a large amount of C, any base position in the random sequence is selected from set H (i.e., not being G); when the common sequence contains a large amount of T, any base position in the random sequence is selected from set B (i.e., not being A); or when a common sequence contains a large amount of A, any base position in a random sequence is selected from set V (i.e., not being T).

A random sequence is 4-16, bases long. In certain embodiments, the length of the random sequence is 5 bases. In certain embodiments, the length of the random sequence is 8 bases. Theoretically, if each base position of a random sequence is randomly selected from three types of bases, A, T, G, then a variable sequence with a length of 4 bases can generate 3⁴=81 types of possible random sequences by combination, a random sequence with a length of 5 bases can generate 3⁵=243 types of possible random sequences by combination, and so forth. These random sequences can complementarily pair with corresponding sequences at different positions in genomic DNA, and thereby replication is initiated at different positions in genomic DNA.

Each base Xₐᵢ (i=1-n) at any base position i in the first random sequence belongs to a same set, and wherein the set is selected from one of B, D, H or V. As a non-limiting example, the first primer can have a common sequence and a first random sequence, wherein n=5, and each of any Xₐᵢ (i=1-5) of the random sequence belongs to the same set B, i.e., the random sequence can be represented as BBBBB or (B)₅, a random sequence can be selected from {TTTTT, TGTTT, TCTTT, TTGTT, TTCTT...... }, with a total of 3⁵=243 types of sequence combination. In a specific first reaction mixture which comprises such first primer, these first primers all have a same common sequence and the first random sequence described above, i.e., the first primer in this specific first reactant is a group of primers, these primers all have a same common sequence, and have different random sequences consisting of bases selected from set B.

Unless otherwise explicitly indicated, all descriptions herein concerning a first primer and each part thereof are applicable to a third primer and corresponding part thereof. Similarly, when a first reaction mixture further comprises a third primer, a third variable sequence in the third primer may comprise a third random sequence, wherein the third random sequence is successively, in a 5' to 3' orientation, X_{b1}X_{b2}......X_{bn}, wherein X_{bi} (i=1-n) of the third random sequence all belong to a same set, said set is selected from B, or D, or H, or V, wherein B={T, G, C}, D={A, T, G}, H={T, A, C}, V={A, C, G}, and X_{bi} (i=1-n) and Xₐᵢ (i=1-n) belong to different sets, wherein X_{bi} refers to the i^{th} nucleotide from 5' end of a third random sequence, and n is a positive integer selected from 4-16. In one specific first reaction mixture, certain amount of first primers are comprised, these first primers all have a same common sequence and a first random sequence with a length of n, wherein each base Xₐᵢ of the first random sequence belongs to a same set, and wherein the set is selected from B, D, H or V; meanwhile, the first reaction mixture described above further comprises certain amount of third primers, these third primers all have a same common sequence and a third random sequence with a length of n, wherein each base X_{bi} of the third random sequence belongs to a same set, and wherein the set is selected from B, D, H or V, and X_{bi} and Xₐᵢ belong to different sets. In some embodiments, a first random sequence and a third random sequence have the same length. In other embodiments, the first random sequence and the third random sequence have different lengths.

### b) Fixed sequence

A variable sequence may further comprise a fixed sequence at its 3 'end, and said fixed sequence can be selected from any base combination capable of improving genome coverage. The fixed sequence described herein include, but are not limited to, sequences selected from CCC, AAA, TGGG, GTTT, GGG, TTT, TNTNG or GTGG. N used for description of a fixed sequence in the present application represents any type of single nucleotide selected from A, T, C, and G, but not a random sequence selected from N. In the same group of primers, e.g., in a first primer, a same common sequence, a random sequence containing different sequence combinations, and a same fixed sequence (for example, all first primers comprise either of TGGG or GTTT at their 3' ends) may be comprised successively, in a 5' to 3' orientation. Alternatively, in the same group of primers, e.g., in a first primer, a same common sequence, a random sequence containing different sequence combinations, and different fixed sequences (for example, a first primer comprises a mixture of primers, all of which comprise TGGG at their 3' end, and a mixture of primers, all of which comprise GTTT at their 3' ends) may be comprised successively, in a 5' to 3' orientation. In some embodiments, the first reaction mixture comprises a first primer and a third primer, wherein a first variable sequence of the first primer is selected from Xₐ₁Xₐ₂......XₐₙGGG, Xₐ₁Xₐ₂......XₐₙTTT, Xₐ₁Xₐ₂......XₐₙTGGG or Xₐ₁Xₐ₂......XₐₙGTTT, and a third variable sequence of the third primer is selected from X_{b1}X_{b2}......X_{bn}GGG, X_{b1}X_{b2}......X_{bn}TTT, X_{b1}X_{b2}......X_{bn}TGGG or X_{b1}X_{b2}......X_{bn}GTTT.

In certain embodiments, variable sequences that are more evenly distributed in genome and with higher coverage can also be selected through statistical calculations, thereby increasing recognition opportunity between the variable sequence and genomic DNA.

In certain embodiments, a variable sequence is selected from the group consisting of: (B)ₙCCC, (B)ₙ AAA, (B)ₙ TGGG, (B)ₙ GTTT, (B)ₙ GGG, (B)ₙ TTT, (B)ₙ TNTNG, (B)ₙ GTGGGGG, (D)ₙCCC, (D)ₙ AAA, (D)ₙ TGGG, (D)ₙ GTTT, (D)ₙ GGG, (D)ₙ TTT, (D)ₙ TNTNG, (D)ₙ GTGGGGG, (H)nCCC, (H)ₙ AAA, (H)ₙ TGGG, (H)ₙ GTTT, (H)ₙ GGG, (H)ₙ TTT, (H)ₙ TNTNG, (H)ₙ GTGGGGG, (V)ₙCCC, (V)ₙ AAA, (V)ₙ TGGG, (V)ₙ GTTT, (V)ₙ GGG, (V)ₙ TTT, (V)ₙ TNTNG, (V)ₙ GTGGGGG, wherein n is a positive integer selected from 4-16. In certain embodiments, the first variable sequence in the first primer can have one or more sequences of (B)ₙ CCC, (B)ₙ AAA, (B)ₙ TGGG, (B)ₙ GTTT, (B)ₙ GGG, (B)ₙ TTT, (B)ₙ TNTNG, (B)ₙ GTGGGGG. In certain embodiments, the third variable sequence in the third primer can have one or more sequences of (D)ₙ CCC, (D)ₙ AAA, (D)ₙ TGGG, (D)ₙ GTTT, (D)ₙ GGG, (D)ₙ TTT, (D)ₙ TNTNG, (D)ₙ GTGGGGG.

### A spacer sequence

A common sequence and a variable sequence of a first type of primer may be directly adjacent, or a spacer sequence of one or more bases can be included between them. In certain embodiments, the common sequence and the variable sequence are linked by a spacer sequence with a length of m, wherein m is a positive integer selected from 1-3. When some extent of limitation is applied to a random sequence in a variable sequence to exclude some undesired conditions (e.g., a primer dimer, etc.) or to increase matching degree to a target genomic DNA, m bases completely randomly selected from bases A, T, G, C (a spacer sequence with a length of m) can be introduced into region between a common sequence and a variable sequence, in order to further increase coverage rate of a first type of primer on target genomic DNA without increasing the extent of primer-dimer generation.

In some embodiments, the common sequence in the first primer is linked to the first variable sequence through a first spacer sequence, said first spacer sequence is Yₐ₁......Yₐₘ, wherein Yₐⱼ (j=1-m) ∈ {A, T, G, C}, wherein Yₐⱼ represents the j^{th} nucleotide from 5' end of the first spacer sequence, m is a positive integer selected from 1-3. In some embodiments, the common sequence in the third primer is linked to the third variable sequence through a third spacer sequence, said third spacer sequence is Y_{b1}......Y_{bm}, wherein Y_{bj} (j=1-m) ∈ {A, T, G, C}, wherein Y_{bj} represents the j^{th} nucleotide from 5' end of a third spacer sequence, m is a positive integer selected from 1-3. In some embodiments, m is 1, i.e., the common sequence in the first primer is linked to the first variable sequence through one base selected from set N, and the common sequence in the third primer is linked to the third variable sequence through one base selected from set N.

In certain embodiments, a first primer and a third primer are designed such that the amplification product thereof can be used directly on Illumina's NGS sequencing platform, wherein the first primer comprises a sequence set forth in GCTCTTCCGATCTYₐ₁Xₐ₁Xₐ₂Xₐ₃Xₐ₄Xₐ₅TGGG, GCTCTTCCGATCTYₐ₁Xₐ₁Xₐ₂Xₐ₃Xₐ₄Xₐ₅GTTT, or a combination thereof; the third primer comprises a sequence set forth in GCTCTTCCGATCTY_{b1}X_{b1}X_{b2}X_{b3}X_{b4}X_{b5}TGGG, GCTCTTCCGATCTY_{b1}X₃₁X_{b2}X_{b3}X_{b4}X_{b5}GTTT, or a combination thereof, wherein each base Xₐᵢ (i=1-n) at any base position i belongs to a same set, wherein the set is selected from one of B, D, H or V, and each of base X_{bi} (i=1-n) at any base position i belongs to a same set, wherein the set is selected from one of B, D, H or V, and X_{bi} (i=1-n) and Xₐᵢ (i=1-n) belong to different sets; wherein Yₐ₁ ∈ {A, T, G, C}, Y_{b1} ∈ {A, T, G, C}. In some specific embodiments, the aforesaid Xₐᵢ (i=1-5) ∈ {T, G, C}, X_{bi} (i=1-5) ∈ {A, T, G}, i.e., the first primer comprises a sequence set forth in SEQ ID NO: 7, SEQ ID NO: 11, or a combination thereof; the third primer comprises a sequence set forth in SEQ ID NO: 8, SEQ ID NO: 12, or a combination thereof.

In certain embodiments, the first type of primer comprises or consists of a sequence selected from the group consisting of SEQ ID NO: 7, SEQ ID NO: 8, SEQ ID NO: 9, SEQ ID NO: 10, SEQ ID NO: 11, SEQ ID NO: 12, SEQ ID NO: 13 or SEQ ID NO: 14, wherein the common sequence of each first type of primer comprises or consists of SEQ ID NO: 6. In certain embodiments, the first type of primer comprises a primer that consists of a sequence set forth in SEQ ID NO: 7 and /or a primer that consists of a sequence set forth in SEQ ID NO: 11. In certain embodiments, the first type of primer comprises a primer that consists of a sequence set forth in SEQ ID NO: 8 and a primer that consists of a sequence set forth in SEQ ID NO: 12. In certain embodiments, the first type of primer comprises a primer that consists of a sequence set forth in SEQ ID NO: 7 or a primer that consists of a sequence set forth in SEQ ID NO: 11; and a primer that consists of a sequence set forth in SEQ ID NO: 8 or a primer that consists of a sequence set forth in SEQ ID NO: 12. In certain embodiments, a first type of primer comprises a primer that consists of a sequence set forth in SEQ ID NO: 7, a primer that consists of a sequence set forth in SEQ ID NO: 11, a primer that consists of a sequence set forth in SEQ ID NO: 8 and a primer that consists of a sequence set forth in SEQ ID NO: 12.

In certain embodiments, the first type of primer comprises or consists of a sequence set forth in SEQ ID NOs: 15-22, wherein the common sequence of each first type of primer comprises or consists of SEQ ID NO: 1. In certain embodiments, the first type of primer comprises a primer that consists of a sequence set forth in SEQ ID NO: 15 and /or a primer that consists of a sequence set forth in SEQ ID NO: 19. In certain embodiments, the first type of primer comprises a primer that consists of a sequence set forth in SEQ ID NO: 16 and/or a primer that consists of a sequence set forth in SEQ ID NO: 20. In certain embodiments, the first type of primer comprises a primer that consists of a sequence set forth in SEQ ID NO: 15 or a primer that consists of a sequence set forth in SEQ ID NO: 19; and a primer that consists of a sequence set forth in SEQ ID NO: 16 or a primer that consists of a sequence set forth in SEQ ID NO: 20. In certain embodiments, the first type of primer comprises a primer that consists of a sequence set forth in SEQ ID NO: 15, a primer that consists of a sequence set forth in SEQ ID NO: 19, a primer that consists of a sequence set forth in SEQ ID NO: 16 and a primer that consists of a sequence set forth in SEQ ID NO: 20.

In certain embodiments, the first type of primer comprises or consists of a sequence set forth in SEQ ID NOs: 23-30, wherein a common sequence of each first type of primer comprises or consists of SEQ ID NO: 2. In certain embodiments, the first type of primer comprises one or two of the following: a primer that consists of a sequence set forth in SEQ ID NO: 23 and/or a primer that consists of a sequence set forth in SEQ ID NO: 27. In certain embodiments, the first type of primer comprises one or two of the following: a primer that consists of a sequence set forth in SEQ ID NO: 24 and/or a primer that consists of a sequence set forth in SEQ ID NO: 28. In certain embodiments, the first type of primer comprises a primer that consists of a sequence set forth in SEQ ID NO: 23 or a primer that consists of a sequence set forth in SEQ ID NO: 27; and a primer that consists of a sequence set forth in SEQ ID NO: 24 or a primer that consists of a sequence set forth in SEQ ID NO: 28. In certain embodiments, a first type of primer comprises a primer that consists of a sequence set forth in SEQ ID NO: 23, a primer that consists of a sequence set forth in SEQ ID NO: 27, a primer that consists of a sequence set forth in SEQ ID NO: 24 and a primer that consists of a sequence set forth in SEQ ID NO: 28.

In some embodiments, the total concentration of the first and the third primers in the first reaction mixture is 10-150 ng/µL. In some embodiments, the total concentration of the first and the third primers in the first reaction mixture is 10-120 ng/µL, 10-100 ng/µL, 10-90 ng/µL, 10-80 ng/µL, 10-70 ng/µL, 10-60 ng/µL, 10-50 ng/µL, 10-40 ng/µL, 20-120 ng/µL, 20-100 ng/µL, 20-80 ng/µL, 20-70 ng/µL, 20-60 ng/µL, 20-50 ng/µL, 30-140 ng/µL, 30-120 ng/µL, 30-100 ng/µL, 30-80 ng/µL, 30-60 ng/µL or 30-40 ng/µL. In some embodiments, the concentration of the first and the third primers in the first reaction mixture are respectively 10-140 ng/µL, 10-120ng/µL, 10-100 ng/µL, 10-80 ng/µL, 10-60 ng/µL, 10-30 ng/µL, 10-20 ng/µL, 20-120 ng/µL, 20-100 ng/µL, 20-80 ng/µL, 20-60 ng/µL, 20-40 ng/µL or 20-30 ng/µL. In some embodiments, the concentration of the first and the third primers in the first reaction mixture are respectively 15 ng/µL, 30 ng/µL or 60 ng/µL. In some embodiments, the concentration of the first primer and the third primer are the same. In some embodiments, the first and the third primers in the first reaction mixture are 100-800 pmol, respectively. In some embodiments, the first and the third primers in the first reaction mixture are 400-600 pmol in total.

### iii. Other components

The first reaction mixture further comprises other components required for DNA amplification, such as nucleic acid polymerase, a mixture of nucleotide monomers, and suitable metal ions and buffer components required for enzymatic activity, and the like. For at least one or more types of these components, reagents known in the art can be used.

Nucleic acid polymerase in the present application refers to an enzyme capable of synthesizing a new nucleic acid strand. Any nucleic acid polymerase suitable for the method of the present application can be used. Preferably, DNA polymerase is used. In certain embodiments, the method of the present application uses a thermostable nucleic acid polymerase, such as those whose polymerase activity does not decrease or decrease by less than 1%, 3%, 5%, 7%, 10%, 20%, 30%, 40% or 50% at a temperature for PCR amplification (e.g., 95°C). In certain embodiments, the nucleic acid polymerase used in the method of the present application has strand displacement activity. The "strand displacement activity" of the present application refers to an activity of nucleic acid polymerase that enables separation of a nucleic acid template from the complementary strand with which it pairs and binds, and where such separation performs in a 5' to 3' direction, and is accompanied with generation of a new nucleic acid strand that is complementary to the template. Nucleic acid polymerases with strand displacement ability and applications thereof are known in the art, see e.g., U.S. Patent No. 5824517. Suitable nucleic acid polymerases include, but are not limited to: one or more of Phi29 DNA polymerase, Bst DNA polymerase, Bst 2.0 DNA polymerase, Pyrophage 3137, Vent polymerase (e.g. Thermococcus litoralis Vent polymerase, Deep Vent polymerase, Vent(-exo) polymerase, Deep Vent(-exo) polymerase), TOPOTaq DNA Polymerase, 9° Nm polymerase, Klenow Fragment DNA polymerase I, MMLV reverse transcriptase, AMV reverse transcriptase, HIV reverse transcriptase, T7 phase DNA polymerase variant (lacking 3'-5' exonuclease activity), Phusion^{®} High-Fidelity DNA polymerase, Taq polymerase, Psp GBD (exo-) DNA polymerase, Bst DNA polymerase (full-length), E. coli DNA polymerase, LongAmp Taq DNA polymerase, OneTaq DNA polymerase.

The mixture of nucleotide monomers herein refers to a mixture of dATP, dTTP, dGTP, dCTP.

In certain embodiments, a first reaction mixture contains one or more of *Thermococcus litoralis* Vent polymerase, Deep Vent polymerase, Vent(-exo) polymerase, or Deep Vent(-exo) polymerase. In certain embodiments, the reaction mixture contains *Thermococcus litoralis* Vent polymerase. *Thermococcus litoralis* Vent polymerase refers to a natural polymerase isolated from *Thermococcus litoralis.* In certain embodiments, the reaction mixture contains Deep Vent polymerase. The Deep Vent polymerase refers to a natural polymerase isolated from Pyrococcus *species GB-D.* In certain embodiments, the reaction mixture contains Vent(-exo) polymerase. Vent(-exo) polymerase refers to an enzyme resulted from D141A/E143A gene engineering of *Thermococcus litoralis* Vent polymerase. In certain embodiments, the reaction mixture contains Deep Vent(-exo) polymerase. Deep Vent (-exo) polymerase refers to an enzyme resulted from D141A/E143A gene engineering of Deep Vent polymerase. The various Vent polymerases in the present application are commercially available, e.g., from New England Biolabs Company.

A first reaction mixture can also comprise suitable metal ions required for exerting enzymatic activity of nucleic acid polymerase (e.g., Mg²⁺ ions in suitable concentration (e.g., at a final concentration of about 1.5 mM to about 8 mM), a mixture of nucleotide monomers (e.g., dATP, dGTP, dTTP, and dCTP), bovine serum albumin (BSA), dTT (e.g., at a final concentration of about 2 mM to about 7 mM), purified water, and the like.

In certain embodiments, a first reaction mixture can also further comprise a pH regulator, such that pH value of the reaction mixture is maintained between 7.0-9.0. Suitable pH regulators may include, e.g, Tris HCl and Tris SO₄. In certain embodiments, a first reaction mixture can also further comprise one or more types of other components, e.g., DNase inhibitor, RNase, SO₄²⁻, Cl⁻, K⁺, Ca²⁺, Na⁺, and/or (NH₄)⁺, and the like.

### Step (b): placing in a first thermal cycle program

The method provided herein comprises step (b): placing the first reaction mixture in a first thermal cycle program, such that the variable sequence of the first type of primer (a first primer, or a first primer and a third primer) can bind to the genomic DNA through base-pairing, and that genomic DNA is replicated under the action of a nucleic acid polymerase.

"Amplification" used in the present application means adding nucleotides complementary to a nucleic acid template to the 3' end of a primer under the action of a nucleic acid polymerase, in order to synthesize a new nucleic acid strand that is base-complementary to the nucleic acid template. Suitable methods for amplifying nucleic acids, such as polymerase chain reaction (PCR), ligase chain reaction (LCR), or other suitable amplification methods, may be used. These methods are all known in the art, see for example, U.S. Patents U.S. 4,683,195 and U.S. 4,683,202, as well as Innis et al. PCR protocols: a guide to method and applications. Academic Press, Incorporated (1990) and Wu et al. (1989) Genomics 4: 560-569.

During the process of amplification, the reaction mixture is placed in a suitable thermal cycle program, such that DNA template double strands are unwound into single strands, the first/third primer hybridizes with template single strand, and then elongation occurs at 3' end of a primer under the action of a DNA polymerase. Thus, a thermal cycle program typically comprises: a denaturing or melting temperature at which DNA template double strands are unwound into single strands; an annealing temperature at which a primer specifically hybridizes with a single-strand DNA template; and an elongation temperature at which DNA polymerase adds nucleotides complementary to DNA template bases at the 3' end of a primer, so that the primer elongates, and a new DNA strand that is complementary to the DNA template is obtained. The newly synthesized DNA strand can serve as a new DNA template in the next reaction cycle, for a new cycle of DNA synthesis.

In the first cycle of a first thermal cycle program, the first the reaction mixture is placed in a thermal program capable of opening double strands of the genomic DNA (step (b1)). In the first cycle, to ensure that genomic DNA double strands are completely unwound into single strand (i.e., denaturation/melting), a high reaction temperature (such as 90°C-95°C) can be used and maintained for a long reaction time (e.g., reaction at a temperature between 90-95°C for 1-20 min). However, in subsequent cycles, double strands that need to be unwounded are those generated during amplification. In this case, there is no need for a long melting time, as long as the semi-amplicon or full-amplicon double strands to be amplified can denature into single strands (e.g., melting at a temperature between 90-95°C for 3-50 s).

Next, the first reaction mixture is placed in a thermal program that enables binding of the first type of primer (a first primer or a first primer and a third primer) to the single-strand DNA template (step (b2)). In this thermal program, the variable sequence in the first type of primer binds to complementary sequences at different positions in genomic DNA through base complementarity (i.e., annealing), and thereby replications are initiated at different positions in genomic DNA. Due to the diversity of variable sequences in the first type of primer, wherein differences exist with regard to both base ratio and sequence, the optimal binding temperature for each variable sequence to genomic DNA also varies greatly. Thus, at a given annealing temperature, it is possible that only some of the primers can bind to genomic DNA well, while the binding of the others to genomic DNA may not be ideal. In certain embodiments, the step (b2) comprises a program of placing the reaction mixture in more than one temperature, to facilitate sufficient binding of the first type of primer to the DNA template. For example, DNA denatured reaction mixture can be rapidly cooled to a low temperature, such as about 10°C-20°C, followed by allowing the reaction mixture to react for a suitable period at different annealing temperatures respectively, by means of gradient heating, whereby to ensure that as many primers as possible pair with genomic DNA. In certain embodiments, step (b2) comprises reaction for a suitable period (e.g., 3-60 s) at a first annealing temperature between 10-20°C (e.g., 15°C), reaction for a suitable period (e.g., 3-50 s) at a second annealing temperature between 20-30°C (e.g., 25°C), and reaction for a suitable period (e.g., 3-50 s) at a third annealing temperature between 30-50°C (e.g., 35°C).

It is well known in the art that annealing temperature of a primer is generally no more than 5°C lower than Tm value of a primer, and an excessively low annealing temperature will lead to primer-primer non-specific binding, whereby resulting in primer aggregation and nonspecific amplification products. Therefore, low temperatures such as 10°C-20°C will not usually be used as primer annealing temperature. However, it is unexpectedly found by the inventors, that even if gradient heating starts from a low temperature (e.g., 10°C-20°C), pairing between primers and genomic DNA can still maintain good specificity, and amplification results still retain very low variability, indicating accurate and reliable amplification results. Meanwhile, since annealing temperatures for primers cover circumstance of low temperature, binding of wider range of primer sequences to genomic DNA is ensured, whereby better genomic coverage and amplification depth are provided. After primer annealing thermal program, the reaction mixture is placed in a thermal program that enables elongation of the first type of primer that binds to a single-strand DNA template under the action of the nucleic acid polymerase, to produce an amplification product (step (b3)).

The elongation temperature is usually related to the optimum temperature for DNA polymerase, for which those skilled in the art can make specific selection according to specific reaction mixture. In certain embodiments, the DNA polymerase in the reaction mixture may have strand-displacement activity, such that if during elongation, the primer encounters a primer or amplicon that binds to the downstream template, the strand-displacement activity of the DNA polymerase can enable separation of the downstream-binding primer from the template strand, thereby ensuring that the elongating primer continues to elongate, so that longer amplification sequences are obtained. DNA polymerases with strand-displacement activity include, but are not limited to, e.g., phi29 DNA polymerase, T5 DNA polymerase, SEQUENASE 1.0 and SEQUENASE 2.0. In certain embodiments, the DNA polymerase in the reaction mixture is a thermostable DNA polymerase. Thermostable DNA polymerases include, but are not limited to, e.g., Taq DNA polymerase, OmniBase^{™} Sequence enzyme, Pfu DNA polymerase, TaqBead^{™} Hot Start polymerase, Vent DNA polymerase (e.g., *Thermococcus litoralis* Vent polymerase, Deep Vent polymerase, Vent (-exo) polymerase and Deep Vent (-exo) polymerase), Tub DNA polymerase, TaqPlus DNA polymerase, Tfl DNA polymerase, Tli DNA polymerase, and Tth DNA polymerase. In certain embodiments, the DNA polymerase in the reaction mixture may be a DNA polymerase that is thermostable and has strand-displacement activity. In certain embodiments, the DNA polymerase in the reaction mixture is selected from the group consisting of: one or more of Phi29 DNA polymerase, Bst DNA polymerase, Pyrophage 3137, Vent polymerase (e.g., *Thermococcus litoralis* Therm polymerase, Deep Vent polymerase, Vent(-exo) polymerase, Deep Vent(-exo) polymerase), TOPOTaq DNA polymerase, 9° Nm polymerase, Klenow Fragment DNA polymerase I, MMLV reverse transcriptase, AMV reverse transcriptase, HIV reverse transcriptase, T7 phase DNA polymerase variant (lacking 3'-5' exonuclease activity), Phusion^{®} High-Fidelity DNA polymerase, Taq polymerase, Bst DNA polymerase (full length), E. coli DNA polymerase, LongAmp Taq DNA polymerase, OneTaq DNA polymerase.

In certain embodiments, step (b3) comprises reaction at an elongation temperature between 60-90°C (e.g., 65-90°C, 70-90°C, 75-90°C, 80-90°C, 60-85°C, 60-80°C, 60-75°C, 70-80°C, or at 60, 61, 62, 63, 64, 65, 66, 67, 68, 69, 70, 71, 72, 73, 74, 75°C) for 10 s-15 minutes (e.g., 1-14, 1-13, 1-12, 1-11, 1-10, 1-9, 1-8, 1-7, 1-6, 1-5, 1-4, 1-3, 1-2, 2-14, 3-14, 5-14, 6-14, 7-14, 8-14, 9-14, 10-14, 11-14, 12-14, or 13-14 minutes, or 10-60, 10-50, 10-40, 10-30, 10-20, 20-60, 20-50, 20-40, 20-30, 30-60, 30-50, 30-40 s). In certain embodiments, step (b3) comprises reaction at one or more temperatures between 60-80°C for 30 s-2 minutes. In certain embodiments, step (b3) comprises reaction at 65°C for 40 s. In certain embodiments, step (b3) comprises reaction at 75°C for 40 s. In certain embodiments, step (b3) comprises reaction at 65°C for 40 s, and then reaction at 75°C for 40 s.

After primer extension program, steps (b1) to (b3) are repeated up to a designated first cycle number, and as described above, in subsequent cycles the melting temperature in step (b1) is close to that in the first cycle, but maybe with a slightly shorter reaction time. In certain embodiments, step (b1) in cycles following the first cycle comprises reacting at a temperature between 90-95°C for 10-50 seconds.

The first cycle number of the present application is at least 2. In the first cycle, the sequence at 3' end of the variable sequence of the first type of primer is elongated, and the obtained amplification product has a common sequence at its 5' end and a complementary sequence of the genomic template single-strand sequence at its 3' end; such amplification products are also known as semi-amplicon. In the second cycle, the previous semi-amplicons themselves can also serve as DNA templates to bind to the variable sequences in the first type of primers. The primer extends toward 5' end of the amplification product under the action of nucleic acid polymerase until replication of the common sequence at 5' end of the amplification product is completed, thereby obtaining a genomic amplification product having a common sequence at its 5' end and a complementary sequence of the common sequence at its 3 'end; such amplification product is also referred to as full-amplicon. The pre-amplification product of the present application mainly refers to a full-amplicon, having a common sequence at its 5' end and a complementary sequence of the common sequence at its 3' end.

In subsequent amplifications after the first cycle, DNA single strands in the reaction mixture contain not only original genomic DNA single strand, but also newly-synthesized DNA single strand obtained from amplification, wherein the original genomic DNA templates as well as semi-amplicons produced during initial amplification can both be used as new DNA templates, bind to primers and start a new cycle of DNA synthesis; however, a full-amplicon will form a hairpin structure by itself, since its ends comprise complementary sequences (a common sequence comprised at the 5' end, and a complementary sequence of the common sequence at the 3' end), and thereby it cannot serve again as a new DNA template in the next reaction cycle for a new cycle of DNA synthesis.

In certain embodiments, number of the first cycle is controlled within a suitable range to ensure obtaining sufficient pre-amplification products to be used for subsequent reactions without affecting reaction time of the entire process due to excessive number of cycles. In certain embodiments, a first cycle number is 2-40 cycles (e.g., 2-40, 4-40, 6-40, 8-40, 10-40, 12-40, 14-40, 16-40, 18-40, 20-40, 15-40, 20-40, 25-40, 30-40, 5-35, 10-35, 15-35, 20-35, 25-35, 30-35, 10-30, 15-30, 20-30, 25-30, 2-20, 2-18, 2-16, 2-14, 2-12, 2-10, 2-8, 2-6, 2-4, 4-20, 4-18, 4-16, 4-14, 4-12, 4-10, 4-8, 4-6, 6-20, 6-18, 6-16, 6-14, 6-12, 6-10, 6-8, 8-20, 8-18, 8-16, 8-14, 8-12, 8-10, 10-20, 10-18, 10-16, 10-14, 10-12, 12-20, 12-18, 12-16, 12-14, 14-20, 1-18, 14-16, 16-20, 16-18 and 18-20 cycles). For example, a first cycle number is at least 3, at least 4, at least 5, or at least 6, at least 7, at least 8, at least 9, or at least 10, at least 11, at least 12, at least 13, at least 14, at least 15, or at least 16, at least 17, at least 18, at least 19 or at least 20, or preferably no more than 8, no more than 9, no more than 10, no more than 11, or no more than 12, no more than 13, no more than 14, no more than 15, no more than 16, no more than 17, or no more than 18, no more than 19, no more than 20, no more than 21, no more than 22, no more than 23, no more than 24, or no more than 25, no more than 26, no more than 27, no more than 28, no more than 29, no more than 30, no more than 31, no more than 32, no more than 33, no more than 34, no more than 35, no more than 36, no more than 37, no more than 38, no more than 39 or no more than 40. If the number of the first cycle is too low, then less pre-amplification products are obtained; to obtain sufficient amplification products, it is necessary to increase cycle number in amplification step (d), which will reduce the accuracy of amplification results. However, if the number of the first cycle is too high, it would take too much time, resulting in excessively long reaction time for the entire process.

**In** certain embodiments, a step (b3') is further comprised after step (b3), wherein the reaction mixture is placed in a suitable thermal program, such that the 3' end and 5' end of the full-amplicon in the genomic pre-amplification product hybridize to form a loop structure. It was previously considered that step (b3') can protect ends of a full-amplicon, thereby avoiding head-to-end polymerization between two or among more full-amplicons, thereby avoiding binding of two original non-adjacent sequences in a genome. This will help improve the accuracy of amplification result.

**In** certain embodiments, the method directly proceeds to subsequent step (b1) or (c) after step (b3), without undergoing other steps (e.g., step (b3')). In this way, full-amplicons have not been subject to particular steps avoiding head-to-end polymerization, and thus, theoretically, results of such amplification should be somewhat defective with regard to accuracy. However, unexpectedly, in the method of the present application, even without a particular step after step (b3), which enables full-amplicons to loop, the final amplification result still has considerably high accuracy, which is comparable to the effect of the method which employs step (b3'). This simplifies reaction steps while still retains specificity of reactions.

### Step(c): providing a second reaction mixture

**In** step (c), a second reaction mixture comprises the pre-amplification product obtained in step (b), a second primer, a mixture of nucleotide monomers and a nucleic acid polymerase, and the second primer comprises, in a 5' to 3' orientation, a specific sequence and the common sequence. The common sequence is substantially not complementary to a genomic sequence, therefore a second type of primer will not directly pair with the genomic DNA nor initiate replication of genomic DNA, if the other parts of the second type of primer are designed to be substantially not complementary to the genomic sequence, and thus in some specific embodiments, a second reaction mixture can be obtained by adding a second primer directly to the reaction mixture obtained after completion of step (b). In some other embodiments, the reaction mixture obtained after completion of step (b) is purified prior to step (c), to obtain a purified pre-amplification product, which is then mixed with a second primer, a mixture of nucleotide monomers, and a nucleic acid polymerase, and optionally, any other reagents known in the art useful in an amplification reaction, to obtain a second reaction mixture.

### i. Second type of primer

The "second primer" used herein belongs to the second type of primer described above. A second type of primer comprises a common sequence of a first type of primer, so that the second type of primer can bind to a complementary sequence of a common sequence at 3' end of a full-amplicon, thereby further replicating the full-amplicon and greatly increasing the amount thereof.

The second type of primer comprises or consists of, in a 5' to 3' orientation, a specific sequence and a common sequence. A second type of primer can be selected specifically based on different sequencing platforms. In certain embodiments, the second type of primer can be selected specifically based on a second-generation sequencing platform. In certain embodiments, the second type of primer can be selected specifically based on Illumina's NGS sequencing platform (such as, but not limited to, Hiseq, Miseq, etc.) or Life technologies's Ion torrent NGS sequencing platform. In certain embodiments, the second type of primer comprises a sequence partially or completely complementary or identical to a primer used for sequencing. In certain embodiments, the sequence in a second type of primer, which is partially or completely complementary or identical to a primer used for sequencing as described above, comprises or consists of the common sequence.

The second primer of the present application may be a primer pair having structural characteristics of the second type of primers or single primers having the same structure and sequence. In some embodiments, the specific sequence of the second primer comprises at its 3' end a sequence complementary or identical to part or all of a primer used for sequencing. In some embodiments, the sequence complementary or identical to part or all of a primer used for sequencing comprised in the specific sequence of the second primer comprises or consists of SEQ ID NO: 31 [ACACTCTTTCCCTACACGAC], or SEQ ID NO: 32 [GTGACTGGAGTTCAGACGTGT]. In some embodiments, the specific sequence of the second primer further comprises at its 5' end a sequence complementary or identical to part or all of a capture sequence of a sequencing platform. A capture sequence refers to a sequence contained on a sequencing plate in a sequencing platform, which is used for capturing fragments to be sequenced. In some embodiments, the sequence complementary or identical to part or all of a capture sequence of a sequencing platform comprised in the specific sequence of the second primer comprises or consists of SEQ ID NO: 33 [AATGATACGGCGACCACCGAGATCT], or SEQ ID NO: 34 [CAAGCAGAAGACGGCATACGAGAT]. In some embodiments, the specific sequence of the second primer further comprises a segment of barcode sequence between the sequence complementary or identical to part or all of a capture sequence of a sequencing platform and the sequence complementary or identical to part or all of a primer used for sequencing, wherein said barcode sequence refers to a sequence used to identify a specific set of fragments to be sequenced. When a sequencing platform performs sequencing for multiple sets of fragments to be sequenced at the same time, sequencing data can be differentiated by screening sequencing results for barcode sequence that each set harbors.

In some embodiments, the second primer is a primer pair comprising having the same common sequence and different specific sequences, wherein the different specific sequences comprise a sequence complementary or identical to part or all of a pair of capture sequences used in the same sequencing platform, respectively, and/or the different specific sequences comprise a specific sequence complementary or identical to different primers in a sequencing primer pair used during the same sequencing. In some embodiments, the second primer comprises a mixture of sequences set forth in SEQ ID NO: 35 [AATGATACGGCGACCACCGAGATCTACACTCTTTCCCTACACGAC GCTCTTCCGATCT] and [CAAGCAGAAGACGGCATACGAGATX...XGTGACTGGAGTTCAGACGTGTGCTCT TCCGATCT], wherein X...X is a barcode sequence, the length and specific sequence of which can be selected by those skilled in the art based on practical need. In some embodiments, the second primer comprises a mixture of sequences set forth in SEQ ID NO: 35 [AATGATACGGCGACCACCGAGATCTACACTCTTTCCCTACACGAC GCTCTTCCGATCT], SEQ ID NO: 36 [CAAGCAGAAGACGGCATACGAGATCGTGATGTGACTGGAGTTCAGACGTGTGCT CTTCCGATCT]. In some embodiments, the second primer comprises a mixture of sequences set forth in SEQ ID NO: 37 [CCACTACGCCTCCGCTTTCCTCTCTATGGGCAGTCGGTGATGCTCTTCCGATCT] and SEQ ID NO: 38 [CCATCTCATCCCTGCGTGTCTCCGACTCAGCTAAGGTAACGATGCTCTTCCGATC T]. In some embodiments, the second primer comprises a mixture of sequences set forth in SEQ ID NO: 39 [CCACTACGCCTCCGCTTTCCTCTCTATGGGCAGTCGGTGATTTGGTAGTGAGTG] and SEQ ID NO: 40 [CCATCTCATCCCTGCGTGTCTCCGACTCAGCTAAGGTAACGATTTGGTAGTGAGT G].

In some embodiments, the concentration of the second primer in the second reaction mixture is 1-15 ng/µL. In some embodiments, the concentration of the second primer in the second reaction mixture is 1-12 ng/µL, 1-10 ng/µL, 1-8 ng/µL, 1-7 ng/µL, 1-6 ng/µL, 1-5 ng/µL, 1-4 ng/µL, 2-3 ng/µL, 2-12 ng/µL, 2-10 ng/µL, 2-8 ng/µL, 2-6 ng/µL, 2-5 ng/µL, 2-4 ng/µL, 2-3 ng/µL, 3-12 ng/µL, 3-10ng/µL, 3-8ng/µL, 3-6 ng/µL or 3-4 ng/µL. In some embodiments, the concentration of the second primer in a second reaction mixture is 2-3ng/µL. In some embodiments, the second primer in the second reaction mixture is 5-50 pmol. In some embodiments, the second primer in the second reaction mixture is 10 pmol, 15 pmol or 20 pmol.

### ii. Other components

In certain embodiments, the nucleic acid polymerase contained in the second reaction mixture is one or more selected from the group consisting of *Thermococcus litoralis* Vent polymerase, Deep Vent polymerase, Vent(-exo) polymerase, or Deep Vent (-exo) polymerase. In certain embodiments, the second reaction mixture contains *Thermococcus litoralis* Vent polymerase. In certain embodiments, the second reaction mixture contains Deep Vent polymerase. In certain embodiments, the second reaction mixture contains Vent(-exo) polymerase. In certain embodiments, the second reaction mixture contains Deep Vent(-exo) polymerase. The various polymerases in the present application are all commercially available, for example, from New England Biolabs Co..

In certain embodiments, the second reaction mixture can further comprise suitable metal ions which the nucleic acid polymerase requires to exert its enzymatic activity (e.g., Mg²⁺ ions at a suitable concentration (e.g., the final concentration of which may be about 1.5 mM to about 8 mM); a mixture of nucleotide monomers (e.g., dATP, dGTP, dTTP and dCTP), bovine serum albumin (BSA), dTT (e.g., the final concentration of which may be about 2 mM to about 7 mM), purified water, suitable buffer component (e.g., pH regulator such as Tris HCl and Tris SO₄) or one or more other components commonly used in the art (such as DNase inhibitor, RNase, SO₄²⁻, Cl⁻, K⁺, Ca²⁺, Na⁺, and/or (NH₄)⁻, etc), and the like.

### Step(d): placing in a second thermal cycle program

The method provided herein further comprises step (d): placing the second reaction mixture obtained from step (c) in a second thermal cycle program such that the common sequence of the second type of primer can pair with 3' end of the genomic amplification product and the genomic pre-amplification product is amplified to obtain an expanded genomic amplification product.

Since the genomic pre-amplification product obtained from step (b), i.e. the full amplicon, has a complementary sequence to the common sequence at 3' end, it can be complementary to the common sequence of the second type of primer; under the action of nucleic acid polymerase, the second type of primer extends and full length of the full amplicon is replicated.

In the second thermal cycle program, the reaction mixture is first placed in a thermal program capable of opening DNA double strands (step (d1)). The DNA double strands herein mainly refers to the double strands of genomic pre-amplification product (i.e., full amplicon) obtained from step (b) (including single-strand hairpin structure molecule of full-amplicon). Although original genomic DNA still exists in the second reaction mixture at this point, the original genomic DNA is not DNA template to be amplified in step (c), since the second type of primer substantially does not bind to the genomic DNA. A higher reaction temperature such as 90°C-95°C can be used for reaction for a suitable period, such that the double-strand/hairpin structure of the full-amplicon to be amplified can denature into linear single strands. In certain embodiments, in the thermal program in step (dl), the reaction mixture is placed under a temperature capable of opening DNA double-strand and allowed reacting for sufficient time, to ensure that all template DNA double-strands or hairpin structures denature into single strands, said thermal program comprises reaction at a denaturing temperature between 90-95°C (such as 95°C) for 5 s-20 min (such as 30 s or 30 min). After step (dl), the reaction mixture is placed in a thermal program which enables that double strands of amplification product produced during the x^{th} amplification cycle (x is an integer≥1) comprised therein denature into single-strand templates (step (d2)), i.e., reaction at a melting temperature between 90-95°C (such as 95°C) for 3-50 s (such as 20 s). It should be understood that step (d2) is not essential in the first cycle, but as denaturation and melting programs use similar temperatures, and melting time is very short as compared to denaturation time, it can be considered that step (d2) is prolongation of step (d1) in the first cycle.

After step (d2), the reaction mixture is placed in a thermal program that enables binding of the second type of primer to DNA single-strands obtained from step (d1) or (d2). On the basis of base composition in the second type of primer, Tm value of the second type of primer can be calculated and a suitable annealing temperature for the second type of primer can be determined based on this Tm value. In certain embodiments, thermal program in step (c2) comprises reaction for 3-50 s (e.g., 40 s) at an annealing temperature between 45-65°C (e.g., 63°C). In certain embodiments, the second type of primer is a mixture of SEQ ID NO: 35 and SEQ ID NO: 36 and the thermal program in step (d3) comprises reaction for 3-50 s at 63°C. In certain embodiments, the annealing temperature in step (d3) is higher than that in step (b2). In step (d3), the reaction mixture may still contain the first type of primer that did not undergo reaction in step (b), and variable sequences of these first type of primers may pair with the DNA single-strand templates obtained from step (d3), resulting in incomplete amplification sequences. When annealing temperature in step (d3) is higher than that suitable for the first type of primer, binding of the first type of primer with single-strand DNA template can be reduced or avoided, thereby selectively allowing amplification by the second type of primer.

After completion of primer annealing, the reaction mixture is placed in a thermal program that enables elongation of the second type of primer that binds to single strands of the amplification product, under the action of the nucleic acid polymerase. In certain embodiments, the thermal program in step (d4) comprises reaction for 10 s-15 minutes (e.g., 40 s or 3 minutes) at an elongation temperature between 60-80°C (e.g., 72°C).

Steps (d2) to (d4) can be repeated to a second cycle number to obtain the desired expanded genomic amplification product. During this process, the genomic amplification product obtained in step (b) is further replicated and amplified, the number of which is greatly increased, in order to provide sufficient genomic DNA sequences for subsequent studies or operations. In certain embodiments, the second cycle number in the step (d5) is greater than the first cycle number in the step (b4). In certain embodiments, the second cycle number is controlled within a suitable range such that it can provide sufficient amount of DNA without compromising the accuracy of amplification due to excessive number of cycles. In certain embodiments, the second cycle number is 2-40 cycles (e.g., 2-40, 4-40, 6-40, 8-40, 10-40, 12-40, 14-40, 16-40, 18-40, 20-40, 15-40, 20-40, 25-40, 30-40, 5-35, 10-35, 15-35, 20-35, 25-35, 30-35, 10-30, 15-30, 20-30, 25-30, 15-28, 15-26, 15-24, 15-22, 15-20, 15-18, 15-17, 16-30, 17-30, 18-30, 20-30, 22-30, 24-30, 26-30, 28-30, 32-40, 32-38, 32-36 or 32-34 cycles).

In certain embodiments, step (d) further comprises placing the reaction mixture in the same thermal program as that in step (d4) (e.g. 72°C) and allowing reaction for a suitable period (e.g., 40 s) after the second thermal cycle program. The reaction mixture is then placed at a temperature of 4°C to terminate reaction. In certain embodiments, the reaction mixture is placed at a temperature of 4°C to terminate reaction directly after completion of the reaction of step (d).

In certain embodiments, genomic DNA in the reaction mixture in step (a) is present within a cell, i.e., the reaction mixture contain cells in wheih the genomic DNA to be amplified is contained. In certain embodiments, the reaction mixture in step (a) contains celsl and further comprises components capable of lysing cells, such as surfactant and/or lyase, etc. Suitable surfactants, such as one or more of NP-40, Tween, SDS, TritonX-100, EDTA, and guanidine isothiocyanate, can be used. Suitable lyases, such as one or more of Protease K, pepsin and papain, can also be selected. In such embodiments, the method of amplifying cell genome as described above further comprises placing a reaction mixture in a lysing thermal cycle program after step (a) and prior to step (b) (e.g., placing a reaction mixture at 50°C for 20 minutes, then at 80°C for 10 minutes), to allow lysing of the cell and release of the genomic DNA.

### Application

In certain embodiments, the product obtained by amplification using the method of the present application can be further used for sequencing, such as for whole genome sequencing. Due to the high requirement on initial amount of samples to be analyzed (more than 100 ng) by various sequencing analysis platforms such as Next Generation Sequencing (NGS), Microarray, and fluorescent quantitative PCR, etc., whole genome amplification is needed if sufficient nucleic acid material for analysis need to be obtained from a single human cell (about 6pg) or a sample in a small initial amount. Genomic DNA in a biological sample (e.g., a single cell) can be amplified by the method of the present application, and the product obtained from amplification can be sequenced by a suitable sequencing method in the art. Exemplary sequencing methods include, sequencing by hybridization (SBH), sequencing by ligase (SBL), quantitative incremental fluorescent nucleotide addition sequencing (QIFNAS), stepwise ligation and cleavage, molecular beacons, pyrosequencing, fluorescent in situ sequencing (FISSEQ), fluorescence resonance energy transfer (FRET), multiplex sequencing (U.S. Patent Application No. 12/027039; porreca et al. (2007) NAT. Methods 4:931), polymerized colony (POLONY) sequencing (U.S. 6,432,360, U.S. 6,485,944 and PCT/US05/06425); swing sequencing (PCT US05/27695), TaqMan reporter probe digestion, nanogrid rolling circle sequencing (ROLONY) (U.S. Patent Application No. 12/120541), FISSEQ beads (U.S. 7,425,431), and allele-specific oligonucleotide ligation assay, etc.

In certain embodiments, sequencing of amplification products of the method herein can be accomplished by high-throughput method. High-throughput methods typically fragmentize nucleic acid molecules to be sequenced (e.g., by means of enzymatic cleavage or mechanical shearing, etc.), to form large amount of short fragments ranging from tens to hundreds of bp in length. By sequencing tens of thousands, hundreds of thousands, millions, tens of millions, or even hundreds of millions of such short fragments in parallel in one sequencing reaction, throughput of sequencing can be greatly increased and time required for sequencing can be shortened. The measured sequences of short fragments can be joined into a complete sequence after data processing via software. A variety of high-throughput sequencing platforms are known in the art, such as Roche 454, Illumina Solexa, AB-SOLiD, Helicos, and Polonator platform technology, and the like. A variety of light-based sequencing techniques are also known in the art, see, e.g., those described in Landegren et al. (1998) Genome Res. 8: 769-76, Kwok (2000) Pharmacogenomics 1: 95- 100, and Shi (2001) Clin. Chem. 47: 164- 172.

In certain embodiments, products obtained from amplification using the method of the present application can also be used to analyze genotypes or genetic polymorphisms in genomic DNA, such as single nucleotide polymorphism (SNP) analysis, short tandem repeat (STR) analysis, restriction fragment length polymorphism (RFLP) analysis, variable number of tandem repeats (VNTRs) analysis, complex tandem repeat (CTR) analysis, or microsatellite analysis and the like, see, e.g., Krebs, J.E., Goldstein, E.S. and Kilpatrick, S.T. (2009). Lewin's Genes X (Jones & Bartlett Publishers) for reference.

In certain embodiments, amplification products obtained by the method of the present application can also be used for medical and/or diagnostic analysis. For example, a biological sample from an individual may be amplified using the method of the present application, and whether abnormalities such as mutations, deletions, insertions or fusion between chromosomes are present in gene or DNA sequence of interest in the amplification product can be analyzed, whereby to evaluate the risk of developing certain disease for the individual, the progression stage, genotyping and severity of the disease, or the likelihood that the individual respond to certain therapy. The gene or DNA sequence of interest can be analyzed using suitable methods known in the art, such as, but not limited to, nucleic acid probe hybridization, primer-specific amplification, sequencing a sequence of interest, single-stranded conformational polymorphism (SSCP), etc.

In certain embodiments, the methods of the present application can be used to compare genomes derived from different single cells, in particular different single cells from the same individual. For example, when differences exist between genomes of different single cells of the same individual, such as between tumor cells and normal cells, genomic DNA of different single cells can be amplified separately using the method herein, and the amplification product can be further analyzed, for example, analyzed and compared by sequencing, or subject to comparative genomic hybridization (CGH) analysis. See, Fan, H.C., Wang, J., Potanina, A., and Quake, S.R. (2011). Whole-genome molecular haplotyping of single cells. Nature Biotechnology 29, 51-57., and Navin, N., Kendall, J., Troge, J., Andrews, P., Rodgers, L., McIndoo, J., Cook, K., Stepansky, A., Levy, D., Esposito, D., et al. (2011). Tumour evolution inferred by single-cell sequencing. Nature 472, 90-94, for reference.

In certain embodiments, the methods of the present application can be used to identify haploid structures or haploid genotypes in homologous chromosomes. Haploid genotype refers to the combination of alleles at multiple loci that are co-inherited on chromosome of the same haplotype. A biological sample (e.g., a single cell from an individual's diploid) can be divided into enough portions so that DNA sequences on two homologous haplotypes are statistically separated into different portions. Each section is assigned as one reaction mixture, and each reaction mixture is subjected to DNA amplification by the method of the present application, and then the amplification product is subjected to sequence analysis and is aligned with a reference genomic sequence (e.g., publically available standard genomic sequence of humans, see, International Human Genome Sequencing Consortium, Nature 431, 931-945 (2004)), to identify single nucleotide mutations therein. If no reference genome sequence is readily available, a region of suitable length assembled from multiple fragment sequences of genome by means of de-novo genome assembly can also be used for comparison.

In certain embodiments, products obtained from amplification using the method of the present application can be further used for analysis such as gene cloning, fluorescence quantitative PCR and the like.

In certain embodiments, the method of the present application can also further comprise analyzing the amplification product to identify disease- or phenotype-associated sequence features. In some embodiments, analyzing the amplification product comprises genotyping of DNA amplicon. In some other embodiments, analyzing the amplification product includes identifying polymorphism of DNA amplicons, such as single nucleotide polymorphism (SNP) analysis. SNP can be detected by some well-known methods such as oligonucleotide ligation assay (OLA), single base extension, allele-specific primer extension, mismatch hybridization and the like. A disease can be diagnosed by comparison of SNP to those of known disease phenotypes.

In some embodiments, the disease- or phenotype-associated sequence features include chromosomal abnormalities, chromosomal translocation, aneuploidy, deletion or duplication of a part of or all chromosomes, fetal HLA haplotypes and paternal mutations.

In some embodiments, the disease or phenotype may be beta-thalassemia, Down's syndrome, cystic fibrosis, sickle cell disease, Tay-Sachs disease, Fragile X syndrome, spinal muscular atrophy, hemoglobinopathy, Alpha-thalassemia, X-linked diseases (diseases dominated by genes on the X chromosome), spinal bifida, anencephaly, congenital heart disease, obesity, diabetes, cancer, fetal sex, and fetal RHD.

### Kit

Another aspect of the application also provides a kit for genomic DNA amplification, wherein the kit comprises primers consisting of a first primer and a third primer as defined in claim 14 at the same time. In certain embodiments, the kit further comprises a nucleic acid polymerase, wherein the nucleic acid polymerase is selected from the group consisting of: Phi29 DNA polymerase, Bst DNA polymerase, Pyrophage 3137, Vent polymerase, TOPOTaq DNA polymerase, 9° Nm polymerase, Klenow Fragment DNA polymerase I, MMLV reverse transcriptase, AMV reverse transcriptase, HIV reverse transcriptase, T7 phase DNA polymerase variant, Phusion^{®} High-Fidelity DNA polymerase, Taq polymerase, Bst DNApolymerase, E.coli DNA polymerase, LongAmp Taq DNA polymerase, OneTaq DNA polymerase, Deep Vent DNA polymerase, Vent (exo-)DNA polymerase, Deep Vent (exo-)DNA polymerase, and any combination thereof. In certain embodiments, a kit further comprises one or more components selected from the group consisting of: a mixture of nucleotide monomers (e.g., dATP, dGTP, dTTP, and dCTP, e.g., with an overall concentration between 1 mmol-8 mmol/µL), dTT (e.g., with a concentration between 1 mmol-7 mmol/µL) and Mg²⁺ solution (e.g., with a concentration between 2 mmol-8 mmol/µL), bovine serum albumin (BSA), a pH-regulator (e.g., Tris HCl), DNase inhibitor, RNase, SO₄²⁻, Cl⁻, K⁺, Ca²⁺, Na⁺, and/or (NH₄)⁺. In certain embodiments, the kit further comprises a component capable of lysing a cell, such as one or more surfactants (e.g., NP-40, Tween, SDS, Triton X-100, EDTA, and guanidinium isothiocyanate), and/or one or more lyases (e.g. Protease K, pepsin, and papain). In some embodiments, the kit further comprises a second type of primer (i.e., a second primer). It should be understood that the first primer, the second primer and the third primer in the kit all have the structure and the sequence feature specifically described above.

In some embodiments, all components in the kit are stored separately in separate containers. In some embodiments, all components in the kit are stored together in a same container. In some embodiments, each type of primer in the kit was stored in separate containers, while all other components other than primers are all stored in a same container. When the kit comprises a nucleic acid polymerase, the nucleic acid polymerase can be stored in substantially pure form in a separate container, or optionally, form a mixture with other components.

In some embodiments, the kit may comprise a mixture containing all reactants required for a linear amplification reaction, except for genomic DNA. When such kit is used for the linear amplification reaction described herein, a genomic DNA-containing sample can be mixed with a mixture in a kit directly, and, optionally, an appropriate amount of pure water may be added to obtain a desired reaction volume, by which the first reaction mixture in the step (a) of the method of the present application can be obtained. In some embodiments, the kit may comprise a mixture containing all reactants required for an exponential amplification reaction, except for amplification templates. When such kit is used for the exponential amplification reaction described herein, DNA template samples contained in the amplification product of step (b) can be mixed with the mixture in the kit directly, and, optionally, an appropriate amount of pure water may be added to obtain a desired reaction volume, by which the second reaction mixture in the step (c) of the method of the present application can be obtained. In some embodiments, the kit can comprise both a mixture containing all reactants required for an linear amplification reaction, except for an amplification template, and a mixture containing all reactants required for an exponential amplification reaction, except for amplification templates, said mixture above may be separated as two, or a mixed as one.

Further disclosed but not claimed is a kit for genomic DNA amplification, wherein said kit comprises a first type of primer (e.g., a first primer and/or a third primer) and a second type of primer (e.g., a second primer), and further comprises an instruction for users, said instruction records the step of mixing primers and other components to obtain a mixture of first/third primers, before said amplification. In other embodiments, the instruction also records how to carry out the amplification of the present application. The first type of primer and the second type of primer in the kit may be placed separately in different containers, but the instruction may include the step of mixing the two in the same container before the amplification starts.

### EXAMPLES

### Example 1: Preliminary validation of effect of amplification using different mixtures of linear amplification primers

### a) Validation using a standard genomic DNA sample

A standard genomic DNA is a pre-extracted genomic DNA of a human cell. The standard genomic DNA was diluted with nuclease-free water to a DNA solution of 50 pg/µl, and 1 µl of the above solution (as a source of genomic DNA) was added into a PCR tube. In each experimental group, a primer mixture as shown in Table 1, as well as other relevant reagents were added, to generate a first reaction mixture (which contained Na⁺, Mg²⁺, Cl⁻, Tris-Cl, TritonX-100, dNTP, Vent polymerase and a primer mixture).

### Linear amplification

Each group of primer combination/mixture was subject to parallel experiments using two experimental groups to ensure the accuracy thereof, and the reaction mixture of each experimental group was placed in the following first thermal control program for reaction:

| | | |
|---|---|---|
| Repeated for 12 cycles | 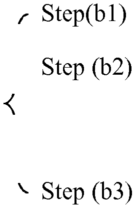 | First cycle: 95°C for 3min / subsequent cycles: 95°C for 15s |
| | | 15°C for 50s |
| | | 25°C for 40s |
| | | 35°C for 30s |
| | | 65°C for 40s |
| | | 75°C for 40s |

Primer mixture used in each experimental group is shown in Table 1 below,

**Table 1: Primers used in each experimental group**

| Experimental groups | Primer (or primer mixture) used |
|---|---|
| 1-2 | common sequence+NNNNNTTT and common sequence+NNNNNGGG |
| 3-4 | common sequence+NNNNNGTTT and common sequence+NNNNNTGGG |
| 5-6 | SEQ ID NO: 7 and SEQ ID NO: 11 |
| 7-8 | SEQ ID NO: 8 and SEQ ID NO: 12 |
| 9-10 | SEQ ID NO: 7, SEQ ID NO: 8, SEQ ID NO: 11 and SEQ ID NO: 12 |
| 11-12 | common sequence+KKKKK |

wherein in each experimental group, the total amount of the first type of primer used was 600 pmol (if multiple types of primers were comprised therein, then the total amount was 600 pmol and the amount of each type of primer is equal). Different common sequences can be designed according to differences among sequencing platforms. In groups 1-12 of this experiment, SEQ ID NO: 6 was chosen as the common sequence based on the Illumina platform.

After the linear amplification program, 1 µl of second primer at 10 pmol/µl was added to each reaction system to generate 12 groups of second reaction mixture, and the reaction mixture of each experimental group was placed in the following second thermal-control program for reaction:

| | |
|---|---|
| | Step (d1) 95°C for 30s |
| 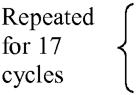 | Step (d2) 95°C for 20s |
| | Step (d3) 63°C for 40s |
| | Step (d4) 72°C for 40s |

Different second primer sequences can be designed according to differences among sequencing platforms. In groups 1-12 of this experiment, the following mixture of second primers was used based on the Illumina platform: 
Second primer-1 (SEQ ID NO：35)：AAT GAT ACG GCG ACC ACC GAG ATC
TAC ACT CTT TCC CTA CAC GAC GCT CTT CCG ATCT
 
Second primer -2 (SEQ ID NO：36)：CAA GCA GAA GAC GGC ATA CGA GAT
CGT GAT GTG ACT GGA GTT CAG ACG TGT GCT CTT CCG ATCT
 wherein in the second primers, the bases marked with double-underline comprise the part corresponding to the capture sequence of the sequencing platform, the bases marked in italics represent the part corresponding to the sequencing primer of the sequencing platform, the part marked by dots is the barcode sequence part, which can be replaced by other barcode sequences as needed, and the part marked with single underline is the common sequence part.

In each experimental group, an amplification product was obtained after completion of the amplification thermal control program as described above.

### Gel electrophoresis (qualitative)

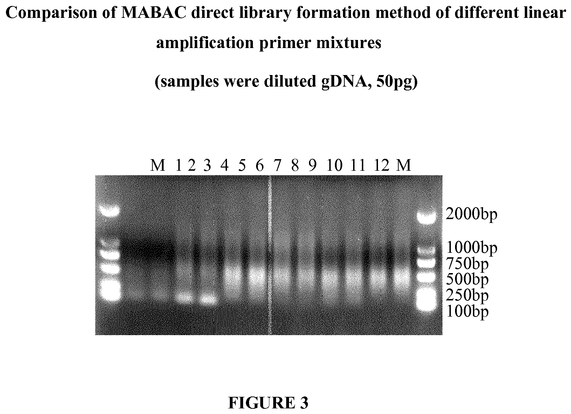

5 microliters of unpurified amplification product of each experimental group in Example 1 were taken respectively, and were respectively added with 1 µl of 6x DNA loading buffer (purchased from Beijing ComWin Biotech Co., Ltd., Cat. No. CW0610A) for sample loading. 1% agarose gel was used as the gel, and DM2000 (purchased from Beijing ComWin Biotech Co., Ltd., Cat. No. CW0632C) was used as the marker. The electrophoresis image is shown in Figure 3, in which from left to right, lane 1 is molecular weight marker, lanes 2-13 are amplified samples of the genomic DNA, and lane 14 is molecular weight marker.

As shown in the electrophoresis image in Figure 3, products of experimental groups 1-4 exhibit an obvious band around 100bp, but a relatively low amount of product between 100-500bp; products of other experimental groups 5-12 were mainly bands around 500bp, and experimental groups 5-10 exhibit an unclear band around 100 bp, and the concentration of which is considerably low compared to experimental groups 1-4. It can be inferred from Figure 3 that there were more primer polymers in experimental groups 1-4, and the reaction efficiency of which was relatively low, and moreover, when the fixed sequence comprised in the variable sequence of a first type of primer is TGGG or GTTT (experimental groups 3-4), the reaction efficiency was higher than a primer whose fixed sequence is GGG or TTT (experimental groups 1-2). There was little difference in amplification products among experimental groups 5-12, but they were significantly higher as compared to experimental groups 1-4, indicating a lower degree of primer polymer generation in groups 5-12 than in groups 1-4.

### Purification of products (quantitative)

50 microliters of unpurified amplification products were taken, and the amplification products were subject to purification treatment using a magnetic-bead DNA purification and recovery kit (purchased from Beijing ComWin Biotech Co., Ltd., Cat. No. CW2508) following purification protocols in accordance with the kit instructions. 20 microliters of EB were used for elution. After purification was completed, 2 µl of purified product was taken, the concentration of which was measured using Nanodrop (AOSHENG, NANO-100). Results of concentration measurement are shown in Table 2.

**Table 2: Concentrations of samples in Figure 3 after recovery**

| Experimental group | Concentration (ng/µl) | Volume (µl) |
|---|---|---|
| Experimental group 1 | 16.647 | 20 |
| Experimental group 2 | 20.158 | 20 |
| Experimental group 3 | 82.085 | 20 |
| Experimental group 4 | 79.712 | 20 |
| Experimental group 5 | 130.52 | 20 |
| Experimental group 6 | 130.024 | 20 |
| Experimental group 7 | 144.456 | 20 |
| Experimental group 8 | 128.041 | 20 |
| Experimental group 9 | 118.145 | 20 |
| Experimental group 10 | 101.04 | 20 |
| Experimental group 11 | 126.059 | 20 |
| Experimental group 12 | 130.079 | 20 |

Amplification efficiency of each experimental group was estimated according to concentration of obtained amplification products after purification. It can be inferred from the concentration measurement results shown in Table 2, amplification efficiency of groups 1-2 was the lowest, and amplification efficiency of experimental groups 3-4 was relatively lower as compared to experiment groups 5-12, but higher than that of experimental groups 1-2. The total amount of amplification products of experimental groups other than experimental groups 1-4 were comparable, with no significant difference.

### Sequencing (qualitative)

Purified amplification products of the 12 experimental groups as described above were taken, sequenced using Illumina's NGS sequencing platform hiseq2500 sequencer in a shallow-depth sequencing manner, and sequence obtained from sequencing was mapped to human reference genome.

Various indicating parameters of high-throughput sequencing results were provided in Table 3.

Among the various indicating parameters in Table 3, the unique mapped ratio of raw data (unique_mapped_of_raw, i.e., the ratio of data that can be mapped to a unique position of human genome) is the most important measuring index. As shown by data in Table 3, the unique_mapped_of_raws in experimental groups 5-12 were between 83%-86%, with little difference among groups, but the unique_mapped_of_raw in experimental groups 1-4 were relatively lower, being between 67-79%.

Another important indicating parameter is the mapped ratio of raw data (mapped_of_raw, i.e., the ratio of data that can be mapped to certain position of human genome). Similarly, as shown by data in Table 3, the mapped_of_raws in experimental groups 5-12 were between 89%-93%, with little difference among groups, but the mapped_of_raws in experimental groups 1-4 were relatively lower, being between 73-86%.

In addition, data in Table 3 further show that the data quality of reads of experimental groups 1-4 was also lower than that of other experimental groups. For example, the proportions of high-quality data in raw data of experimental groups 3 and 4 were only 77.08% and 76.99%, whereas the proportions of high-quality data in experimental groups 5-12 were between 94%-96%.

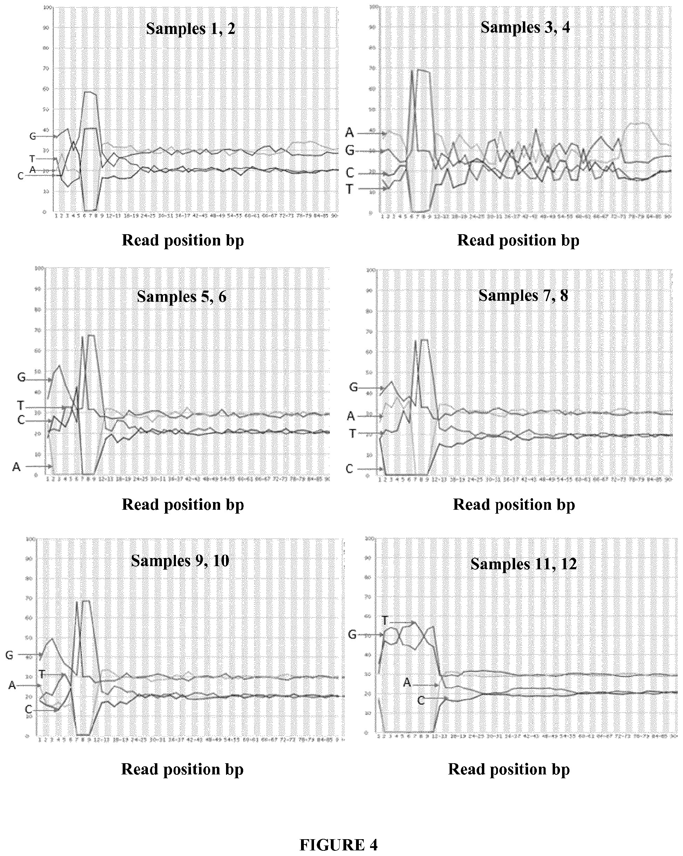

Figure 4 shows each nucleotide read at sequence read starting position in a sequencing library. It can be inferred from Figure 4 that the read starting regions in experimental groups 1-4 and 9-10 comprised four types of bases, A, T, C and G. The read starting regions in experimental groups 5-7 lacked A or C, the read starting regions in experimental groups 11-12 lacked A and C. It will be appreciated by those skilled in the art that, during sequencing, especially during SBS sequencing, it is required that the first several bases for sequencing have high randomness. Where randomness of the first several bases of the entire sample for sequencing is low, certain amount of positive quality-control sample need to be added into each loading well during whole-plate loading to increase base randomness, but this will inevitably result in a waste of some data amount. For example, when the libraries prepared in experimental groups 11-12 were used for whole-plate loading and sequencing, a certain amount of positive quality-control sample need to be added due to lack of A and C in read starting regions, and according to experimental experience, generally, addition of positive quality-control sample in an amount of at least 20% of loading amount is required in order to ensure that SBS sequencing proceeds well.

**Table 3: Major quality indices in high-throughput sequencing results of experimental groups 1-12**

| **Experi mental group** | **Raw data** | **GC %** | **High-quality data** | **High-quality data in raw data (%)** | **Mapped data** | **Mapping rate (%)** | **Mapped data in raw data (%)** | **Unique mapped data** | **Unique mapped rate in raw data (%)** |
|---|---|---|---|---|---|---|---|---|---|
| **1** | **1,612,313** | **40** | **1,441,488** | **89.4** | **1,361,442** | **94.45** | **84.44** | **1,264,120** | **78.4** |
| **2** | **1,505,058** | **40** | **1,362,899** | **90.55** | **1,291,394** | **94.75** | **85.8** | **1,200,317** | **79.75** |
| **3** | **1.596,786** | **39** | **1,230,845** | **77.08** | **1,176,734** | **95.6** | **73.69** | **1,084,876** | **67.94** |
| **4** | **1,662,003** | **39** | **1,279,605** | **76.99** | **1,223,105** | **95.58** | **73.59** | **1,126,790** | **67.8** |
| **5** | **1,605,629** | **41** | **1,508,333** | **93.94** | **1,444,591** | **95.77** | **89.97** | **1,339,990** | **83.46** |
| **6** | **1,570,417** | **41** | **1,475,101** | **93.93** | **1,414,980** | **95.92** | **90.1** | **1,313,734** | **83.66** |
| **7** | **1,566,023** | **38** | **1,496,310** | **95.55** | **1,443,695** | **96.48** | **92.19** | **1,323,155** | **84.49** |
| **8** | **1,613,086** | **38** | **1,544,016** | **95.72** | **1,491,829** | **96.62** | **92.48** | **1,371,421** | **85.02** |
| **9** | **1,507,290** | **40** | **1,426,724** | **94.65** | **1,373,685** | **96.28** | **91.14** | **1,268,153** | **84.13** |
| **10** | **1,691,762** | **40** | **1,601,359** | **94.66** | **1,541,394** | **96.26** | **91.11** | **1,422,638** | **84.09** |
| **11** | **1,396,488** | **40** | **1,340,338** | **95.98** | **1,288.849** | **96.16** | **92.29** | **1,211.672** | **86.77** |
| **12** | **1,559,872** | **40** | **1,492,641** | **95.98** | **1,433,057** | **96.01** | **91.87** | **1,342,959** | **86.09** |

### b) Validation using AFP single-cell sample

The sample to be tested were AFP single cells. Cultured human epidermal fibroblasts (AFP) in a good state were digested with trypsin and the digested cells were collected into a 1.5 ml EP tube. The collected cells were centrifuged and rinsed with 1×PBS solution. After rinsing, 1×PBS was added to suspend the cells. A portion of the cell-containing suspension was aspirated using a pipette, and single cells were picked using a mouth pipette under a 10× microscope, the volume of aspirated PBS solution not exceeding 1 microliter, and the picked single cells were transferred into a PCR tube containing 5 microliters of lysis buffer (containing Tris-Cl, KCl, EDTA, Triton X-100 and Qiagen Protease). After brief centrifugation, the PCR tube was placed on a PCR instrument where a lysing program was performed, and the specific program is shown in Table 4.

**Table 4: Lysing program**

| cycle | Temperature (°C) | time |
|---|---|---|
| 1 | 75 | 10min |
| | 95 | 4min |
| | 22 | ∞ |

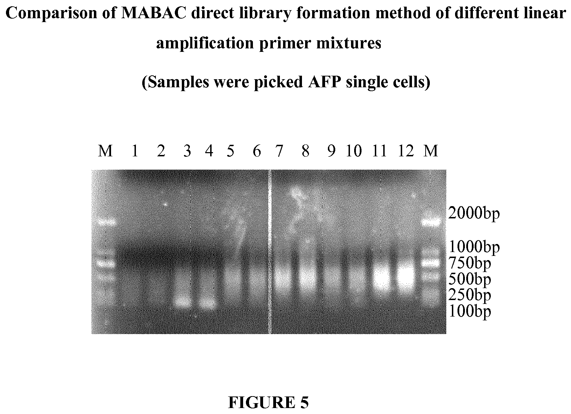

The reaction solution following lysis was used as a source of genomic DNA in place of the standard genomic DNA in Example 1a), while other components and thermal control programs for linear amplification and exponential amplification were the same as those in Example 1a). The obtained amplification product was subject to gel electrophoresis under conditions as described above. See Figure 5 for gel electrophoresis image, in which, from left to right, lane 1 is molecular weight marker, lanes 2-13 are amplified samples of genomic DNA, and lane 14 is molecular weight marker.

As shown in the gel electrophoresis image in Figure 5, the experiment results are similar to those shown in Figure 3. Products of experimental groups 1-4 show apparent bands around 100 bp, but product content between 100-500bp is relatively lower; products of other experimental groups 5-12 are mainly bands around 500bp, and experimental groups 5-12 show no apparent bands around 100 bp. It can be inferred from Figure 5 that, in experimental groups 1-4, there are more primer polymers and the reacting efficiency is relatively lower, however, when the fixed sequence comprised in the variable sequence of the first type of primer is TGGG or GTTT (experimental groups 3-4), the reacting efficiency is higher than that for primers whose fixed sequence is GGG or TTT (experimental groups 1-2). Yields of amplification product differ little among experimental groups 5-10, but are higher compared to experimental groups 1-4 (indicating lower degree of primer polymer production in groups 5-10 than in groups 1-4) and slightly lower compared to experimental groups 11-12.

Amplification products were purified and sequenced according to the protocols as described above, and the various indices of sequencing results are shown in Table 5 below, in which unique_mapped_of_raws in experimental groups 5-12 were between 78%-85%, with little difference among groups, but unique_mapped_of_raws in experimental groups 1-4 were relatively lower, being between 63-70%. As shown by data in Table 5, mapped_of_raws in experimental groups 5-12 were between 84%-92%, with little difference among groups, but mapped_of_raws in experimental groups 1-4 were relatively lower, being between 73-86%. In addition, data in Table 3 further demonstrates that data quality of reads in experimental groups 1-4 are also lower than other experimental groups. For example, proportions of high-quality data in raw data of experimental groups 3 and 4 were only 68.97% and 72.29%, while in experimental groups 5-12, proportions of high-quality data were between 94%-96%.

**Table 5: Major quality indices in high-throughput sequencing results of experimental groups 1-12**

| **Experi mental group** | **Raw data** | **GC %** | **High-quality data** | **High-quality data in raw data (%)** | **Mapped data** | **Mapping rate (%)** | **Mapped data in raw data (%)** | **Unique mapped data** | **Unique mapped rate in raw data (%)** |
|---|---|---|---|---|---|---|---|---|---|
| **1** | **1,150,576** | **41** | **1,040,765** | **90.46** | **802,095** | **77.07** | **69.71** | **740,821** | **64.39** |
| **2** | **1,136,500** | **41** | **1,029,750** | **90.61** | **719,871** | **69.91** | **63.34** | **668,090** | **58.78** |
| **3** | **1,405,646** | **40** | **969,500** | **68.97** | **893,594** | **92.17** | **63.57** | **823,402** | **58.58** |
| **4** | **1,377,299** | **40** | **995,647** | **72.29** | **881,281** | **88.51** | **63.99** | **810,546** | **58.85** |
| **5** | **1,481,780** | **41** | **1,423,899** | **96.09** | **1,279,353** | **89.85** | **86.34** | **1,183,124** | **79.84** |
| **6** | **1,474,132** | **41** | **1,327,580** | **90.06** | **1,245,191** | **93.79** | **84.47** | **1,159,906** | **78.68** |
| **7** | **1,151,213** | **38** | **1,112,499** | **96.64** | **1,061,118** | **95.38** | **92.17** | **978,251** | **84.98** |
| **8** | **1,198,779** | **38** | **1,153,084** | **96.19** | **1,093,981** | **94.87** | **91.26** | **1,008,893** | **84.16** |
| **9** | **1,888,895** | **40** | **1,815,940** | **96.14** | **1,700,129** | **93.62** | **90.01** | **1,569,695** | **83.1** |
| **10** | **1,389,200** | **40** | **1,331,898** | **95.88** | **1,258,269** | **94.47** | **90.58** | **1,162,950** | **83.71** |
| **11** | **1,516,148** | **40** | **1,465,982** | **96.69** | **1,387,184** | **94.62** | **91.49** | **1,282,478** | **84.58** |
| **12** | **1,419,698** | **40** | **1,372,820** | **96.69** | **1,300,475** | **94.73** | **91.6** | **1,202,844** | **84.72** |

From the results above, it can be seen that the linear amplification primer mixtures used in experimental groups 1-4 produce more primer polymers, resulting in largely reduced amplification efficiency, and correspondingly, lower data amount under equivalent amplification conditions. Although A, T, C and G were evenly distributed in sequencing starting regions in experimental groups 1-4, mapped rate, especially unique mapped rate in data were lower, and thus subsequent processing of these sequencing data is more difficult.

### Example 2: Further validation of amplification effect using different linear amplification primer mixtures

Human epidermal fibroblasts were isolated and lysed according to the method of Example 1b), to obtain single-cell genomic DNA, and amplification was performed using the primer mixture used in experimental groups 11/12 and the primer mixture used in experimental groups 9/10 in Table 1, respectively. Ten parallel experiments were performed for each primer mixture (designated as 1_1, 1_2...1_10 and 2_1, 2_2...2_10, respectively). Amplification was performed according to the protocols in Example 1a) and amplification products were obtained. The amplification products were subject to gel electrophoresis, and the result of electrophoresis is shown in Figure 6, in which the concentrations of amplification products in experimental groups 2_1, 2_2 ... 2_10 were slightly lower than those in experimental groups 1_1, 1_2 ... 1_10.

The amplification products above were purified according to the protocols of Example 1a) and the same volume of purified amplification product was taken for sequencing. The relative index parameters of sequencing results are shown in Tables 6-7 below.

**Table 6: Major quality indices in high throughput sequencing results of experimental groups 1_1, 1_2 ... 1_10**

| **Experi mental group** | **Raw data** | **G C %** | **High-quality data** | **High-quality data in raw data (%)** | **Mapped data** | **Mapping rate (%)** | **Mapped data in raw data (%)** | **Unique mapped data** | **Unique mapped rate in raw data (%)** | **CV** |
|---|---|---|---|---|---|---|---|---|---|---|
| **1_1** | **205,947** | **40** | **191,338** | **92.9** | **178,630** | **93.35** | **86.73** | **165,140** | **80.18** | **0.094542** |
| **1_2** | **1,516,148** | **40** | **1,465,982** | **96.69** | **1,387,184** | **94.62** | **91.49** | **1,282,478** | **84.58** | **0.045219** |
| **1_3** | **1,506,646** | **40** | **1,455,503** | **96.6** | **1,380,829** | **94.86** | **91.64** | **1,274,956** | **84.62** | **0.046309** |
| **1_4** | **1,933,765** | **40** | **1,871,558** | **96.78** | **1,764,141** | **94.26** | **91.22** | **1,632,090** | **84.39** | **0.044001** |
| **1_5** | **1,637,643** | **40** | **1,582,541** | **96.63** | **1,496,495** | **94.56** | **91.38** | **1,384,228** | **84.52** | **0.049991** |
| **1_6** | **1,811,740** | **40** | **1,750,193** | **96.6** | **1,664,244** | **95.08** | **91.85** | **1,537,198** | **84.84** | **0.040277** |
| **1_7** | **1,797,392** | **40** | **1,741,542** | **96.89** | **1,652,776** | **94.9** | **91.95** | **1,527,220** | **84.96** | **0.047065** |
| **1_8** | **1,419,698** | **40** | **1,372,820** | **96.69** | **1,300,475** | **94.73** | **91.6** | **1,202,844** | **84.72** | **0.04839** |
| **1_9** | **1,752,538** | **40** | **1,694,424** | **96.68** | **1,610,586** | **95.05** | **91.9** | **1,489,148** | **84.97** | **0.045286** |
| **1_10** | **2,050,600** | **40** | **1,988,093** | **96.95** | **1,895,099** | **95.32** | **92.41** | **1,747,232** | **85.2** | **0.046521** |

**Table 7: Main quality indexes of high throughput sequencing results in experimental groups 2_1, 2_2...2-10**

| **Experi mental group** | **Raw data** | **G C %** | **High-quality data** | **High-quality data in raw data (%)** | **Mapped data** | **Mapping rate (%)** | **Mapped data in raw data (%)** | **Unique mapped data** | **Unique mapped rate in raw data (%)** | **CV** |
|---|---|---|---|---|---|---|---|---|---|---|
| **1_1** | **205,947** | **40** | **191,338** | **92.9** | **178,630** | **93.35** | **86.73** | **165,140** | **80.18** | **0.094542** |
| **1_2** | **1,516,148** | **40** | **1,465,982** | **96.69** | **1,387,184** | **94.62** | **91.49** | **1,282,478** | **84.58** | **0.045219** |
| **1_3** | **1,506,646** | **40** | **1,455,503** | **96.6** | **1,380,829** | **94.86** | **91.64** | **1,274,956** | **84.62** | **0.046309** |
| **1_4** | **1,933,765** | **40** | **1,871,558** | **96.78** | **1,764,141** | **94.26** | **91.22** | **1,632,090** | **84.39** | **0.044001** |
| **1_5** | **1,637,643** | **40** | **1,582,541** | **96.63** | **1,496,495** | **94.56** | **91.38** | **1,384,228** | **84.52** | **0.049991** |
| **1_6** | **1,811,740** | **40** | **1,750,193** | **96.6** | **1,664,244** | **95.08** | **91.85** | **1,537,198** | **84.84** | **0.040277** |
| **1_7** | **1,797,392** | **40** | **1,741,542** | **96.89** | **1,652,776** | **94.9** | **91.95** | **1.527,220** | **84.96** | **0.047065** |
| **1_8** | **1,419,698** | **40** | **1,372,820** | **96.69** | **1,300,475** | **94.73** | **91.6** | **1,202,844** | **84.72** | **0.04839** |
| **1_9** | **1,752,538** | **40** | **1,694,424** | **96.68** | **1,610,586** | **95.05** | **91.9** | **1,489,148** | **84.97** | **0.045286** |
| **1_10** | **2,050,600** | **40** | **1,988,093** | **96.95** | **1,895,099** | **95.32** | **92.41** | **1,747,232** | **85.2** | **0.046521** |

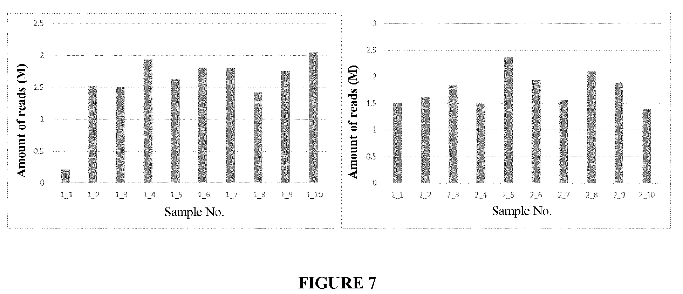

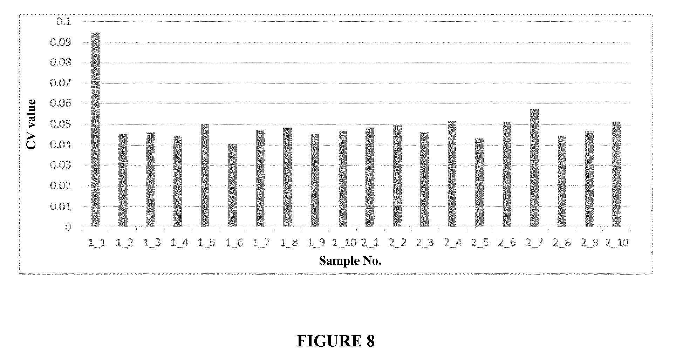

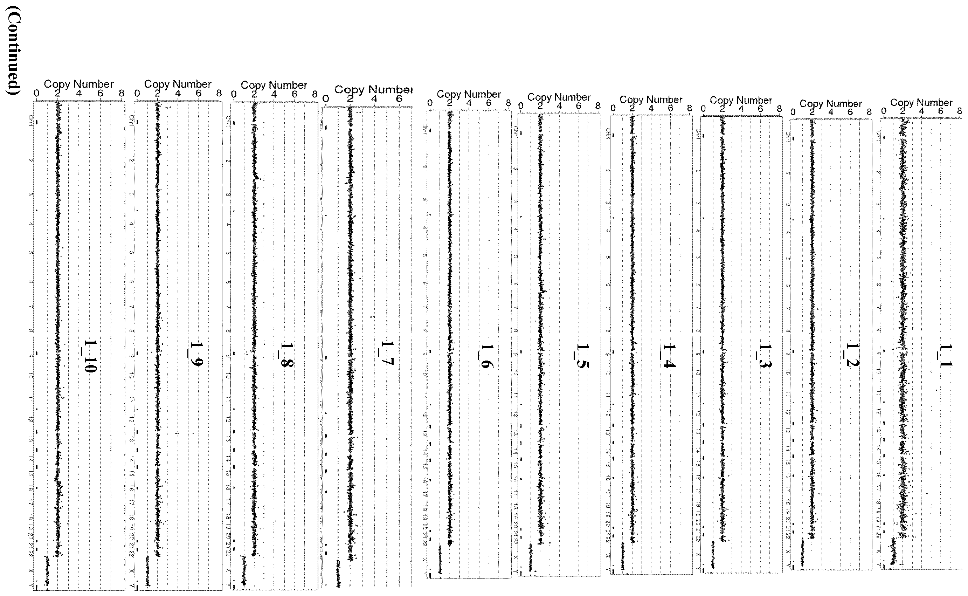

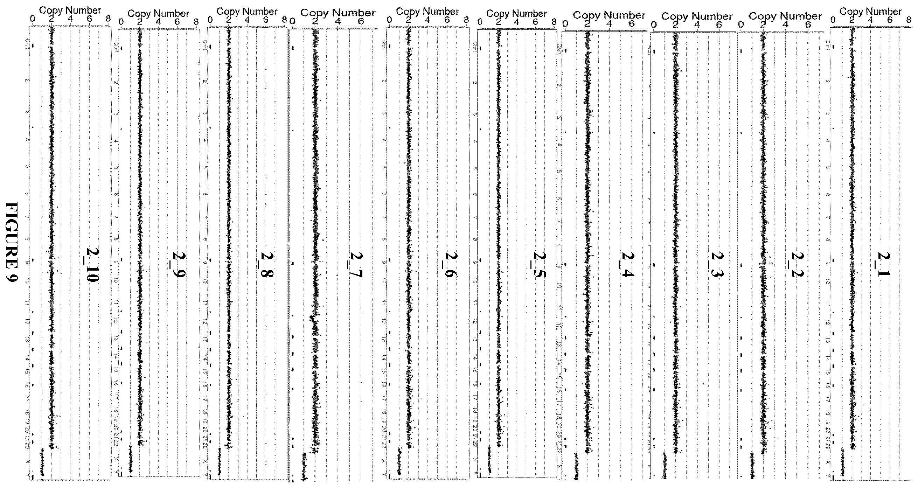

Data in Tables 6 and 7 show that in experimental groups 2_1, 2_2...2_10, the unique_mapped_of_raws were around 83%-84% and the mapped_of_raws were around 90%-91%, while in experimental groups 1_1, 1_2...1_10, the unique_mapped_of_raws were around 84%-85% and the mapped_of_raws were around 91%-92%, with little difference between the two groups. Statistical results of amount of loaded data of various samples in each group are shown in Figure 7: among experimental groups 1_1, 1_2...1_10, data in experimental groups except for the abnormal experimental group 1_1 (the data amount of which is extremely low, probably due to misconduct during the recovery process) were all between 1.5-2 M, and the average amount of loaded data in experimental groups 1_2, 1_3...1_10 was around about 1.7 M. Among experimental groups 2_1, 2_2...2_10, the data amount were all between 1.5-2.5M, and the average amount of loaded data in experimental groups 2_1, 2_2...2_10 was around about 1.8 M. The data amount was slightly higher in experimental groups 2_1, 2_2...2_10. In addition, the copy number variation coefficient CV in the loaded data of the experimental groups are summarized as in Figure 8. After excluding the obviously abnormal experimental group 1_1, the average copy number variation coefficient CV of experimental groups 1_2, 1_3...1_10 was about 0.046, and the average copy number variation coefficient CV of experimental groups 2_1, 2_2...2_10 was about 0.049, with no significant difference in the copy number variation coefficient between the two groups. The image of copy number variation for each experimental group is shown separately in Figure 9, in which the ordinate represents copy number of chromosome, which is 2 in normal persons; the abscissa represents chromosomes 1-22 and sex chromosomes. As shown in the figure, in each experimental group, chromosomes 1-22 all roughly have two copies, except for some particular data points, whereas both sex chromosomes X and Y have roughly one copy, respectively.

### Example 3: Detection of pathogenic sites and detection using quality detecting primers

### Detection of pathogenic sites

35 pathogenic sites were randomly selected (see table 8 below for sites selected) and primers were designed. The selected pathogenic sites and corresponding primers thereof are shown in Table 8 and Table 9, respectively.

**Table 8: 35 pathogenic sites randomly selected**

| | **Names of pathogenic sites** | **Chromosomal location** |
|---|---|---|
| **1** | SMN1-1 | chr5 |
| **2** | SMN1-2 | chr5 |
| **3** | SMN1-3 | chr5 |
| **4** | SMN1-4 | chr5 |
| **5** | SMN1-1R | chr5 |
| **6** | SMN1-2R | chr5 |
| **7** | SMN1-3R | chr5 |
| **8** | SMN1-4R | chr5 |
| **9** | PDS-IV15 | chr7 |
| **10** | PDS-EXON5 | chr7 |
| **11** | PDS-EXON7+8 | chr7 |
| **12** | PDS-EXON10 | chr7 |
| **13** | PDS-EXON17 | chr7 |
| **14** | PDS-EXON19 | chr7 |
| **15** | HBB3 | chr11 |
| **16** | HBB | chr11 |
| **17** | MMACHC | chr1 |
| **18** | HBA2 | chr16 |
| **19** | GJB2 | chr13 |
| **20** | GJB2-C796 | chr13 |
| **21** | ATP7B-8 | chr13 |
| **22** | PKHD1-3681 | chr6 |
| **23** | PKHD1-1713 | chr6 |
| **24** | WASP-C21 | chrX |
| **25** | WASP-C12 | chrX |
| **26** | DMD-13exe | chrX |
| **27** | GJB2 | chr13 |
| **28** | GJB2-c79 | chr13 |
| **29** | PDS-7+8 | chr7 |
| **30** | PDS-10 | chr7 |
| **31** | CFTR-IVS13 | chr17 |
| **32** | IL2RG | chrX |
| **33** | IL2RGIVS4 | chrX |
| **34** | FLG-c3319 | chr1 |
| **35** | IDS | chrX |

**Table 9: Primers corresponding to the pathogenic sites in Table 8**

| **Names of pathogenic sites** | **Primer sequences** |
|---|---|
| SMN1-1+ | AAAATGTCTTGTGAAACAAAATGC |
| SMN1-1- | TTTTACAAAAGTAAGATTCACTTTCATAAT |
| SMN1-2+ | AGGGTTTCAGACAAAATCAAAAAGAAG |
| SMN1-2- | CTAATAGTTTTGGCATCAAAATTCTTTAAT |
| SMN1-3+ | CTTTATGGTTTGTGGAAAACAAATG |
| SMN1-3- | GTCTGCCTACTAGTGATATAAAATGG |
| SMN1-4+ | CTGGAATGTGAAGCGTTATAG |
| SMN1-4- | CAAAATCTAATCCACATTCAAATTTT |
| SMN1-1R+ | TGTGGGATTGTAGGCATGAG |
| SMN1-1R- | GCTGGCAGACTTACTCCTTAAT |
| SMN1-2R+ | AAGTCTGCCAGCATTATGAAAG |
| SMN1-2R- | CCACATAACCAACCAGTTAAG |
| SMN1-3R+ | GTTCAGATGTTAAAAAGTTGAAAG |
| SMN1-3R- | TGGTCTGCCTACTAGTGATATAAA |
| SMN1-4R+ | GGAAGTGGAATGGGTAACTCTT |
| SMN1-4R- | CCACATACGCCTCACATACAT |
| PDS-IV15+ | CCAAAGGTTGGATTTGATGCC |
| PDS-IV15- | GAATAGCTCAGTTGTTCTTTGATACG |
| PDS-EXON5+ | CCGACGAACACTTTCTCGTATC |
| PDS-EXONS- | GGGTTCCAGGAAATTACTTTGTTT |
| PDS-EXON7+8+ | AAGTCTCCCTGTTCTGTCCTA |
| PDS-EXON7+8- | AGGGTGTTGCAGACAAAGT |
| PDS-EXON10+ | TTCACTGCTGGATTGCTCAC |
| PDS-EXON10- | CCCCTTGGGATGGATTTAAC |
| PDS-EXON17+ | GGAGGAACTTGATATCCCAACC |
| PDS-EXON17- | ATACTGGACAACCCACATCATT |
| PDS-EXON19+ | GAGCAATGCGGGTTCTTTG |
| PDS-EXON19- | GCTAGACTAGACTTGTGTAATGTTTG |
| HBB3+ | TCATGCCTCTTTGCACCATT |
| HBB3- | AATCCAGCCTTATCCCAACCA |
| HBB+ | GGTTGGCCAATCTACTCCCA |
| HBB- | AAGGTGCCCTTGAGGTTGTC |
| MMACHC+ | GGAGTCGAAGCTGACTCA |
| MMACHC- | CAGTTGCAACGAAGCCAATC |
| HBA2+ | CTTCTCTGCACAGCTCCTAAG |
| HBA2- | GCTGCCCACTCAGACTTTAT |
| GJB2+ | GACGCCAAGTTTGAAGGAAC |
| GJB2- | CTACTGCTAGAAACAGCCTACTC |
| GJB2-C79+ | TCGCATTATGATCCTCGTTG |
| GJB2-C79- | GGACACAAAGCAGTCCACAG |
| ATP7B-8+ | AAAAGCTGAGAAGTTCAGAAAAC |
| ATP7B-8- | AAATTTGTATTTAACAAGTGCTTGTC |
| PKHD1-3681+ | AGTGATTGTCATTGAAATTGGTGATTC |
| PKHD1-3681- | AGCCAATGACTCCCTTTGAC |
| PKHD1-1713+ | CAGAGCGATGACATCTTAACCT |
| PKHD1-1713- | GTGAACACCAGGGCAGATGAG |
| WASP-C21+ | TGTCCCTTGTGGTTTTTTGCATTTC |
| WASP-C21- | TTTCGTCCAAGCATCTCAAAGAGTC |
| WASP-C12+ | CTCTTCTTACCCTGCACCCAGAG |
| WASP-C12- | GCATTTTCGTCCAAGCATCTCAAAGAG |
| DMD-13exe+ | AAGAACAAGTCAGGGTCAAT |
| DMD-13exe- | TTAAAATACTTTTCAAGTTATAGTTCTTTT |
| GJB2+ | GACGCCAAGTTTGAAGGAAC |
| GJB2- | CTACTGCTAGAAACAGCCTACTC |
| GJB2-c79+ | TCGCATTATGATCCTCGTTG |
| GJB2-c79- | GGACACAAAGCAGTCCACAG |
| PDS-7+8+ | AAGTCTCCCTGTTCTGTCCTA |
| PDS-7+8- | AGGGTGTTGCAGACAAAGT |
| PDS-10+ | TTCACTGCTGGATTGCTCAC |
| PDS-10- | CCCCTTGGGATGGATTTAAC |
| CFTR-IVS13+ | TTTGCAGAGAATGGGATAGAGAG |
| CFTR-IVS13- | CACCTATTCACCAGATTTCGTAGT |
| IL2RG+ | TGACCAGGAAATAGAGAGGAAATG |
| IL2RG- | CATTCTGCCATACCAACAATGG |
| IL2RGIVS4+ | ATTGGAAGCCGTGGTTATCTC |
| IL2RGIVS4- | CTTCCATCACCAAACCCTCTT |
| FLG-c3319+ | CTGAGTGAATCCCAGCTAGAAC |
| FLG-c3319- | GCAGAGAACAGGAGCTTGAT |
| IDS+ | CTCCAGACACTCAGGCATTC |
| IDS- | GTGCTCACCTGGTAGATGAAA |

Amplification products in experimental groups 1_1, 1_2, 2_1, 2_2 according to Example 2 were randomly selected as template DNA, respectively. PCR detection was performed on the template DNA using 2x GoldstarMasterMix (purchased from Beijing ComWin Biotech Co., Ltd., Cat. No. CW0960). Composition of amplification system is shown in Table 10, and amplification program is shown in Table 11.

**Table 10: PCR reaction system for detection of pathogenic sites**

| **Components** | **Volume (µl)** |
|---|---|
| 2xGoldstar stock mixture | 25 |
| Forward primer (10uM) | 1 |
| Reverse primer (10uM) | 1 |
| Template DNA (10ng/µl ) | 1 |
| RNase-free water | 22 |
| total | *50* |

**Table 11: PCR amplification system for detection of pathogenic sites**

| **Cycle number** | **Temperature (centigrade)** | **Step** | **Time** |
|---|---|---|---|
| 1 | 95 | Pre-denaturation | 10min |
| 40 | 95 | Denaturation | 30s |
| | 55 | Anneal | 30s |
| | 72 | Extension | 1min |
| 1 | 72 | Final extension | 5min |

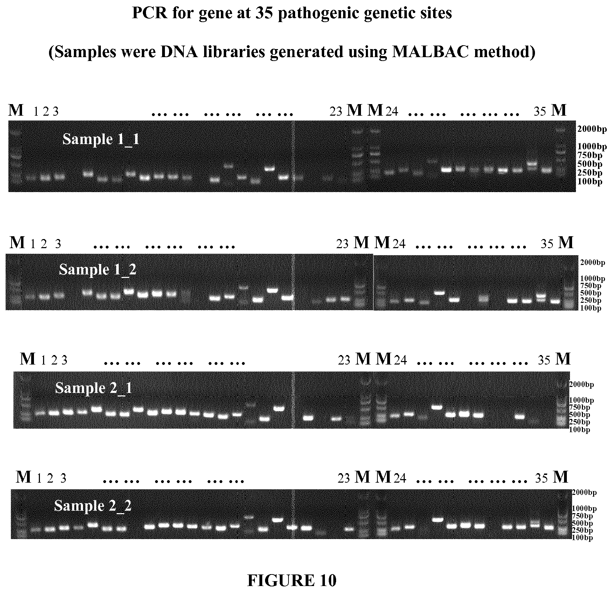

Amplification results are shown in gel electrophoresis image in Figure 10. The amplification results show that, neither pathogenic site 4 nor 13 was amplified in either of samples 1_1 and 1_2, while pathogenic site 21 was not amplified in sample 1_1, and pathogenic sites 20, 29, 31 were not amplified in sample 1_2. Pathogenic site 31 was amplified in neither samples 2_1 nor 2_2, while pathogenic sites 18, 21, 32, 35 were not amplified in sample 2_1, and pathogenic sites 8 and 22 were not amplified in sample 1_2. The results showed that the two groups of primer samples (1_1, 1_2 and 2_1, 2_2) had no significant difference in amplification accuracy and amount of amplification product.

### q-PCR detection using quality testing primers

Amplification products in experimental groups 1_1, 1_2, 2_1, 2_2 in example 2 described above, positive control (gDNA in the same concentration), and negative control (without template) were used as template DNA, respectively. q-PCR was performed on template DNA using 6 groups of quality testing primers as shown in Table 12, which target DNA sequences on different chromosomes, respectively. 2xFastSYBR Mixture (purchased from Beijing ComWin Biotech Co., Ltd., Cat. No. CW0955) was used in fluorescent quantitative PCR. Composition of amplification system is shown in Table 13, and amplification program is shown in Table 14.

**Table 12: Information of 6 pairs of random primers used for quality detection**

| **Primer name** | **Primer sequence** | **Chromosomal location** |
|---|---|---|
| CH1+ | AGGAAAGGCATACTGGAGGGACAT | chr1 |
| CH1- | TTAGGGATGGCACCACACTCTTGA | |
| CH2+ | TCCCAGAGAAGCATCCTCCATGTT | chr2 |
| CH2- | CACCACACTGCCTCAAATGTTGCT | |
| CH4+ | ATGGGCAAATCCAGAAGAGTCCAG | chr4 |
| CH4- | CCATTCACTTCCTTGGAAAGGTAGCC | |
| CH5+ | AATAGCGTGCAGTTCTGGGTAGCA | chr5 |
| CHS- | TTCACATCCTGGGAGGAACAGCAT | |
| CH6+ | TGAATGCCAGGGTGAGACCTTTGA | chr6 |
| CH6- | TGTTCATTATCCCACGCCAGGACT | |
| CH7+ | ACCAAAGGAAAGCCAGCCAGTCTA | chr7 |
| CH7- | ACTCCACAGCTCCCAAGCATACAA | |

**Table 13: Reaction system used for qPCR detection**

| **Components** | **Volume (µl)** |
|---|---|
| 2xFastSYBR Mixture | 25 |
| Forward primer (10uM) | 1 |
| Reverse primer (10uM) | 1 |
| Template DNA (10ng/ul) | 1 |
| RNase-free water | 22 |
| total | *50* |

**Table 14: Amplification program used for qPCR detection**

| **Cycle number** | **Temperature (centigrade)** | **Step** | **Time** |
|---|---|---|---|
| 1 | 95 | Pre-denaturation | 1 min |
| 40 | 95 | Denaturation | 15 s |
| | 60 | Anneal | 40 s |

Amplification results are shown in Table 15, in which qPCR detection data for template DNA of each group by primer pairs CH1, CH2, CH4, CH5, CH6 and CH7 are listed, respectively, wherein a larger Ct value indicates a lower template number that the primer corresponds to, and correspondingly, a poorer amplification efficiency in gDNA amplification. Amplification results show that in sample 2_1, CH1, CH2, CH4, CH5, CH6 and CH7 all had a high amplification efficiency, and in sample 2_2, CH1, CH2, CH4, CH5, CH6 and CH7 all had a high amplification efficiency, which is not substantially different from amplification of samples 1-1 and 1-2.

**Table 15: Amplification efficiency of the 4 samples in Figure 6 by qPCR using the 6 pairs of primers in Table 12**

| | **Sample** | **Chromosome targeted by the primer** | **Ct value** |
|---|---|---|---|
| Amplification product of experimental group 11/12 | 1_1 | CH1 | 21.78 |
| | 1_1 | CH2 | 26.71 |
| | 1_1 | CH4 | 23.33 |
| | 1_1 | CH5 | 25.85 |
| | 1_1 | CH6 | 24.66 |
| | 1_1 | CH7 | 28.57 |
| | 1_2 | CH1 | 22.98 |
| | 1_2 | CH2 | 23.11 |
| | 1_2 | CH4 | 23.7 |
| | 1_2 | CH5 | 23.74 |
| | 1_2 | CH6 | 23.57 |
| | 1_2 | CH7 | 23.72 |
| Amplification product of experimental group 9/10 | 2_1 | CH1 | 21.72 |
| | 2_1 | CH2 | 24.43 |
| | 2_1 | CH4 | 24.74 |
| | 2_1 | CH5 | 25.16 |
| | 2_1 | CH6 | 27.46 |
| | 2_1 | CH7 | 29.8 |
| | 2_2 | CH1 | 22.76 |
| | 2_2 | CH2 | 23.06 |
| | 2_2 | CH4 | 27.46 |
| | 2_2 | CH5 | 25.48 |
| | 2_2 | CH6 | 28.36 |
| | 2_2 | CH7 | 27.6 |
| Positive control (genome DNA) | + | CH1 | 23.03 |
| | + | CH2 | 23.42 |
| | + | CH4 | 23.7 |
| | + | CH5 | 23.74 |
| | + | CH6 | 23.57 |
| | + | CH7 | 23.72 |
| Negative control | - | CH1 | 33.41 |
| | - | CH2 | 34.62 |
| | - | CH4 | 37.75 |
| | - | CHS | 29.72 |
| | - | CH6 | 31.1 |
| | - | CH7 | 32.94 |

### Example 4: Applying the amplification method of the present application to Ion torrent sequencing platform

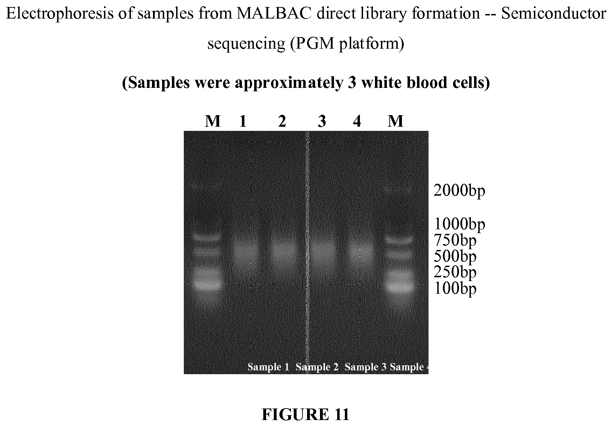

Fresh blood was drawn and lymphocytes were separated using lymphocyte separating fluid, from which a portion of cell-containing suspension was pipetted. Approximately 3 white blood cells were picked using a mouth pipette under a 10x microscope and the volume of aspirated PBS solution did not exceed 1 microliter. The around 3 picked white blood cells were transferred into a PCR tube containing 4 microliters of lysis buffer (containing Tris-Cl, KCl, EDTA, Triton X-100 and Qiagen Protease), and were lysed according to the protocols described in Example 1b). Genomic DNA was subject to linear amplification using the primer mixture used in experimental groups 9/10 in Table 1, and exponential amplification using the following mixture of second primers to obtain amplification products: second primer-1 (SEQ ID NO: 37): CCA CTA CGC CTC CGC TTT CCT CTC TAT GGG CAG TCG GTG ATG CTC TTC CGA TCT;
second primer-2 (SEQ ID NO: 38): CCA TCT CAT CCC TGC GTG TCT CCG ACT CAG CTA AGG TAA CGA TGC TCT TCC GAT CT; (in which the double-underlined bases in the second primers comprise the part corresponding to the capture sequence of the sequencing platform, the dotted part is the part of identifier sequence, which can be replaced by other identifier sequences as needed, and the single-underlined part is the part of common sequence). 4 experiments were conducted in parallel. All other reacting conditions were consistent with those described in Example 1b). The amplification effect is shown by gel electrophoresis in Figure 11.

### Homogeneity detection

Subsequently, 2 samples (shown as Sample 1 and Sample 2 in Figure 11) amplified according to Example 4 were randomly selected as template DNA, respectively. PCR was performed for the template DNA using 2xGoldstarMasterMix (purchased from Beijing ComWin Biotech Co., Ltd., Cat. No. CW0960), in which primers shown in Table 9 were used to amplify the 35 pathogenic sites shown in Table 8. Composition of amplification system is shown in Table 10, and amplification program is shown in Table 11.

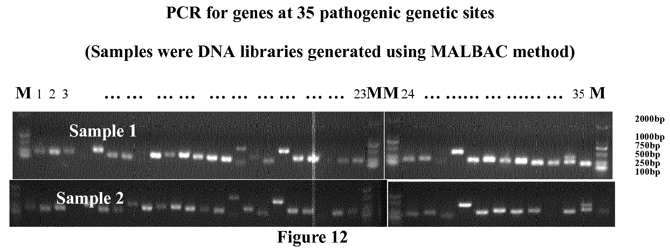

Amplification results are shown in Figure 12. The amplification results show that the 35 pathogenic sites were all well amplified in the two amplification product samples described above. There were no significant differences in amplification accuracy and amount of amplification product between the two samples.

### Gene sequencing

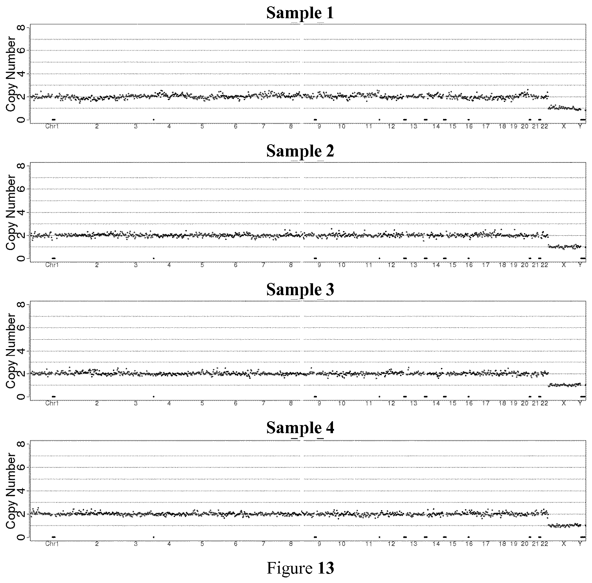

Equal volumes of the 4 samples shown in Figure 11 were taken after purification, sequenced using PGM^{™} Sequencer in Life Technologies' Ion Torrent Sequencing Platform, and sequences obtained from sequencing were mapped to human reference genome. The sequencing results are shown in Table 16 below and in Figure 13.

**Table 16: Sequencing results of semiconductor sequencing (in PGM platform) for samples in Table 11**

| **Experi mental group** | **Raw data** | **G C %** | **High-quality data** | **High-quality data in raw data (%)** | **Mapped data** | **Mappi ng rate (%)** | **Mapped data in raw data (%)** | **Unique mapped data** | **Unique mapped rate in raw data (%)** | **CV** |
|---|---|---|---|---|---|---|---|---|---|---|
| **1** | **481,125** | **39** | **430,878** | **89.56** | **348,586** | **80.9** | **72.45** | **327,548** | **68.08** | **0.08128** |
| **2** | **383,156** | **39** | **343,681** | **89.7** | **280,050** | **81.49** | **73.09** | **262,919** | **68.62** | **0.06395** |
| **3** | **531,872** | **39** | **476,298** | **89.55** | **379,113** | **79.6** | **71.28** | **355,493** | **66.84** | **0.06163** |
| **4** | **400,573** | **39** | **360,498** | **90** | **297,439** | **82.51** | **74.25** | **279,379** | **69.74** | **0.06073** |

As shown by data in Table 16, in samples 1, 2, 3 and 4, the unique_mapped_of_raws were around 68%, the mapped_of_raws were around 72%-73%, the data amount was between 0.38-0.53 M, and the copy number variation coefficient CV of loaded data was about 0.06. In addition, the image of the copy number variation of sequencing reads is shown in Figure 13. In each experimental group, chromosomes 1-22 all roughly have two copies, except for some particular data points, whereas both sex chromosomes X and Y have roughly one copy, respectively.

It is noteworthy that designing a sequence-platform specific common sequence is not required in the Ion Torrent sequencing platform. In principle, any sequence that substantially does not bind to genomic DNA to produce an amplified sequence within a range of 6-60bp in length, can be selected as common sequence. We also used two other primer combinations in the Ion Torrent sequencing platform for detection: i) using a mixture of first type of primers similar to that used in the experimental groups 9/10 in Table 1: SEQ ID NO: 15, 16, 19 and 20 (in which, however, the common sequence was SEQ ID NO: 1), and the corresponding primer mixture for exponential amplification was SEQ ID NO: 39 CCA CTA CGC CTC CGC TTT CCT CTC TAT GGG CAG TCG GTG ATT TGG TAG TGA GTG and SEQ ID NO: 40 CCA TCT CAT CCC TGC GTG TCT CCG ACT CAG CTA AGG TAA CGA TTT GGT AGT GAG TG; ii) using a mixture of first type of primers similar to that used in the experimental groups 9/10 in Table 1: SEQ ID NO: 23, 24, 27 and 28 (in which, however, the common sequence was SEQ ID NO: 2), and the corresponding exponential amplification primer mixture was SEQ ID NO: 41 CCA CTA CGC CTC CGC TTT CCT CTC TAT GGG CAG TCG GTG ATG AGG TGT GAT GGA and SEQ ID NO: 42 CCA TCT CAT CCC TGC GTG TCT CCG ACT CAG CTA AGG TAA CGA TGA GGT GTG ATG GA. The amplification and sequencing results showed no significant difference from the results shown in Figure 11 and Table 16 (detailed data not provided here).

### Example 5: Using the amplification method of the present application for chromosome detection of blastula trophoblast prior to embryo implantation

Zygotes were cultured *in vitro* and several cells (about 3 cells) in the trophectoderm were taken at blastula stage (day 5 of *in vitro* culture) for detection of chromosome copy number abnormity. The method of collecting blastula trophectoderm cells may be any method known to those skilled in the art, such as but not limited to, the method described in Wang L, Cram DS, et al. Validation of copy number variation sequencing for detecting chromosome imbalances in human preimplantation embryos. Biol Reprod, 2014, 91(2):37. The collected blastula trophectoderm cells were transferred into a PCR tube containing 5 microliters of lysis buffer, added with lysate, and were lysed according to the protocols described in Example 1b), and genomic DNA was amplified using the primer mixture used in experimental groups 9/10 in Table 1 (4 experiments were performed in parallel). Amplification products were purified and sequenced according to the protocols in Example 1. Sequencing results are shown in Table 17 below.

**Table 17: Sequencing results of blastula trophoblast samples**

| **Experi mental group** | **Raw data** | **GC %** | **High-quality data** | **High-quality data in raw data (%)** | **Mapped data** | **Mapping rate (%)** | **Mapped data in raw data (%)** | **Unique mapped data** | **Unique mapped rate in raw data (%)** |
|---|---|---|---|---|---|---|---|---|---|
| **1** | **2,546,518** | **42** | **2,225,557** | **87.39** | **1,819,333** | **81.47.** | **71.44** | **1,683,670** | **66.11** |
| **2** | **1,444,316** | **41** | **1,188,076** | **82.25** | **1,036,408** | **87.23.** | **71.75** | **959,980** | **66.46** |
| **3** | **2,139,214** | **41** | **1,847,833** | **86.37** | **1,682,192** | **91.03.** | **78.63** | **1,563,008** | **73.06** |

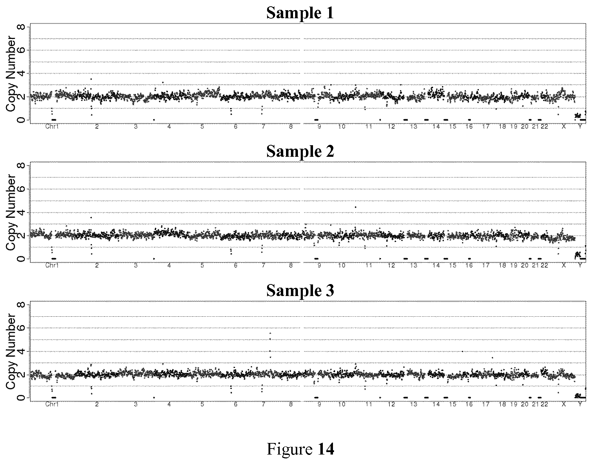

As shown by data in Table 17, in samples 1, 2, and 3, the unique_mapped_of_raws were around 66-73%, the mapped_of_raws were around 71%-78%, and the data amount was between 1.4-2.6 M. In addition, the image of copy number variation of sequencing reads is shown in Figure 14. In each experimental group, chromosomes 1-22 all roughly have two copies, except for some particular data points, whereas both sex chromosomes X and Y have roughly one copy, respectively.

Although various aspects and embodiments have been disclosed by the present disclosure, other aspects and embodiments will be apparent to those skilled in the art. The various aspects and embodiments disclosed herein are for the purpose of illustration only, and are not intended to limit the scope of the present disclosure. The actual scope of the protection of the present disclosure is governed by the claims.

## Claims

1. A method of amplifying genomic DNA, said method comprising:
(a) providing a first reaction mixture, wherein the first reaction mixture comprises a sample containing the genomic DNA, primers, a mixture of nucleotide monomers, and a nucleic acid polymerase, wherein the primers consist of first primers and third primers,
wherein the first primers consist of, in a 5' to 3' orientation, a common sequence and a first variable sequence, or the first primers consist of, in a 5' to 3' orientation, a common sequence, a first spacer sequence and a first variable sequence, wherein the first primers are a mixture of primers comprising the same common sequence and different first variable sequences, wherein each first variable sequence consists of a first random sequence and a fixed sequence at its 3' end, wherein the first random sequence is, in a 5' to 3' orientation, sequentially Xₐ₁Xₐ₂......Xₐₙ, and Xₐᵢ (i=1-n) of the first random sequence all belong to a same set, said set is selected from B, or D, or H, or V, wherein B={T, G, C}, D={A, T, G}, H={T, A, C}, V={A, C, G}, wherein Xₐᵢ represents the i^{th} nucleotide from 5' end of the first random sequence, n is a positive integer selected from 4-16, and wherein the first spacer sequence is Yₐ₁......Yₐₘ, wherein Yₐⱼ (j=1-m) ∈ {A, T, G, C}, wherein Yₐⱼ represents the j^{th} nucleotide from 5' end of the first spacer sequence, m is a positive integer selected from 1-3;
wherein the third primers consist of, in a 5' to 3' orientation, the common sequence and a third variable sequence, or wherein the third primers consist of, in a 5' to 3' orientation, the common sequence, a third spacer sequence and a third variable sequence, wherein the third primers are a mixture of primers comprising the same common sequence and different third variable sequences, wherein each third variable sequence consists of a third random sequence and a fixed sequence at its 3' end, wherein the third random sequence is, in a 5' to 3' orientation, sequentially X_{b1}X_{b2}......X_{bn}, and X_{bi} (i=1-n) of the third random sequence all belong to a same set, said set is selected from B- , or D, or H, or V, wherein B={T, G, C}, D={A, T, G}, H={T, A, C}, V={A, C, G}, and X_{bi} (i=1-n) and Xₐᵢ (i=1-n) belong to different sets, wherein X_{bi} represents the i^{th} nucleotide from 5' end of the third random sequence, n is a positive integer selected from 4-16, and wherein the third spacer sequence is Y_{b1}......Y_{bm}, wherein Y_{bj} (j=1-m) ∈ {A, T, G, C}, wherein Y_{bj} represents the j^{th} nucleotide from 5' end of the third spacer sequence, m is a positive integer selected from 1-3;
(b) placing the first reaction mixture in a first thermal cycle program for pre-amplification, to obtain a pre-amplification product;
(c) providing a second reaction mixture, wherein said second reaction mixture comprises said pre-amplification product obtained from step (b), a second primer, a mixture of nucleotide monomers, and a nucleic acid polymerase, wherein the second primer comprises or consists of, in a 5' to 3' orientation, a specific sequence and the common sequence;
(d) placing the second reaction mixture in a second thermal cycle program for amplification, to obtain an amplification product.

2. The method of claim 1,
(a) wherein Xₐᵢ (i=1-n) of the first random sequence all belong to set B, X_{bi} (i=1-n) of the third random sequence all belong to set D; or
(b) wherein the fixed sequence is individually selected from the group consisting of CCC, AAA, TGGG, GTTT, GGG, TTT, TNTNG or GTGG; or
(c) wherein the first variable sequence is selected from Xₐ₁Xₐ₂......XₐₙTGGG or Xₐ₁Xₐ₂......XₐₙGTTT, the third variable sequence is selected from X_{b1}X_{b2}......X_{bn}TGGG or X_{b1}X_{b2}......X_{bn}GTTT; or
(d) wherein the common sequence is selected from SEQ ID NO: 1 [TTGGTAGTGAGTG], SEQ ID NO: 2 [GAGGTGTGATGGA], SEQ ID NO: 3 [GTGATGGTTGAGGTA], SEQ ID NO: 4 [AGATGTGTATAAGAGACAG], SEQ ID NO: 5 [GTGAGTGATGGTTGAGGTAGTGTGGAG] or SEQ ID NO: 6 [GCTCTTCCGATCT]; or
(e) wherein the method further comprises a step of sequencing an amplification product obtained in step (d), wherein the second primer comprises a sequence complementary or identical to part of or whole of a primer used for sequencing; or
(f) wherein the second primer comprises a primer mixture having identical common sequences and different specific sequences, said different specific sequences are complementary or identical to part of or whole of different primers in sequencing primer pairs used in a same sequencing, respectively; or
(g) wherein the second primer comprises a mixture of sequences set forth in SEQ ID NO: 35 [AATGATACGGCGACCACCGAGATCTACACTCTTTCCCTACACGAC GCTCTTCCGATCT] and SEQ ID NO: 36 [CAAGCAGAAGACGGCATACGAGATCGTGATGTGACTGGAGTTCAGACGTGTGCT CTTCCGATCT]; or
(h) wherein step (b) enables the variable sequence of the first primers to pair with the genomic DNA and the genomic DNA is amplified to obtain a genomic pre-amplification product, wherein the genomic pre-amplification product comprises the common sequence at its 5' end and a complementary sequence of the common sequence at its 3' end;
wherein the first thermal cycle program comprises:
(b1) a thermal program capable of opening the DNA double strands to obtain a single-strand DNA template;
(b2) a thermal program that enables binding of the first primers and the third primers to the single-strand DNA template;
(b3) a thermal program that enables extension of the length of the first primers that binds to the single-strand DNA template under the action of the nucleic acid polymerase, to produce a pre-amplification product;
(b4) repeating steps (b1) to (b3) to a designated first cycle number, wherein the designated first cycle number is more than 1; or
(i) wherein the step (d) enables the common sequence of the second primer to pair with 3' end of the genomic pre-amplification product and the genomic pre-amplification product is amplified to obtain an extended genomic amplification product;
wherein the step (d) comprises:
(d1) a thermal program capable of opening DNA double strands;
(d2) a thermal program further capable of opening DNA double strands;
(d3) a thermal program that enables binding of the second primer to single strand of the genomic pre-amplification product obtained in step (b);
(d4) a temperature program that enables extension of the length of the second primer that binds to the single strand of the genomic pre-amplification product, under the action of the nucleic acid polymerase;
(d5) repeating steps (d2) to (d4) to a designated second cycle number, wherein the designated second cycle number is more than 1; or
(j) further comprising analyzing the amplification product to identify disease- or phenotype-associated sequence features.

3. The method of claim 1, wherein the first primers comprise GCTCTTCCGA TCTYₐ₁Xₐ₁Xₐ₂Xₐ₃Xₐ₄Xₐ₅TGGG, GCTCTTCCGATCTYₐ₁Xₐ₁Xₐ₂Xₐ₃Xₐ₄Xₐ₅GTTT, or a combination thereof,
the third primers comprise GCTCTTCCGATCTY_{b1}X_{b1}X_{b2}X_{b3}X_{b4}X_{b5}TGGG, GCTCTTCCGATCTY_{b1}X_{b1}X_{b2}X_{b3}X_{b4}X_{b5}GTTT, or a combination thereof, wherein Yₐ₁ ∈ {A, T, G, C}, Y_{b1} ∈ {A, T, G, C}, said Xₐᵢ (i=1-5) ∈ {T, G, C}, said X_{bi} (i=1-5) ∈ {A, T, G}.

4. The method of claim 2(e), wherein (a) the common sequence comprises a sequence complementary to or identical to part of or whole of a primer used for sequencing; or
(b) the specific sequence of the second primer comprises a sequence complementary to or identical to part of or whole of a primer used for sequencing.

5. The method of claim 4(b), wherein (a) the specific sequence of the second primer further comprises a sequence complementary to or identical to part of or whole of a capture sequence of a sequencing platform; or (b) the sequence which is comprised in the specific sequence of the second primer and complementary or identical to part of or whole of a primer used for sequencing comprises or consists of SEQ ID NO: 31 [ACACTCTTTCCCTACACGAC], or SEQ ID NO: 32 [GTGACTGGAGTTCAGACGTGT].

6. The method of claim 5(b), wherein
(a) the sequence which is comprised in the specific sequence of the second primer and complementary or identical to part of or whole of a capture sequence of a sequencing platform, comprises or consists of SEQ ID NO: 33 [AATGATACGGCGACCACCGAGATCT], or SEQ ID NO: 34 [CAAGCAGAAGACGGCATACGAGAT]; or
(b) the specific sequence of the second primer further comprises a barcode sequence, said barcode sequence is located between the sequence complementary or identical to part of or whole of a capture sequence of a sequencing platform and the sequence complementary or identical to part of or whole of a primer used for sequencing.

7. The method of claim 2(h), wherein when undergoing the first cycle, the DNA double strands in step (b1) are genomic DNA double strands, the thermal program comprises a denaturing reaction at a temperature between 90-95°C for 1-20 minutes and after the first cycle, the thermal program in step (b1) comprises a melting reaction at a temperature between 90-95°C for 3-50 seconds; wherein when after undergoing a second cycle, the pre-amplification product comprises a genomic pre-amplification product comprising the common sequence at its 5' end and a complementary sequence of the common sequence at its 3' end.

8. The method of claim 2(h), wherein after step (b1) and prior to step (b2), said method does not comprise an additional step of placing the first reaction mixture in a suitable thermal program such that the 3' end and 5' end of the genomic pre-amplification product hybridize to form a hairpin structure.

9. The method of claim 2(j), wherein the disease- or phenotype-associated sequence features include chromosomal abnormalities, chromosomal translocation, aneuploidy, partial or complete chromosomal deletion or duplication, fetal HLA haplotypes and paternal mutations, or the disease or phenotype is selected from the group consisting of: beta-thalassemia, Down's syndrome, cystic fibrosis, sickle cell disease, Tay-Sachs disease, Fragile X syndrome, spinal muscular atrophy, hemoglobinopathy, Alpha-thalassemia, X-linked diseases (diseases dominated by genes on the X chromosome), spina bifida, anencephaly, congenital heart disease, obesity, diabetes, cancer, fetal sex, and fetal RHD.

10. The method of claim 2(j), wherein the genomic DNA is derived from a blastomere, blastula trophoblast layer, cultured cells, extracted gDNA or blastula culture medium.

11. The method of claim 1, wherein the first random sequence and the third random sequence have the same length, or wherein the first random sequence and the third random sequence have different lengths.

12. The method of claim 1, wherein steps (b) and (d) are as follows:
(b) placing the first reaction mixture in a first thermal cycle program, such that the first variable sequence of the first primers and the third variable sequence of the third primers are capable of pairing with the genomic DNA and the genomic DNA is amplified to obtain a genomic pre-amplification product, wherein the genomic pre-amplification product comprises the common sequence at its 5' end and a complementary sequence of the common sequence at its 3' end; wherein the first thermal cycle program comprises:
(b1) for the first cycle, reacting at a first denaturing temperature at a temperature between 90-95°C for 1-20 min; for the cycle following the first cycle, reacting at a first denaturing temperature at a temperature between 90-95°C for 3-50 s;
(b2) reacting at a first annealing temperature between 10-20°C for 3-60 s, reacting at a second annealing temperature between 20-30°C for 3-50 s, and reacting at a third annealing temperature between 30-50°C for 3-50 s;
(b3) reacting at a first extension temperature between 60-80°C for 10 s-15 min;
(b4) repeating steps (b1) to (b3) for 2-40 cycles. and
(d) placing the second reaction mixture in a second thermal cycle program, such that the common sequence of the second primer is capable of pairing with 3' end of the genomic pre-amplification product and the genomic pre-amplification product is amplified to obtain an extended genomic amplification product, wherein the second thermal cycle program comprises:
(d1) reacting at a second denaturing temperature between 90-95°C for 5 s-20 min;
(d2) reacting at a second melting temperature between 90-95°C for 3-50 s;
(d3) reacting at a fourth annealing temperature between 45-65°C for 3-50 s;
(d4) reacting at a second extension temperature between 60-80°C for 10 s-15 min;
(d5) repeating steps (d2) to (d4) for 2-40 cycles.

13. The method of claim 12, wherein the common sequence comprises or consists of SEQ ID NO: 6;
Xₐᵢ (i=1-n) of the first random sequence all belong to D, X_{bi} (i=1-n) of the third random sequence all belong to B.

14. A kit for amplifying genomic DNA, said kit comprises primers, wherein the primers consist of first primers and third primers,
wherein the first primers consist of, in a 5' to 3' orientation, a common sequence and a first variable sequence, or wherein the first primers consist of, in a 5' to 3' orientation, a common sequence, a first spacer sequence and a first variable sequence, wherein the first primers are a mixture of primers comprising the same common sequence and different first variable sequences, wherein each first variable sequence consists of a first random sequence and a fixed sequence at its 3' end, wherein the first random sequence is, in a 5' to 3' orientation, sequentially Xₐ₁Xₐ₂......Xₐₙ, and Xₐᵢ (i=1-n) of the first random sequence all belong to a same set, said set is selected from B , or D, or H, or V, wherein B={T, G, C}, D={A, T, G}, H={T, A, C}, V={A, C, G}, wherein Xₐᵢ represents the i^{th} nucleotide from 5' end of a first random sequence, n is a positive integer selected from 4-16, and wherein the first spacer sequence is Yₐ₁......Yₐₘ, wherein Yₐⱼ (j=1-m) ∈ {A, T, G, C}, wherein Yₐⱼ represents the j^{th} nucleotide from 5' end of the first spacer sequence, m is a positive integer selected from 1-3,
wherein the third primers consist of, in a 5' to 3' orientation, the common sequence and a third variable sequence, or wherein the third primers consist of, in a 5' to 3' orientation, the common sequence, a third spacer sequence and a third variable sequence, wherein the third primers are a mixture of primers comprising the same common sequence and different third variable sequences, wherein each third variable sequence consists of a third random sequence and a fixed sequence at its 3' end, wherein the third random sequence is, in a 5' to 3' orientation, sequentially X_{b1}X_{b2}......X_{bn}, and X_{bi} (i=1-n) of the third random sequence all belong to a same set, said set is selected from B , or D, or H, or V, wherein B={T, G, C}, D={A, T, G}, H={T, A, C}, V={A, C, G}, and X_{bi} (i=1-n) and Xₐᵢ (i=1-n) belong to different sets, wherein X_{bi} represents the i^{th} nucleotide from 5' end of the third random sequence, n is a positive integer selected from 4-16, and wherein the third spacer sequence is Y_{b1}......Y_{bm}, wherein Y_{bj} (j=1-m) ∈ {A, T, G, C}, wherein Y_{bj} represents the j^{th} nucleotide from 5' end of the third spacer sequence, m is a positive integer selected from 1-3.

## Patentansprüche

1. Verfahren zum Amplifizieren genomischer DNA, wobei das Verfahren Folgendes umfasst:
(a) Bereitstellen einer ersten Reaktionsmischung, wobei die erste Reaktionsmischung eine Probe, die die genomische DNA enthält, Primer, eine Mischung von Nukleotidmonomeren und eine Nukleinsäurepolymerase umfasst, wobei die Primer aus ersten Primern und dritten Primern bestehen,
wobei die ersten Primer in 5'-3'-Orientierung aus einer gemeinsamen Sequenz und einer ersten variablen Sequenz bestehen, oder die ersten Primer in 5'-3'-Orientierung aus einer gemeinsamen Sequenz, einer ersten Spacersequenz und einer ersten variablen Sequenz bestehen, wobei die ersten Primer eine Mischung von Primern sind, die die gleiche gemeinsame Sequenz und unterschiedliche erste variable Sequenzen umfassen, wobei jede erste variable Sequenz aus einer ersten Zufallssequenz und einer festen Sequenz an ihrem 3'-Ende besteht, wobei die erste Zufallssequenz, in 5'-3'-Orientierung, sequentiell Xₐ₁Xₐ₂......Xₐₙ ist, und Xₐᵢ (i=1-n) der ersten Zufallssequenz alle zu einem gleichen Satz gehören, wobei der Satz aus B oder D oder H oder V ausgewählt ist, wobei B={T, G, C}, D={A, T, G}, H={T, A, C}, V={A, C, G}, wobei Xₐᵢ das i^{te} Nukleotid vom 5'-Ende der ersten Zufallssequenz aus darstellt, n eine positive ganze Zahl, ausgewählt aus 4-16, ist und wobei die erste Spacersequenz Yₐ₁......Yₐₘ ist, wobei Yₐⱼ (j=1-m) ∈ {A, T, G, C}, wobei Yₐⱼ das j^{te} Nukleotid vom 5'-Ende der ersten Spacersequenz aus darstellt, m eine positive ganze Zahl, ausgewählt aus 1-3, ist;
wobei die dritten Primer in 5'-3'-Orientierung aus der gemeinsamen Sequenz und einer dritten variablen Sequenz bestehen, oder wobei die dritten Primer in 5'-3'-Orientierung aus der gemeinsamen Sequenz, einer dritten Spacersequenz und einer dritten variablen Sequenz bestehen, wobei die dritten Primer eine Mischung von Primern sind, die die gleiche gemeinsame Sequenz und unterschiedliche dritte variable Sequenzen umfassen, wobei jede dritte variable Sequenz aus einer dritten Zufallssequenz und einer festen Sequenz an ihrem 3'-Ende besteht, wobei die dritte Zufallssequenz in 5'-3'-Orientierung sequentiell X_{b1}X_{b2}......X_{bn} ist, und X_{bi} (i=1-n) der dritten Zufallssequenz alle zu einem gleichen Satz gehören, wobei der Satz aus B-, oder D oder H oder V ausgewählt ist, wobei B={T, G, C}, D={A, T, G}, H={T, A, C}, V={A, C, G}, und X_{bi} (i=1-n) und Xₐᵢ (i=1-n) zu verschiedenen Sätzen gehören, wobei X_{bi} das i^{te} Nukleotid vom 5'-Ende der dritten Zufallssequenz aus darstellt, n eine positive ganze Zahl, ausgewählt aus 4-16, ist, und wobei die dritte Spacersequenz Y_{b1}......Y_{bm} ist, wobei Y_{bj} (j=1-m) ∈ {A, T, G, C}, wobei Y_{bj} das j^{te} Nukleotid vom 5'-Ende der dritten Spacersequenz aus darstellt, m eine positive ganze Zahl, ausgewählt aus 1-3, ist;
(b) Platzieren der ersten Reaktionsmischung in ein erstes Thermozyklusprogramm zur Voramplifikation, um ein Voramplifikationsprodukt zu erhalten;
(c) Bereitstellen einer zweiten Reaktionsmischung, wobei die zweite Reaktionsmischung das aus Schritt (b) erhaltene Voramplifikationsprodukt, einen zweiten Primer, eine Mischung von Nukleotidmonomeren und eine Nukleinsäurepolymerase umfasst, wobei der zweite Primer in 5'-3'-Orientierung eine spezifische Sequenz und die gemeinsame Sequenz umfasst oder daraus besteht;
(d) Platzieren der zweiten Reaktionsmischung in ein zweites Thermozyklusprogramm zur Amplifikation, um ein Amplifikationsprodukt zu erhalten.

2. Verfahren nach Anspruch 1,
(a) wobei Xₐᵢ (i=1-n) der ersten Zufallssequenz alle zu Satz B gehören, X_{bi} (i=1-n) der dritten Zufallssequenz alle zu Satz D gehören; oder
(b) wobei die feste Sequenz individuell ausgewählt ist aus der Gruppe bestehend aus CCC, AAA, TGGG, GTTT, GGG, TTT, TNTNG oder GTGG; oder
(c) wobei die erste variable Sequenz ausgewählt ist aus Xₐ₁Xₐ₂......XₐₙTGGG oder Xₐ₁Xₐ₂......XₐₙGTTT, die dritte variable Sequenz ausgewählt ist aus X_{b1}X_{b2}......X_{bn}TGGG oder X_{b1}X_{b2}......X_{bn}GTTT; oder
(d) wobei die gemeinsame Sequenz ausgewählt ist aus SEQ ID NO: 1 [TTGGTAGTGAGTG], SEQ ID NO: 2 [GAGGTGTGATGGA], SEQ ID NO: 3 [GTGATGGTTGAGGTA], SEQ ID NO: 4 [AGATGTGTATAAGAGACAG], SEQ ID NO: 5 [GTGAGTGATGGTTGAGGTAGTGTGGAG] oder SEQ ID NO: 6 [GCTCTTCCGATCT]; oder
(e) wobei das Verfahren ferner einen Schritt des Sequenzierens eines in Schritt (d) erhaltenen Amplifikationsprodukts umfasst, wobei der zweite Primer eine Sequenz umfasst, die komplementär oder identisch zu einem Teil oder der Gesamtheit eines Primers ist, der zur Sequenzierung verwendet wird; oder
(f) wobei der zweite Primer eine Primermischung mit identischen gemeinsamen Sequenzen und unterschiedlichen spezifischen Sequenzen umfasst, wobei die unterschiedlichen spezifischen Sequenzen komplementär oder identisch zu einem Teil oder der Gesamtheit von verschiedenen Primern in Sequenzierungsprimerpaaren sind, die jeweils in einer gleichen Sequenzierung verwendet werden; oder
(g) wobei der zweite Primer eine Mischung von Sequenzen umfasst, die in SEQ ID NO: 35 [AATGATACGGCGACCACCGAGATCTACACTCTTTCCCTACACGACGCTCTTCCG ATCT] und SEQ ID NO: 36 [CAAGCAGAAGACGGCATACGAGATCGTGATGTGACTGGAGTTCAGACGTGTGCT CTTCCGATCT] dargelegt sind; oder
(h) wobei Schritt (b) ermöglicht, dass die variable Sequenz der ersten Primer mit der genomischen DNA paart und die genomische DNA amplifiziert wird, um ein genomisches Voramplifikationsprodukt zu erhalten, wobei das genomische Voramplifikationsprodukt die gemeinsame Sequenz an seinem 5'-Ende und eine komplementäre Sequenz der gemeinsamen Sequenz an seinem 3'-Ende umfasst; wobei das erste Thermozyklusprogramm Folgendes umfasst:
(b1) ein thermisches Programm, das in der Lage ist, die DNA-Doppelstränge zu öffnen, um ein einzelsträngiges DNA-Template zu erhalten;
(b2) ein thermisches Programm, das die Bindung der ersten Primer und der dritten Primer an das einzelsträngige DNA-Template ermöglicht;
(b3) ein thermisches Programm, das die Verlängerung der Länge der ersten Primer, die an das einzelsträngige DNA-Template binden, unter der Wirkung der Nukleinsäurepolymerase ermöglicht, um ein Voramplifikationsprodukt herzustellen;
(b4) Wiederholen der Schritte (b1) bis (b3) zu einer designierten ersten Zykluszahl, wobei die designierte erste Zykluszahl größer als 1 ist; oder
(i) wobei der Schritt (d) ermöglicht, dass die gemeinsame Sequenz des zweiten Primers mit dem 3'-Ende des genomischen Voramplifikationsprodukts paart und das genomische Voramplifikationsprodukt amplifiziert wird, um ein verlängertes genomisches Amplifikationsprodukt zu erhalten;
wobei der Schritt (d) Folgendes umfasst:
(d1) ein thermisches Programm, das DNA-Doppelstränge öffnen kann;
(d2) ein thermisches Programm, das weiter in der Lage ist, DNA-Doppelstränge zu öffnen;
(d3) ein thermisches Programm, das die Bindung des zweiten Primers an einen Einzelstrang des in Schritt (b) erhaltenen genomischen Voramplifikationsprodukts ermöglicht;
(d4) ein Temperaturprogramm, das die Verlängerung der Länge des zweiten Primers, der an den Einzelstrang des genomischen Voramplifikationsprodukts bindet, unter der Wirkung der Nukleinsäurepolymerase ermöglicht;
(d5) Wiederholen der Schritte (d2) bis (d4) zu einer designierten zweiten Zykluszahl, wobei die designierte zweite Zykluszahl größer als 1 ist; oder
(j) ferner umfassend das Analysieren des Amplifikationsprodukts, um Krankheits- oder Phänotypassoziierte Sequenzmerkmale zu identifizieren.

3. Verfahren nach Anspruch 1, wobei die ersten Primer GCTCTTCCGATCTYₐ₁Xₐ₁Xₐ₂Xₐ₃Xₐ₄Xₐ₅TGGG, GCTCTTCCGATCTYₐ₁Xₐ₁Xₐ₂Xₐ₃Xₐ₄Xₐ₅GTTT oder eine Kombination davon umfassen,
die dritten Primer GCTCTTCCGATCTY_{b1}X_{b1}X_{b2}X_{b3}X_{b4}X_{b5}TGGG, GCTCTTCCGATCTY_{b1}X_{b1}X_{b2}X_{b3}X_{b4}X_{b5}GTTT, oder eine Kombination davon, wobei Yₐ₁ ∈ {A, T, G, C}, Y_{b1} ∈ {A, T, G, C}, Xₐᵢ (i=1-5) ∈ {T, G, C}, X_{bi} (i=1-5) ∈ {A, T, G} umfassen.

4. Verfahren nach Anspruch 2(e), wobei (a) die gemeinsame Sequenz eine Sequenz umfasst, die komplementär oder identisch zu einem Teil oder der Gesamtheit eines Primers ist, der zur Sequenzierung verwendet wird; oder (b) die spezifische Sequenz des zweiten Primers eine Sequenz umfasst, die komplementär oder identisch zu einem Teil oder der Gesamtheit eines Primers ist, der zur Sequenzierung verwendet wird.

5. Verfahren nach Anspruch 4(b), wobei (a) die spezifische Sequenz des zweiten Primers ferner eine Sequenz umfasst, die komplementär oder identisch zu einem Teil oder der Gesamtheit einer Einfangsequenz einer Sequenzierungsplattform ist; oder (b) die Sequenz, die in der spezifischen Sequenz des zweiten Primers enthalten ist und komplementär oder identisch zu einem Teil oder der Gesamtheit eines Primers ist, der zur Sequenzierung verwendet wird, SEQ ID NO: 31 [ACACTCTTTCCCTACACGAC], oder SEQ ID NO: 32 [GTGACTGGAGTTCAGACGTGT] umfasst oder daraus besteht.

6. Verfahren nach Anspruch 5(b), wobei
(a) die Sequenz, die in der spezifischen Sequenz des zweiten Primers enthalten ist und komplementär oder identisch zu einem Teil oder der Gesamtheit einer Einfangsequenz einer Sequenzierungsplattform ist, SEQ ID NO: 33 [AATGATACGGCGACCACCGAGATCT] oder SEQ ID NO: 34 [CAAGCAGAAGACGGCATACGAGAT] umfasst oder daraus besteht; oder
(b) die spezifische Sequenz des zweiten Primers ferner eine Barcode-Sequenz umfasst, wobei die Barcode-Sequenz zwischen der Sequenz, die komplementär oder identisch zu einem Teil oder der Gesamtheit einer Einfangsequenz einer Sequenzierungsplattform ist, und der Sequenz, die komplementär oder identisch zu einem Teil oder der Gesamtheit eines Primers ist, der zur Sequenzierung verwendet wird, angeordnet ist.

7. Verfahren nach Anspruch 2(h), wobei, beim Durchlaufen des ersten Zyklus, die DNA-Doppelstränge in Schritt (b1) genomische DNA-Doppelstränge sind, das thermische Programm eine Denaturierungsreaktion bei einer Temperatur zwischen 90-95 °C für 1-20 Minuten umfasst und nach dem ersten Zyklus das thermische Programm in Schritt (b1) eine Schmelzreaktion bei einer Temperatur zwischen 90-95 °C für 3-50 Sekunden umfasst; wobei, nach Durchlaufen eines zweiten Zyklus, das Voramplifikationsprodukt ein genomisches Voramplifikationsprodukt umfasst, das die gemeinsame Sequenz an seinem 5'-Ende und eine komplementäre Sequenz der gemeinsamen Sequenz an seinem 3'-Ende umfasst.

8. Verfahren nach Anspruch 2(h), wobei nach Schritt (b1) und vor Schritt (b2) das Verfahren keinen zusätzlichen Schritt des Platzierens der ersten Reaktionsmischung in ein geeignetes thermisches Programm umfasst, so dass das 3'-Ende und das 5'-Ende des genomischen Voramplifikationsprodukts hybridisieren, um eine Haarnadelstruktur auszubilden.

9. Verfahren nach Anspruch 2(j), wobei die Krankheits- oder Phänotyp-assoziierten Sequenzmerkmale chromosomale Anomalien, chromosomale Translokation, Aneuploidie, partielle oder vollständige chromosomale Deletion oder Duplikation, fötale HLA-Haplotypen und paternale Mutationen umfassen oder die Krankheit oder der Phänotyp ausgewählt ist aus der Gruppe bestehend aus: Beta-Thalassämie, Down-Syndrom, zystische Fibrose, Sichelzellerkrankung, Tay-Sachs-Erkrankung, fragiles-X-Syndrom, spinaler Muskelatrophie, Hämoglobinopathie, Alpha-Thalassämie, X-verknüpften Krankheiten (Erkrankungen, die von Genen auf dem X-Chromosom dominiert werden), Spina bifida, Anenzephalie, angeborener Herzerkrankung, Fettleibigkeit, Diabetes, Krebs, fötalem Geschlecht und fötaler RHD.

10. Verfahren nach Anspruch 2(j), wobei die genomische DNA aus einem Blastomer, einer Blastula-Trophoblastenschicht, kultivierten Zellen, extrahierter gDNA oder einem Blastula-Kulturmedium abgeleitet ist.

11. Verfahren nach Anspruch 1, wobei die erste Zufallssequenz und die dritte Zufallssequenz die gleiche Länge aufweisen oder wobei die erste Zufallssequenz und die dritte Zufallssequenz unterschiedliche Längen aufweisen.

12. Verfahren nach Anspruch 1, wobei die Schritte (b) und (d) wie folgt sind:
(b) Platzieren der ersten Reaktionsmischung in ein erstes Thermozyklusprogramm, so dass die erste variable Sequenz der ersten Primer und die dritte variable Sequenz der dritten Primer in der Lage sind, mit der genomischen DNA zu paaren, und die genomische DNA amplifiziert wird, um ein genomisches Voramplifikationsprodukt zu erhalten, wobei das genomische Voramplifikationsprodukt die gemeinsame Sequenz an seinem 5'-Ende und eine komplementäre Sequenz der gemeinsamen Sequenz an seinem 3'-Ende umfasst; wobei das erste Thermozyklusprogramm Folgendes umfasst:
(b1) für den ersten Zyklus, Umsetzen (Reagierenlassen) bei einer ersten Denaturierungstemperatur mit einer Temperatur zwischen 90-95 °C für 1-20 min; für den Zyklus nach dem ersten Zyklus, Umsetzen bei einer ersten Denaturierungstemperatur mit einer Temperatur zwischen 90-95°C für 3-50 s;
(b2) Umsetzen bei einer ersten Annealingtemperatur zwischen 10-20 °C für 3-60 s, Umsetzen bei einer zweiten Annealingtemperatur zwischen 20-30 °C für 3-50 s, und
Umsetzen bei einer dritten Annealingtemperatur zwischen 30-50 °C für 3-50 s;
(b3) Umsetzen bei einer ersten Verlängerungstemperatur zwischen 60-80 °C für 10 s-15 min;
(b4) Wiederholen der Schritte (b1) bis (b3) für 2-40 Zyklen,
und
(d) Platzieren der zweiten Reaktionsmischung in ein zweites Thermozyklusprogramm, so dass die gemeinsame Sequenz des zweiten Primers in der Lage ist, mit dem 3'-Ende des genomischen Voramplifikationsprodukts zu paaren, und das genomische Voramplifikationsprodukt amplifiziert wird, um ein verlängertes genomisches Amplifikationsprodukt zu erhalten, wobei das zweite Thermozyklusprogramm Folgendes umfasst:
(d1) Umsetzen bei einer zweiten Denaturierungstemperatur zwischen 90-95 °C für 5 s-20 min;
(d2) Umsetzen bei einer zweiten Schmelztemperatur zwischen 90-95 °C für 3-50 s;
(d3) Umsetzen bei einer vierten Annealingtemperatur zwischen 45-65 °C für 3-50 s;
(d4) Umsetzen bei einer zweiten Verlängerungstemperatur zwischen 60-80 °C für 10 s-15 min;
(d5) Wiederholen der Schritte (d2) bis (d4) für 2-40 Zyklen.

13. Verfahren nach Anspruch 12, wobei die gemeinsame Sequenz SEQ ID NO: 6 umfasst oder daraus besteht;
Xₐᵢ (i=1-n) der ersten Zufallssequenz alle zu D gehören,
X_{bi} (i=1-n) der dritten Zufallssequenz alle zu B gehören.

14. Kit zum Amplifizieren genomischer DNA, wobei das Kit Primer umfasst, wobei die Primer aus ersten Primern und dritten Primern bestehen,
wobei die ersten Primer in 5'-3'-Orientierung aus einer gemeinsamen Sequenz und einer ersten variablen Sequenz bestehen, oder wobei die ersten Primer in 5'-3'-Orientierung aus einer gemeinsamen Sequenz, einer ersten Spacersequenz und einer ersten variablen Sequenz bestehen, wobei die ersten Primer eine Mischung von Primern sind, die die gleiche gemeinsame Sequenz und unterschiedliche erste variable Sequenzen umfassen, wobei jede erste variable Sequenz aus einer ersten Zufallssequenz und einer festen Sequenz an ihrem 3'-Ende besteht, wobei die erste Zufallssequenz, in 5'-3'-Orientierung, sequentiell Xₐ₁Xₐ₂......Xₐₙ ist, und Xₐᵢ (i=1-n) der ersten Zufallssequenz alle zu einem gleichen Satz gehören, wobei der Satz aus B oder D oder H oder V ausgewählt ist, wobei B={T, G, C}, D={A, T, G}, H={T, A, C}, V={A, C, G}, wobei Xₐᵢ das i^{te} Nukleotid vom 5'-Ende der ersten Zufallssequenz aus darstellt, n eine positive ganze Zahl, ausgewählt aus 4-16, ist und wobei die erste Spacersequenz Yₐ₁......Yₐₘ ist, wobei Yₐⱼ (j=1-m) ∈ {A, T, G, C}, wobei Yₐⱼ das j^{te} Nukleotid vom 5'-Ende der ersten Spacersequenz aus darstellt, m eine positive ganze Zahl, ausgewählt aus 1-3, ist,
wobei die dritten Primer in 5'-3'-Orientierung aus der gemeinsamen Sequenz und einer dritten variablen Sequenz bestehen, oder wobei die dritten Primer in 5'-3'-Orientierung aus der gemeinsamen Sequenz, einer dritten Spacersequenz und einer dritten variablen Sequenz bestehen, wobei die dritten Primer eine Mischung von Primern sind, die die gleiche gemeinsame Sequenz und unterschiedliche dritte variable Sequenzen umfassen, wobei jede dritte variable Sequenz aus einer dritten Zufallssequenz und einer festen Sequenz an ihrem 3'-Ende besteht, wobei die dritte Zufallssequenz in 5'-3'-Orientierung sequentiell X_{b1}X_{b2}......X_{bn} ist, und X_{bi} (i=1-n) der dritten Zufallssequenz alle zu einem gleichen Satz gehören, wobei der Satz aus B oder D oder H oder V ausgewählt ist, wobei B={T, G, C}, D={A, T, G}, H={T, A, C}, V={A, C, G}, und X_{bi} (i=1-n) und Xₐᵢ (i=1-n) zu verschiedenen Sätzen gehören, wobei X_{bi} das i^{te} Nukleotid vom 5'-Ende der dritten Zufallssequenz aus darstellt, n eine positive ganze Zahl, ausgewählt aus 4-16, ist, und wobei die dritte Spacersequenz Y_{b1}......Y_{bm} ist, wobei Y_{bj} (j=1-m) ∈ {A, T, G, C}, wobei Y_{bj} das j^{te} Nukleotid vom 5'-Ende der dritten Spacersequenz aus darstellt, m eine positive ganze Zahl, ausgewählt aus 1-3, ist.

## Revendications

1. Procédé d'amplification d'ADN génomique, ledit procédé comprenant :
(a) la fourniture d'un premier mélange réactionnel, le premier mélange réactionnel comprenant un échantillon contenant l'ADN génomique, des amorces, un mélange de monomères nucléotidiques, et une acide nucléique polymérase, les amorces étant constituées de premières amorces et de troisièmes amorces,
dans lequel les premières amorces sont constituées, dans une orientation 5'-3', d'une séquence commune et d'une première séquence variable, ou les premières amorces sont constituées, dans une orientation 5'-3', d'une séquence commune, d'une première séquence d'espaceur et d'une première séquence variable, les premières amorces étant un mélange d'amorces comprenant la même séquence commune et des premières séquences variables différentes, chaque première séquence variable étant constituée d'une première séquence aléatoire et d'une séquence fixe à son extrémité 3', la première séquence aléatoire étant, dans une orientation 5'-3', séquentiellement Xₐ₁Xₐ₂......Xₐₙ et Xₐᵢ (i = 1 à n) de la première séquence aléatoire appartiennent tous à un même ensemble, ledit ensemble étant choisi parmi B ou D ou H ou V, dans lequel B = {T, G, C}, D = {A, T, G}, H = {T, A, C}, V = {A, C, G}, Xₐᵢ représentant le i^{ème} nucléotide à partir de l'extrémité 5' de la première séquence aléatoire, n étant un entier positif de 4 à 16, et dans lequel la première séquence d'espaceur est Yₐ₁......Yₐₘ, dans laquelle Yₐⱼ (j = 1 à m) E {A, T, G, C}, Yₐⱼ représentant le j^{ème} nucléotide à partir de l'extrémité 5' de la première séquence d'espaceur, m étant un entier positif de 1 à 3 ;
dans lequel les troisièmes amorces sont constituées, dans une orientation 5'-3', de la séquence commune et d'une troisième séquence variable, ou dans lequel les troisièmes amorces sont constituées, dans une orientation 5'-3', de la séquence commune, d'une troisième séquence d'espaceur et d'une troisième séquence variable, les troisièmes amorces étant un mélange d'amorces comprenant la même séquence commune et des troisièmes séquences variables différentes, chaque troisième séquence variable étant constituée d'une troisième séquence aléatoire et d'une séquence fixe à son extrémité 3', la troisième séquence aléatoire étant, dans une orientation 5'-3', séquentiellement X_{b1}X_{b2}......X_{bn}, et X_{bi} (i = 1 à n) de la troisième séquence aléatoire appartiennent tous à un même ensemble, ledit ensemble étant choisi parmi B ou D ou H ou V, dans lequel B = {T, G, C}, D = {A, T, G}, H = {T, A, C}, V = {A, C, G}, et X_{bi} (i = 1 à n) et Xₐᵢ (i = 1 à n) appartiennent à des ensembles différents, X_{bi} représentant le i^{ème} nucléotide à partir de l'extrémité 5' de la troisième séquence aléatoire, n étant un entier positif de 4 à 16, et la troisième séquence d'espaceur étant Y_{b1}......Y_{bm}, dans laquelle Y_{bj} (j = 1 à m) E {A, T, G, C}, Y_{bj} représentant le j^{ème} nucléotide à partir de l'extrémité 5' de la troisième séquence d'espaceur, m étant un entier positif de 1 à 3 ;
(b) le placement du premier mélange réactionnel dans un premier programme de cycle thermique de préamplification, afin d'obtenir un produit de préamplification ;
(c) la fourniture d'un deuxième mélange réactionnel, ledit deuxième mélange réactionnel comprenant ledit produit de préamplification obtenu à l'étape (b), une deuxième amorce, un mélange de monomères nucléotidiques, et une acide nucléique polymérase, la deuxième amorce comprenant ou étant constituée, dans une orientation 5'-3', d'une séquence spécifique et de la séquence commune ;
(d) le placement du deuxième mélange réactionnel dans un deuxième programme de cycle thermique d'amplification, afin d'obtenir un produit d'amplification.

2. Procédé selon la revendication 1,
(a) dans lequel Xₐᵢ (i = 1 à n) de la première séquence aléatoire appartiennent tous à l'ensemble B, X_{bi} (i = 1 à n) de la troisième séquence aléatoire appartiennent tous à l'ensemble D ; ou
(b) dans lequel la séquence fixe est individuellement choisie dans le groupe constitué par CCC, AAA, TGGG, GTTT, GGG, TTT, TNTNG ou GTGG ; ou
(c) dans lequel la première séquence variable est choisie parmi Xₐ₁Xₐ₂......XₐₙTGGG ou Xₐ₁Xₐ₂......XₐₙGTTT, la troisième séquence variable est choisie parmi X_{b1}X_{b2}......X_{bn}TGGG ou X_{b1}X_{b2}......X_{bn}GTTT ; ou
(d) dans lequel la séquence commune est choisie parmi SEQ ID NO : 1 [TTGGTAGTGAGTG], SEQ ID NO : 2 [GAGGTGTGATGGA], SEQ ID NO : 3 [GTGATGGTTGAGGTA], SEQ ID NO : 4 [AGATGTGTATAAGAGACAG], SEQ ID NO : 5 [GTGAGTGATGGTTGAGGTAGTGTGGAG] ou SEQ ID NO : 6 [GCTCTTCCGATCT] ; ou
(e) le procédé comprenant en outre une étape de séquençage d'un produit d'amplification obtenu à l'étape (d), dans lequel la deuxième amorce comprend une séquence complémentaire ou identique à une partie ou à la totalité d'une amorce utilisée pour le séquençage ; ou
(f) dans lequel la deuxième amorce comprend un mélange d'amorces ayant des séquences communes identiques et des séquences spécifiques différentes, lesdites différentes séquences spécifiques étant complémentaires d'une partie ou de la totalité des différentes amorces dans les paires d'amorces de séquençage utilisées dans un même séquençage ou identiques à celles-ci, respectivement ; ou
(g) dans lequel la deuxième amorce comprend un mélange de séquences décrites dans SEQ ID NO : 35 [AATGATACGGCGACCACCGAGATCTACACTCTTTCCCTACACGACGCTCTTCCG ATCT] et SEQ ID NO : 36 [CAAGCAGAAGACGGCATACGAGATCGTGATGTGACTGGAGTTCAGACGTGTGCT CTTCCGATCT] ; ou
(h) dans lequel l'étape (b) permet à la séquence variable des premières amorces de s'apparier avec l'ADN génomique et l'ADN génomique est amplifié pour obtenir un produit de préamplification génomique, le produit de préamplification génomique comprenant la séquence commune à son extrémité 5' et une séquence complémentaire de la séquence commune à son extrémité 3' ;
dans lequel le premier programme de cycle thermique comprend :
(b1) un programme thermique permettant d'ouvrir les doubles brins d'ADN pour obtenir une matrice d'ADN simple brin ;
(b2) un programme thermique qui permet la liaison des premières amorces et des troisièmes amorces à la matrice d'ADN simple brin ;
(b3) un programme thermique qui permet l'extension de la longueur des premières amorces qui se lient à la matrice d'ADN simple brin sous l'action de l'acide nucléique polymérase, pour produire un produit de préamplification ;
(b4) la répétition des étapes (b1) à (b3) jusqu'à un premier nombre de cycles désigné, le premier nombre de cycle désigné étant supérieur à 1 ; ou
(i) dans lequel l'étape (d) permet à la séquence commune de la deuxième amorce de s'apparier à l'extrémité 3' du produit de préamplification génomique et le produit de préamplification génomique est amplifié pour obtenir un produit d'amplification génomique étendu ;
dans lequel l'étape (d) comprend :
(d1) un programme thermique permettant d'ouvrir des doubles brins d'ADN ;
(d2) un programme thermique permettant d'ouvrir davantage des doubles brins d'ADN ;
(d3) un programme thermique qui permet la liaison de la deuxième amorce au simple brin du produit de préamplification génomique obtenu à l'étape (b) ;
(d4) un programme de température qui permet l'extension de la longueur de la deuxième amorce qui se lie au simple brin du produit de préamplification génomique, sous l'action de l'acide nucléique polymérase ;
(d5) la répétition des étapes (d2) à (d4) jusqu'à un deuxième nombre de cycles désigné, le premier nombre de cycles désigné étant supérieur à 1 ; ou
(j) comprenant en outre l'analyse du produit d'amplification pour identifier des caractéristiques de séquence associées à une maladie ou à un phénotype.

3. Procédé selon la revendication 1, dans lequel les premières amorces comprennent GCTCTTCCGATCTYₐ₁Xₐ₁Xₐ₂Xₐ₃Xₐ₄Xₐ₅TGGG, GCTCTTCCGATCTYₐ₁Xₐ₁Xₐ₂Xₐ₃Xₐ₄Xₐ₅GTTT, ou une combinaison de ceux-ci,
Les troisièmes amorces comprennent GCTCTTCCGATCTY_{b1}X_{b1}X_{b2}X_{b3}X_{b4}X_{b5}TGGG, GCTCTTCCGATCTY_{b1}X_{b1}X_{b2}X_{b3}X_{b4}X_{b5}GTTT, ou une combinaison de ceux-ci, dans lequel Yₐ₁ E {A, T, G, C}, Y_{b1} E {A, T, G, C}, ledit Xₐᵢ (i=1-5) E {T, G, C}, ledit X_{bi} (i=1-5) E {A, T, G}.

4. Procédé selon la revendication 2(e), dans lequel (a) la séquence commune comprend une séquence complémentaire ou identique à une partie ou à la totalité d'une amorce utilisée pour le séquençage ; ou
(b) la séquence spécifique de la deuxième amorce comprend une séquence complémentaire ou identique à une partie ou à la totalité d'une amorce utilisée pour le séquençage.

5. Procédé selon la revendication 4(b), dans lequel (a) la séquence spécifique de la deuxième amorce comprend en outre une séquence complémentaire ou identique à une partie ou à la totalité d'une séquence de capture d'une plateforme de séquençage ; ou (b) la séquence qui est comprise dans la séquence spécifique de la deuxième amorce et complémentaire ou identique à une partie ou à la totalité d'une amorce utilisée pour le séquençage comprend ou est constituée de SEQ ID NO : 31 [ACACTCTTTCCCTACACGAC], ou SEQ ID NO : 32 [GTGACTGGAGTTCAGACGTGT].

6. Procédé selon la revendication 5(b), dans lequel
(a) la séquence comprise dans la séquence spécifique de la deuxième amorce et complémentaire ou identique à une partie ou à la totalité d'une séquence de capture d'une plateforme de séquençage, comprend ou est constituée de SEQ ID NO : 33 [AATGATACGGCGACCACCGAGATCT], ou SEQ ID NO : 34 [CAAGCAGAAGACGGCATACGAGAT] ; ou
(b) la séquence spécifique de la deuxième amorce comprend en outre une séquence de code-barres, ladite séquence de code-barres est située entre la séquence complémentaire ou identique à une partie ou à la totalité d'une séquence de capture d'une plateforme de séquençage et la séquence complémentaire ou identique à une partie ou à la totalité d'une amorce utilisée pour le séquençage.

7. Procédé selon la revendication 2(h), dans lequel, après avoir subi le premier cycle, les doubles brins d'ADN à l'étape (b1) sont des doubles brins d'ADN génomique, le programme thermique comprend une réaction de dénaturation à une température comprise entre 90 et 95°°C pendant 1 à 20 minutes et après le premier cycle, le programme thermique à l'étape (b1) comprend une réaction de fusion à une température comprise entre 90 et 95°°C pendant 3 à 50 secondes ; dans lequel, après avoir subi un deuxième cycle, le produit de préamplification comprend un produit de préamplification génomique comprenant la séquence commune à son extrémité 5' et une séquence complémentaire de la séquence commune à son extrémité 3'.

8. Procédé selon la revendication 2(h), dans lequel, après l'étape (b1) et avant l'étape (b2), ledit procédé ne comprend pas une étape supplémentaire de placement du premier mélange réactionnel dans un programme thermique approprié, de telle sorte que l'extrémité 3' et l'extrémité 5' du produit de préamplification génomique s'hybrident pour former une structure en épingle à cheveux.

9. Procédé selon la revendication 2(j), dans lequel les caractéristiques de séquence associées à une maladie ou à un phénotype comprennent des anomalies chromosomiques, une translocation chromosomique, une aneuploïdie, une délétion ou duplication chromosomique partielle ou complète, des haplotypes HLA fœtaux et des mutations paternelles, ou la maladie ou le phénotype est choisi dans le groupe constitué par : la bêta-thalassémie, le syndrome de Down, la mucoviscidose, la drépanocytose, la maladie de Tay-Sachs, le syndrome du X fragile, l'atrophie musculaire spinale, l'hémoglobinopathie, l'alpha-thalassémie, les maladies liées à l'X (maladies dominées par les gènes sur le chromosome X), la spina bifida, l'anencéphalie, une cardiopathie congénitale, l'obésité, le diabète, le cancer, le sexe fœtal et la RHD fœtale.

10. Procédé selon la revendication 2(j), dans lequel l'ADN génomique est issu d'un blastomère, d'une couche de trophoblaste de la blastula, de cellules en culture, d'ADNg extrait ou de milieu de culture de blastula.

11. Procédé selon la revendication 1, dans lequel la première séquence aléatoire et la troisième séquence aléatoire ont la même longueur, ou dans lequel la première séquence aléatoire et la troisième séquence aléatoire ont des longueurs différentes.

12. Procédé selon la revendication 1, dans lequel les étapes (b) et (d) sont les suivantes :
(b) le placement du premier mélange réactionnel dans un premier programme de cycle thermique, de sorte que la première séquence variable des premières amorces et la troisième séquence variable des troisièmes amorces peuvent s'apparier à l'ADN génomique et l'ADN génomique est amplifié pour obtenir un produit de préamplification génomique, le produit de préamplification génomique comprenant la séquence commune à son extrémité 5' et une séquence complémentaire de la séquence commune à son extrémité 3' ; dans lequel le premier programme de cycle thermique comprend :
(b1) pour le premier cycle, une réaction à une première température de dénaturation à une température comprise entre 90 et 95°°C pendant 1 à 20 min ; pour le cycle suivant le premier cycle, une réaction à une première température de dénaturation à une température comprise entre 90 et 95°°C pendant 3 à 50 s ;
(b2) une réaction à une première température d'hybridation comprise entre 10 et 20 °C pendant 3 à 60 s, une réaction à une deuxième température de dénaturation comprise entre 20 et 30 °C pendant 3 à 50 s,
et une réaction à une troisième température de dénaturation comprise entre 30 et 50 °C pendant 3 à 50 s ;
(b3) une réaction à une première température d'extension comprise entre 60 et 80 °C pendant 10 s à 15 min ;
(b4) la répétition des étapes (b1) à (b3) pendant 2 à 40 cycles ;
et
(d) le placement du deuxième mélange réactionnel dans un deuxième programme de cycle thermique de telle sorte que la séquence commune de la deuxième amorce soit capable de s'apparier avec l'extrémité 3' du produit de préamplification génomique et le produit de préamplification génomique soit amplifié pour obtenir un produit d'amplification génomique étendu, le deuxième programme de cycle thermique comprenant :
(d1) une réaction à une deuxième température de dénaturation comprise entre 90 et 95 °C pendant 5 s à 20 min ;
(d2) une réaction à une deuxième température de fusion comprise entre 90 et 95 °C pendant 3 à 50 s ;
(d3) une réaction à une quatrième température d'hybridation comprise entre 45 et 65 °C pendant 3 à 50 s ;
(d4) une réaction à une deuxième température d'extension comprise entre 60 et 80°°C pendant 10 s à 15 min ;
(d5) la répétition des étapes (d2) à (d4) pendant 2 à 40 cycles.

13. Procédé selon la revendication 12, dans lequel la séquence commune comprend ou est constituée de SEQ ID N° :6 ;
Xₐᵢ (i = 1 à n) de la première séquence aléatoire appartiennent tous à D, X_{bi} (i = 1 à n) de la troisième séquence aléatoire appartiennent tous à B.

14. Kit d'amplification d'ADN génomique, ledit kit comprenant des amorces, dans lequel les amorces sont constituées de premières amorces et de troisièmes amorces,
dans lequel les premières amorces sont constituées, dans une orientation 5'-3', d'une séquence commune et d'une première séquence variable, ou dans lequel les premières amorces sont constituées, dans une orientation 5'-3' d'une séquence commune, d'une première séquence d'espaceur et d'une première séquence variable, les premières amorces étant un mélange d'amorces comprenant la même séquence commune et des premières séquences variables différentes, chaque première séquence variable étant constituée d'une première séquence aléatoire et d'une séquence fixe à son extrémité 3', la première séquence aléatoire étant, dans une orientation 5'-3', séquentiellement Xₐ₁Xₐ₂......Xₐₙ et Xₐᵢ (i = 1 à n) de la première séquence aléatoire appartenant tous à un même ensemble, ledit ensemble étant choisi parmi B ou D ou H ou V, dans lequel B = {T, G, C}, D = {A, T, G}, H = {T, A, C}, V = {A, C, G}, Xₐᵢ représentant le i^{ème} nucléotide à partir de l'extrémité 5' d'une première séquence aléatoire, n étant un entier positif de 4 à 16, et dans lequel la première séquence d'espaceur est Yₐ₁......Yₐₘ, dans laquelle Yₐⱼ (j = 1 à m) ∈ {A, T, G, C}, Yₐⱼ représentant le j^{ème} nucléotide à partir de l'extrémité 5' de la première séquence d'espaceur, m étant un entier positif de 1 à 3,
dans lequel les troisièmes amorces sont constituées, dans une orientation 5'-3', la séquence commune et une troisième séquence variable, ou dans lequel les troisièmes amorces sont constituées, dans une orientation 5'-3', de la séquence commune, d'une troisième séquence d'espaceur et d'une troisième séquence variable, les troisièmes amorces étant un mélange d'amorces comprenant la même séquence commune et des troisièmes séquences variables différentes, chaque troisième séquence variable étant constituée d'une troisième séquence aléatoire et d'une séquence fixe à son extrémité 3', la troisième séquence aléatoire étant, dans une orientation 5'-3', séquentiellement X_{b1}X_{b2}......X_{bn}, et X_{bi} (i = 1 à n) de la troisième séquence aléatoire appartenant tous à un même ensemble, ledit ensemble étant choisi parmi B ou D ou H ou V, dans lequel B = {T, G, C}, D = {A, T, G}, H = {T, A, C}, V = {A, C, G}, et X_{bi} (i = 1 à n) et Xₐᵢ (i = 1 à n) appartiennent à des ensembles différents, X_{bi} représentant le i^{ème} nucléotide à partir de l'extrémité 5' de la troisième séquence aléatoire, n étant un entier positif de 4 à 16, et dans lequel la troisième séquence d'espaceur est Y_{b1}......Y_{bm}, dans laquelle Y_{bj} (j = 1 à m) ∈ {A, T, G, C}, Y_{bj} représentant le j^{ème} nucléotide à partir de l'extrémité 5' de la troisième séquence d'espaceur, m étant un entier positif de 1 à 3.
